(19) Europäisches Patentamt
European Patent Office
Office européen des brevets

(11) EP 1 688 433 A1

(12) EUROPEAN PATENT APPLICATION
published in accordance with Art. 158(3) EPC

(43) Date of publication:
09.08.2006 Bulletin 2006/32

(21) Application number: 04773768.9

(22) Date of filing: 08.10.2004

(51) Int Cl.:
*C07K 16/18* (2006.01) *C12N 15/13* (2006.01)
*C12N 5/10* (2006.01) *C12P 21/08* (2006.01)
*A61K 39/395* (2006.01) *A61P 35/00* (2006.01)

(86) International application number:
PCT/JP2004/015317

(87) International publication number:
WO 2005/035578 (21.04.2005 Gazette 2005/16)

(84) Designated Contracting States:
AT BE BG CH CY CZ DE DK EE ES FI FR GB GR HU IE IT LI LU MC NL PL PT RO SE SI SK TR

(30) Priority: 09.10.2003 JP 2003350168
26.04.2004 JP 2004129431

(71) Applicant: Kyowa Hakko Kogyo Co., Ltd
Tokyo 100-8185 (JP)

(72) Inventors:
- IIDA, Shigeru, c/om Kyowa Hakko Kogyo Co.,Ltd. Tokyo 100-8185 (JP)
- SATOH, Mitsuo, c/om Kyowa Hakko Kogyo Co.,Ltd. Tokyo 100-8185 (JP)
- INOUE, Miho, c/om Kyowa Hakko Kogyo Co.,Ltd. Tokyo 100-8185 (JP)
- WAKITANI, Masako, c/om Kyowa Hakko Kogyo Co.,Ltd. Tokyo 100-8185 (JP)
- UCHIDA, Kazuhisa, c/om Kyowa Hakko Kogyo Co.,Ltd. Tokyo 100-8185 (JP)
- NIWA, Rinpei, c/om Kyowa Hakko Kogyo Co.,Ltd. Tokyo 100-8185 (JP)
- SHITARA, Kenya, c/om Kyowa Hakko Kogyo Co.,Ltd. Tokyo 100-8185 (JP)

(74) Representative: Vossius & Partner
Siebertstrasse 4
81675 München (DE)

# (54) ANTIBODY COMPOSITION SPECIFICALLY BINDING TO GANGLIOSIDE GM2

(57) An anti-ganglioside GM2 antibody composition having enhanced effector function which is useful as a medicament has been desired. The present invention provides an antibody composition comprising an antibody molecule which specifically binds to ganglioside GM2 and has complex type N-glycoside-linked sugar chains in the Fc region, wherein the complex type N-glycoside-linked sugar chains have a structure in which fucose is not bound to N-acetylglucosamine in the reducing end in the sugar chains; a transformant which produces the antibody composition; a process for producing the antibody composition; and a pharmaceutical composition comprising the antibody composition.

EP 1 688 433 A1

SEQUENCE LISTING

<110> KYOWA HAKKO KOGYO CO., LTD.

<120> Ganglioside GM2-specific antibody composition

<130> 11622WO1

<150> P2003-350168
<151> 2003-10-09

<150> P2004-129431
<151> 2004-04-26

<160> 43

<170> PatentIn Ver. 2.1

<210> 1
<211> 1504
<212> DNA
<213> Cricetulus griseus

<220>
<221> CDS
<222> (1)..(1119)

<400> 1

```
atg gct cac gct ccc gct agc tgc ccg agc tcc agg aac tct ggg gac    48
Met Ala His Ala Pro Ala Ser Cys Pro Ser Ser Arg Asn Ser Gly Asp
  1               5                  10                  15

ggc gat aag ggc aag ccc agg aag gtg gcg ctc atc acg ggc atc acc    96
Gly Asp Lys Gly Lys Pro Arg Lys Val Ala Leu Ile Thr Gly Ile Thr
             20                  25                  30

ggc cag gat ggc tca tac ttg gca gaa ttc ctg ctg gag aaa gga tac   144
Gly Gln Asp Gly Ser Tyr Leu Ala Glu Phe Leu Leu Glu Lys Gly Tyr
```

35 40 45

gag gtt cat gga att gta cgg cga tcc agt tca ttt aat aca ggt cga 192
Glu Val His Gly Ile Val Arg Arg Ser Ser Ser Phe Asn Thr Gly Arg
50 55 60

att gaa cat tta tat aag aat cca cag gct cat att gaa gga aac atg 240
Ile Glu His Leu Tyr Lys Asn Pro Gln Ala His Ile Glu Gly Asn Met
65 70 75 80

aag ttg cac tat ggt gac ctc acc gac agc acc tgc cta gta aaa atc 288
Lys Leu His Tyr Gly Asp Leu Thr Asp Ser Thr Cys Leu Val Lys Ile
85 90 95

atc aat gaa gtc aaa cct aca gag atc tac aat ctt ggt gcc cag agc 336
Ile Asn Glu Val Lys Pro Thr Glu Ile Tyr Asn Leu Gly Ala Gln Ser
100 105 110

cat gtc aag att tcc ttt gac tta gca gag tac act gca gat gtt gat 384
His Val Lys Ile Ser Phe Asp Leu Ala Glu Tyr Thr Ala Asp Val Asp
115 120 125

gga gtt ggc acc ttg cgg ctt ctg gat gca att aag act tgt ggc ctt 432
Gly Val Gly Thr Leu Arg Leu Leu Asp Ala Ile Lys Thr Cys Gly Leu
130 135 140

ata aat tct gtg aag ttc tac cag gcc tca act agt gaa ctg tat gga 480
Ile Asn Ser Val Lys Phe Tyr Gln Ala Ser Thr Ser Glu Leu Tyr Gly
145 150 155 160

aaa gtg caa gaa ata ccc cag aaa gag acc acc cct ttc tat cca agg 528
Lys Val Gln Glu Ile Pro Gln Lys Glu Thr Thr Pro Phe Tyr Pro Arg
165 170 175

tcg ccc tat gga gca gcc aaa ctt tat gcc tat tgg att gta gtg aac 576
Ser Pro Tyr Gly Ala Ala Lys Leu Tyr Ala Tyr Trp Ile Val Val Asn
180 185 190

ttt cga gag gct tat aat ctc ttt gcg gtg aac ggc att ctc ttc aat 624
Phe Arg Glu Ala Tyr Asn Leu Phe Ala Val Asn Gly Ile Leu Phe Asn
195 200 205

cat gag agt cct aga aga gga gct aat ttt gtt act cga aaa att agc 672
His Glu Ser Pro Arg Arg Gly Ala Asn Phe Val Thr Arg Lys Ile Ser
210 215 220

cgg tca gta gct aag att tac ctt gga caa ctg gaa tgt ttc agt ttg 720
Arg Ser Val Ala Lys Ile Tyr Leu Gly Gln Leu Glu Cys Phe Ser Leu
225 230 235 240

gga aat ctg gac gcc aaa cga gac tgg ggc cat gcc aag gac tat gtc 768
Gly Asn Leu Asp Ala Lys Arg Asp Trp Gly His Ala Lys Asp Tyr Val
245 250 255

gag gct atg tgg ctg atg tta caa aat gat gaa cca gag gac ttt gtc 816
Glu Ala Met Trp Leu Met Leu Gln Asn Asp Glu Pro Glu Asp Phe Val
260 265 270

ata gct act ggg gaa gtt cat agt gtc cgt gaa ttt gtt gag aaa tca 864
Ile Ala Thr Gly Glu Val His Ser Val Arg Glu Phe Val Glu Lys Ser
275 280 285

ttc atg cac att gga aag acc att gtg tgg gaa gga aag aat gaa aat 912
Phe Met His Ile Gly Lys Thr Ile Val Trp Glu Gly Lys Asn Glu Asn
290 295 300

gaa gtg ggc aga tgt aaa gag acc ggc aaa att cat gtg act gtg gat 960
Glu Val Gly Arg Cys Lys Glu Thr Gly Lys Ile His Val Thr Val Asp
305 310 315 320

ctg aaa tac tac cga cca act gaa gtg gac ttc ctg cag gga gac tgc 1008
Leu Lys Tyr Tyr Arg Pro Thr Glu Val Asp Phe Leu Gln Gly Asp Cys
325 330 335

tcc aag gcg cag cag aaa ctg aac tgg aag ccc cgc gtt gcc ttt gac 1056

Ser Lys Ala Gln Gln Lys Leu Asn Trp Lys Pro Arg Val Ala Phe Asp
340 345 350

gag ctg gtg agg gag atg gtg caa gcc gat gtg gag ctc atg aga acc 1104
Glu Leu Val Arg Glu Met Val Gln Ala Asp Val Glu Leu Met Arg Thr
355 360 365

aac ccc aac gcc tga gcacctctac aaaaaaattc gcgagacatg gactatggtg 1159
Asn Pro Asn Ala
370

cagagccagc caaccagagt ccagccactc ctgagaccat cgaccataaa ccctcgactg 1219
cctgtgtcgt ccccacagct aagagctggg ccacaggttt gtgggcacca ggacggggac 1279
actccagagc taaggccact tcgcttttgt caaaggctcc tctcaatgat tttgggaaat 1339
caagaagttt aaaatcacat actcatttta cttgaaatta tgtcactaga caacttaaat 1399
ttttgagtct tgagattgtt tttctctttt cttattaaat gatctttcta tgacccagca 1459
aaaaaaaaaa aaaaaaggga tataaaaaaa aaaaaaaaaa aaaaa 1504

<210> 2
<211> 372
<212> PRT
<213> Cricetulus griseus

<400> 2
Met Ala His Ala Pro Ala Ser Cys Pro Ser Ser Arg Asn Ser Gly Asp
1 5 10 15

Gly Asp Lys Gly Lys Pro Arg Lys Val Ala Leu Ile Thr Gly Ile Thr
20 25 30

Gly Gln Asp Gly Ser Tyr Leu Ala Glu Phe Leu Leu Glu Lys Gly Tyr
35 40 45

Glu Val His Gly Ile Val Arg Arg Ser Ser Ser Phe Asn Thr Gly Arg
50 55 60

Ile Glu His Leu Tyr Lys Asn Pro Gln Ala His Ile Glu Gly Asn Met

65 70 75 80

Lys Leu His Tyr Gly Asp Leu Thr Asp Ser Thr Cys Leu Val Lys Ile
85 90 95

Ile Asn Glu Val Lys Pro Thr Glu Ile Tyr Asn Leu Gly Ala Gln Ser
100 105 110

His Val Lys Ile Ser Phe Asp Leu Ala Glu Tyr Thr Ala Asp Val Asp
115 120 125

Gly Val Gly Thr Leu Arg Leu Leu Asp Ala Ile Lys Thr Cys Gly Leu
130 135 140

Ile Asn Ser Val Lys Phe Tyr Gln Ala Ser Thr Ser Glu Leu Tyr Gly
145 150 155 160

Lys Val Gln Glu Ile Pro Gln Lys Glu Thr Thr Pro Phe Tyr Pro Arg
165 170 175

Ser Pro Tyr Gly Ala Ala Lys Leu Tyr Ala Tyr Trp Ile Val Val Asn
180 185 190

Phe Arg Glu Ala Tyr Asn Leu Phe Ala Val Asn Gly Ile Leu Phe Asn
195 200 205

His Glu Ser Pro Arg Arg Gly Ala Asn Phe Val Thr Arg Lys Ile Ser
210 215 220

Arg Ser Val Ala Lys Ile Tyr Leu Gly Gln Leu Glu Cys Phe Ser Leu
225 230 235 240

Gly Asn Leu Asp Ala Lys Arg Asp Trp Gly His Ala Lys Asp Tyr Val
245 250 255

Glu Ala Met Trp Leu Met Leu Gln Asn Asp Glu Pro Glu Asp Phe Val
260 265 270

Ile Ala Thr Gly Glu Val His Ser Val Arg Glu Phe Val Glu Lys Ser
275 280 285

Phe Met His Ile Gly Lys Thr Ile Val Trp Glu Gly Lys Asn Glu Asn
290 295 300

Glu Val Gly Arg Cys Lys Glu Thr Gly Lys Ile His Val Thr Val Asp
305 310 315 320

Leu Lys Tyr Tyr Arg Pro Thr Glu Val Asp Phe Leu Gln Gly Asp Cys
325 330 335

Ser Lys Ala Gln Gln Lys Leu Asn Trp Lys Pro Arg Val Ala Phe Asp
340 345 350

Glu Leu Val Arg Glu Met Val Gln Ala Asp Val Glu Leu Met Arg Thr
355 360 365

Asn Pro Asn Ala
370

<210> 3
<211> 1316
<212> DNA
<213> Cricetulus griseus

<400> 3
gccccgcccc ctccacctgg accgagagta gctggagaat tgtgcaccgg aagtagctct 60

tggactggtg gaaccctgcg caggtgcagc aacaatgggt gagccccagg gatccaggag 120

gatcctagtg acagggggct ctggactggt gggcagagct atccagaagg tggtcgcaga 180

tggcgctggc ttacccggag aggaatgggt gtttgtctcc tccaaagatg cagatctgac 240

ggatgcagca caaacccaag ccctgttcca gaaggtacag cccacccatg tcatccatct 300

tgctgcaatg gtaggaggcc ttttccggaa tatcaaatac aacttggatt tctggaggaa 360

gaatgtgcac atcaatgaca acgtcctgca ctcagctttc gaggtgggca ctcgcaaggt 420

ggtctcctgc ctgtccacct gtatcttccc tgacaagacc acctatccta ttgatgaaac 480

aatgatccac aatggtccac cccacagcag caattttggg tactcgtatg ccaagaggat 540

gattgacgtg cagaacaggg cctacttcca gcagcatggc tgcaccttca ctgctgtcat 600

ccctaccaat gtctttggac ctcatgacaa cttcaacatt gaagatggcc atgtgctgcc 660

tggcctcatc cataaggtgc atctggccaa gagtaatggt tcagccttga ctgtttgggg 720

tacagggaaa ccacggaggc agttcatcta ctcactggac ctagcccggc tcttcatctg 780

ggtcctgcgg gagtacaatg aagttgagcc catcatcctc tcagtgggcg aggaagatga 840

agtctccatt aaggaggcag ctgaggctgt agtggaggcc atggacttct gtggggaagt 900

cacttttgat tcaacaaagt cagatgggca gtataagaag acagccagca atggcaagct 960

tcgggcctac ttgcctgatt tccgtttcac acccttcaag caggctgtga aggagacctg 1020

tgcctggttc accgacaact atgagcaggc ccggaagtga agcatgggac aagcgggtgc 1080

tcagctggca atgcccagtc agtaggctgc agtctcatca tttgcttgtc aagaactgag 1140

gacagtatcc agcaacctga gccacatgct ggtctctctg ccagggggct tcatgcagcc 1200

atccagtagg gcccatgttt gtccatcctc gggggaaggc cagaccaaca ccttgtttgt 1260

ctgcttctgc cccaacctca gtgcatccat gctggtcctg ctgtcccttg tctaga 1316

<210> 4
<211> 321
<212> PRT

<213> Cricetulus griseus

<400> 4

Met Gly Glu Pro Gln Gly Ser Arg Arg Ile Leu Val Thr Gly Gly Ser
1 5 10 15

Gly Leu Val Gly Arg Ala Ile Gln Lys Val Val Ala Asp Gly Ala Gly
20 25 30

Leu Pro Gly Glu Glu Trp Val Phe Val Ser Ser Lys Asp Ala Asp Leu
35 40 45

Thr Asp Ala Ala Gln Thr Gln Ala Leu Phe Gln Lys Val Gln Pro Thr
50 55 60

His Val Ile His Leu Ala Ala Met Val Gly Gly Leu Phe Arg Asn Ile
65 70 75 80

Lys Tyr Asn Leu Asp Phe Trp Arg Lys Asn Val His Ile Asn Asp Asn
85 90 95

Val Leu His Ser Ala Phe Glu Val Gly Thr Arg Lys Val Val Ser Cys
100 105 110

Leu Ser Thr Cys Ile Phe Pro Asp Lys Thr Thr Tyr Pro Ile Asp Glu
115 120 125

Thr Met Ile His Asn Gly Pro Pro His Ser Ser Asn Phe Gly Tyr Ser
130 135 140

Tyr Ala Lys Arg Met Ile Asp Val Gln Asn Arg Ala Tyr Phe Gln Gln
145 150 155 160

His Gly Cys Thr Phe Thr Ala Val Ile Pro Thr Asn Val Phe Gly Pro
165 170 175

His Asp Asn Phe Asn Ile Glu Asp Gly His Val Leu Pro Gly Leu Ile

180 185 190

His Lys Val His Leu Ala Lys Ser Asn Gly Ser Ala Leu Thr Val Trp
195 200 205

Gly Thr Gly Lys Pro Arg Arg Gln Phe Ile Tyr Ser Leu Asp Leu Ala
210 215 220

Arg Leu Phe Ile Trp Val Leu Arg Glu Tyr Asn Glu Val Glu Pro Ile
225 230 235 240

Ile Leu Ser Val Gly Glu Glu Asp Glu Val Ser Ile Lys Glu Ala Ala
245 250 255

Glu Ala Val Val Glu Ala Met Asp Phe Cys Gly Glu Val Thr Phe Asp
260 265 270

Ser Thr Lys Ser Asp Gly Gln Tyr Lys Lys Thr Ala Ser Asn Gly Lys
275 280 285

Leu Arg Ala Tyr Leu Pro Asp Phe Arg Phe Thr Pro Phe Lys Gln Ala
290 295 300

Val Lys Glu Thr Cys Ala Trp Phe Thr Asp Asn Tyr Glu Gln Ala Arg
305 310 315 320

Lys

<210> 5
<211> 2008
<212> DNA
<213> Cricetulus griseus

<400> 5
aacagaaact tattttcctg tgtggctaac tagaaccaga gtacaatgtt tccaattctt 60

tgagctccga gaagacagaa gggagttgaa actctgaaaa tgcgggcatg gactggttcc 120
tggcgttgga ttatgctcat tctttttgcc tgggggacct tattgtttta tataggtggt 180
catttggttc gagataatga ccaccctgac cattctagca gagaactctc caagattctt 240
gcaaagctgg agcgcttaaa acaacaaaat gaagacttga ggagaatggc tgagtctctc 300
cgaataccag aaggccctat tgatcagggg acagctacag gaagagtccg tgttttagaa 360
gaacagcttg ttaaggccaa agaacagatt gaaaattaca agaaacaagc taggaatgat 420

ctgggaaagg atcatgaaat cttaaggagg aggattgaaa atggagctaa agagctctgg 480
tttttctac aaagtgaatt gaagaaatta aagaaattag aaggaaacga actccaaaga 540
catgcagatg aaattctttt ggatttagga catcatgaaa ggtctatcat gacagatcta 600
tactacctca gtcaaacaga tggagcaggt gagtggcggg aaaaagaagc caaagatctg 660
acagagctgg tccagcggag aataacatat ctgcagaatc ccaaggactg cagcaaagcc 720
agaaagctgg tatgtaatat caacaaaggc tgtggctatg gatgtcaact ccatcatgtg 780
gtttactgct tcatgattgc ttatggcacc cagcgaacac tcatcttgga atctcagaat 840
tggcgctatg ctactggagg atgggagact gtgtttagac ctgtaagtga gacatgcaca 900
gacaggtctg gcctctccac tggacactgg tcaggtgaag tgaaggacaa aaatgttcaa 960
gtggtcgagc tccccattgt agacagcctc catcctcgtc ctccttactt acccttggct 1020
gtaccagaag accttgcaga tcgactcctg agagtccatg gtgatcctgc agtgtggtgg 1080
gtatcccagt ttgtcaaata cttgatccgt ccacaacctt ggctggaaag ggaaatagaa 1140
gaaaccacca agaagcttgg cttcaaacat ccagttattg gagtccatgt cagacgcact 1200

gacaaagtgg gaacagaagc agccttccat cccattgagg aatacatggt acacgttgaa 1260

gaacattttc agcttctcga acgcagaatg aaagtggata aaaaaagagt gtatctggcc 1320

actgatgacc cttctttgtt aaaggaggca aagacaaagt actccaatta tgaatttatt 1380

agtgataact ctatttcttg gtcagctgga ctacacaacc gatacacaga aaattcactt 1440

cggggcgtga tcctggatat acactttctc tcccaggctg acttccttgt gtgtactttt 1500

tcatcccagg tctgtagggt tgcttatgaa atcatgcaaa cactgcatcc tgatgcctct 1560

gcaaacttcc attctttaga tgacatctac tattttggag gccaaaatgc ccacaaccag 1620

attgcagttt atcctcacca acctcgaact aaagaggaaa tccccatgga acctggagat 1680

atcattggtg tggctggaaa ccattggaat ggttactcta aaggtgtcaa cagaaaacta 1740

ggaaaaacag gcctgtaccc ttcctacaaa gtccgagaga agatagaaac agtcaaatac 1800

cctacatatc ctgaagctga aaaatagaga tggagtgtaa gagattaaca acagaattta 1860

gttcagacca tctcagccaa gcagaagacc cagactaaca tatggttcat tgacagacat 1920

gctccgcacc aagagcaagt gggaaccctc agatgctgca ctggtggaac gcctctttgt 1980

gaagggctgc tgtgccctca agcccatg 2008

<210> 6
<211> 1728
<212> DNA
<213> Mus musculus

<400> 6
atgcgggcat ggactggttc ctggcgttgg attatgctca ttctttttgc ctgggggacc 60

ttgttatttt atataggtgg tcatttggtt cgagataatg accaccctga tcactccagc 120

agagaactct ccaagattct tgcaaagctt gaacgcttaa aacagcaaaa tgaagacttg 180

aggcgaatgg ctgagtctct ccgaatacca gaaggcccca ttgaccaggg gacagctaca 240

ggaagagtcc gtgttttaga agaacagctt gttaaggcca aagaacagat tgaaaattac 300

aagaaacaag ctagaaatgg tctggggaag gatcatgaaa tcttaagaag gaggattgaa 360

aatggagcta aagagctctg gtttttctca caaagcgaac tgaagaaatt aaagcattta 420

gaaggaaatg aactccaaag acatgcagat gaaattcttt tggatttagg acaccatgaa 480

aggtctatca tgacagatct atactacctc agtcaaacag atggagcagg ggattggcgt 540

gaaaaagagg ccaaagatct gacagagctg gtccagcgga gaataacata tctccagaat 600

cctaaggact gcagcaaagc caggaagctg gtgtgtaaca tcaataaagg ctgtggctat 660

ggttgtcaac tccatcacgt ggtctactgt ttcatgattg cttatggcac ccagcgaaca 720

ctcatcttgg aatctcagaa ttggcgctat gctactggtg gatgggagac tgtgtttaga 780

cctgtaagtg agacatgtac agacagatct ggcctctcca ctggacactg gtcaggtgaa 840

gtaaatgaca aaaacattca agtggtcgag ctccccattg tagacagcct ccatcctcgg 900

cctccttact taccactggc tgttccagaa gaccttgcag accgactcct aagagtccat 960

ggtgaccctg cagtgtggtg ggtgtcccag tttgtcaaat acttgattcg tccacaacct 1020

tggctggaaa aggaaataga agaagccacc aagaagcttg gcttcaaaca tccagttatt 1080

ggagtccatg tcagacgcac agacaaagtg ggaacagaag cagccttcca ccccatcgag 1140

gagtacatgg tacacgttga agaacatttt cagcttctcg cacgcagaat gcaagtggat 1200

aaaaaaagag tatatctggc tactgatgat cctactttgt taaaggaggc aaagacaaag 1260

tactccaatt atgaatttat tagtgataac tctatttctt ggtcagctgg actacacaat 1320

cggtacacag aaaattcact tcggggtgtg atcctggata tacactttct ctcacaggct 1380

gactttctag tgtgtacttt ttcatcccag gtctgtcggg ttgcttatga aatcatgcaa 1440

accctgcatc ctgatgcctc tgcgaacttc cattctttgg atgacatcta ctattttgga 1500

ggccaaaatg cccacaatca gattgctgtt tatcctcaca aacctcgaac tgaagaggaa 1560

attccaatgg aacctggaga tatcattggt gtggctggaa accattggga tggttattct 1620

aaaggtatca acagaaaact tggaaaaaca ggcttatatc cctcctacaa agtccgagag 1680

aagatagaaa cagtcaagta tcccacatat cctgaagctg aaaaatag 1728

<210> 7
<211> 575
<212> PRT
<213> Cricetulus griseus

<400> 7
Met Arg Ala Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Thr Ala Thr

65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Asp Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys Lys Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Glu Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Lys Asp Lys Asn Val Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Arg Glu Ile Glu Glu Thr Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val
370 375 380

His Val Glu Glu His Phe Gln Leu Leu Glu Arg Arg Met Lys Val Asp
385 390 395 400

Lys Lys Arg Val Tyr Leu Ala Thr Asp Asp Pro Ser Leu Leu Lys Glu
405 410 415

Ala Lys Thr Lys Tyr Ser Asn Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Thr Leu His Pro Asp Ala Ser Ala Asn Phe His Ser Leu Asp Asp Ile
485 490 495

Tyr Tyr Phe Gly Gly Gln Asn Ala His Asn Gln Ile Ala Val Tyr Pro
500 505 510

His Gln Pro Arg Thr Lys Glu Glu Ile Pro Met Glu Pro Gly Asp Ile
515 520 525

Ile Gly Val Ala Gly Asn His Trp Asn Gly Tyr Ser Lys Gly Val Asn
530 535 540

Arg Lys Leu Gly Lys Thr Gly Leu Tyr Pro Ser Tyr Lys Val Arg Glu
545 550 555 560

Lys Ile Glu Thr Val Lys Tyr Pro Thr Tyr Pro Glu Ala Glu Lys
565 570 575

<210> 8
<211> 575
<212> PRT
<213> Mus musculus

<400> 8
Met Arg Ala Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Thr Ala Thr
65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Gly Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys His Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Asp Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Asn Asp Lys Asn Ile Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Lys Glu Ile Glu Glu Ala Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val
370 375 380

His Val Glu Glu His Phe Gln Leu Leu Ala Arg Arg Met Gln Val Asp
385 390 395 400

Lys Lys Arg Val Tyr Leu Ala Thr Asp Asp Pro Thr Leu Leu Lys Glu
405 410 415

Ala Lys Thr Lys Tyr Ser Asn Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Thr Leu His Pro Asp Ala Ser Ala Asn Phe His Ser Leu Asp Asp Ile
485 490 495

Tyr Tyr Phe Gly Gly Gln Asn Ala His Asn Gln Ile Ala Val Tyr Pro
500 505 510

His Lys Pro Arg Thr Glu Glu Glu Ile Pro Met Glu Pro Gly Asp Ile
515 520 525

Ile Gly Val Ala Gly Asn His Trp Asp Gly Tyr Ser Lys Gly Ile Asn
530 535 540

Arg Lys Leu Gly Lys Thr Gly Leu Tyr Pro Ser Tyr Lys Val Arg Glu
545 550 555 560

Lys Ile Glu Thr Val Lys Tyr Pro Thr Tyr Pro Glu Ala Glu Lys
565 570 575

<210> 9
<211> 383
<212> DNA
<213> Cricetulus griseus

<400> 9
gttaactggg gctcttttaa accctgaatt tttctaaatc cccacctcca agagtttggt 60

ttaaactgat tttttaatg aatacctttt gaagaataga gcattgtctc atcatgcaaa 120

gcttctcagg gattcagcta gcatgttgaa gaaacataag ggtgttaaat tgtttgtcac 180

aagtgctgaa taaatattga cgtagtcttc agctattcta tactggaagt agatgatatt 240

ctcattggaa attctgttag gaagtaaccc ttcttgtctt cttacctgca tagaatccca 300

ggatataaaa cttgtgcttg tcgcccttgc cattgtctct cactggtggc ctttattgca 360

tctcatatct gccttctctt tcc 383

<210> 10
<211> 504
<212> DNA
<213> Cricetulus griseus

<400> 10
taagaattcc tgtgcccagc tgtatgtgag gctctctgca ggtgtaggga tgtttctgct 60

ttctttctgc acatgcttca cagctgaagt cctttgggtg tgagattgac attcagatag 120

actaaagtga ctggacttgt tgggaaacat actgtatgca ttattgccgt tgcctccagg 180

tgaaattaac acctcattca ccaatccctg ttcatccaaa ctttctaccc acatcacttt 240

aaatagaaat tagacccaat atgactcctt ttttcctaag ctgtttatag agattgtgct 300

ggagcagtga gcttttgtgt ttgtttgttt gttttgtaat tttccccatg aaaatttctc 360

taaactcaaa cctaagaggg aaaaaaaaaa aacagactta tatgtgccac acttgtaaaa 420

aaaaatcatg aaagatgtat atgatatttt taaacagttt gaatattaag atcacaattt 480

ctattttaaa aacaatcttg ttttacatat caatcaccca attcccttgc cttcccatcc 540

tcccattccc cccactgatc cccc 564

<210> 11
<211> 120
<212> DNA
<213> Cricetulus griseus

<400> 11

atgaatgttc attctttggg tatatgccca agagtagaat tgctaaatat tgaggtagac 60

tgattcccat tttcttgagg agtcgccata ttgatttcca aagtgactgt acaagttaac 120

<210> 12
<211> 274
<212> DNA
<213> Cricetulus griseus

<400> 12
aggcactagg taaatatttt tgaagaaaga atgagtatct cctatttcag aaaaactttt 60

attgacttaa atttaggata tcagaattag aaaacagtaa aaatttatag gagagttttt 120

aatgaatgtt attttaaggt tccatacaaa tagtaattaa aacttacaca aactatttgt 180

agtaatgatt cagtctggta taccctgatg agcattatac acttttaaat tctttttgta 240

aattttttta ttagttcaaa ttaggaacaa gctt 274

<210> 13
<211> 9196
<212> DNA
<213> Cricetulus griseus

<400> 13
tctagaccag gctggtctcg aactcacaga gaaccacctg cctctgccac ctgagtgctg 60

ggattaaagg tgtgcaccac caccgcccgg cgtaaaatca tatttttgaa tattgtgata 120

atttacatta taattgtaag taaaaatttt cagcctattt tgttatacat ttttgcgtaa 180

attattcttt tttgaaagtt ttgttgtcca taatagtcta gggaaacata aagttataat 240

ttttgtctat gtatttgcat atatatctat ttaatctcct aatgtccagg aaataaatag 300

ggtatgtaat agcttcaaca tgtggtatga tagaattttt cagtgctata taagttgtta 360

cagcaaagtg ttattaattc atatgtccat atttcaattt tttatgaatt attaaattga 420

atccttaagc tgccagaact agaattttat tttaatcagg aagccccaaa tctgttcatt 480

ctttctatat atgtggaaag gtaggcctca ctaactgatt cttcacctgt tttagaacat 540

ggtccaagaa tggagttatg taaggggaat tacaagtgtg agaaaactcc tagaaaacaa 600

gatgagtctt gtgaccttag tttctttaaa aacacaaaat tcttggaatg tgttttcatg 660

ttcctcccag gtggatagga gtgagtttat ttcagattat ttattacaac tggctgttgt 720

tacttgtttc tatgtcttta tagaaaaaca tatttttttt gccacatgca gcttgtcctt 780

atgattttat acttgtgtga ctcttaactc tcagagtata aattgtctga tgctatgaat 840

aaagttggct attgtatgag acttcagccc acttcaatta ttggcttcat tctctcagat 900

cccaccacct ccagagtggt aaacaacttg aaccattaaa cagactttag tctttatttg 960

aatgatagat ggggatatca gatttatagg cacagggttt tgagaaaggg agaaggtaaa 1020

cagtagagtt taacaacaac aaaaagtata ctttgtaaac gtaaaactat ttattaaagt 1080

agtagacaag acattaaata ttccttggga ttagtgcttt ttgaattttg ctttcaaata 1140

atagtcagtg agtatacccc tccccccattc tatattttag cagaaatcag aataaatggt 1200

gtttctggta cattcttttg tagagaattt attttctttg ggtttttgtg catttaaagt 1260

caataaaaat taaggttcag taatagaaaa aaaactctga tttttggaat ccccttcttt 1320

cagcttttct atttaatctc ttaatgataa tttaatttgt ggccatgtgg tcaaagtata 1380

tagccttgta tatgtaaatg ttttaaccaa cctgccttta cagtaactat ataattttat 1440

tctataatat atgacttttc ttccatagct ttagagttgc ccagtcactt taagttacat 1500

tttcatatat gttctttgtg ggaggagata attttatttc taagagaatc ctaagcatac 1560

tgattgagaa atggcaaaca aaacacataa ttaaagctga taaagaacga acatttggag 1620

tttaaaatac atagccaccc taagggttta actgttgtta gccttctttt ggaattttta 1680

ttagttcata tagaaaaatg gattttatcg tgacatttcc atatatgtat ataatatatt 1740

tacatcatat ccacctgtaa ttattagtgt ttttaaatat atttgaaaaa ataatggtct 1800

ggtttgatcc atttgaacct tttgatgttt ggtgtggttg ccaattggtt gatggttatg 1860

ataacctttg cttctctaag gttcaagtca gtttgagaat atgtcctcta aaaatgacag 1920

gttgcaagtt aagtagtgag atgacagcga gatggagtga tgagaatttg tagaaatgaa 1980

ttcacttata ctgagaactt gttttgcttt tagataatga acatattagc ctgaagtaca 2040

tagccgaatt gattaattat tcaaagatat aatcttttaa tccctataaa agaggtatta 2100

cacaacaatt caagaaagat agaattagac ttccagtatt ggagtgaacc atttgttatc 2160

aggtagaacc ctaacgtgtg tggttgactt aaagtgttta ctttttacct gatactgggt 2220

agctaattgt ctttcagcct cctggccaaa gataccatga aagtcaactt acgttgtatt 2280

ctatatctca aacaactcag ggtgtttctt actctttcca cagcatgtag agcccaggaa 2340

gcacaggaca agaaagctgc ctccttgtat caccaggaag atctttttgt aagagtcatc 2400

acagtatacc agagagacta atttgtctg aagcatcatg tgttgaaaca acagaaactt 2460

attttcctgt gtggctaact agaaccagag tacaatgttt ccaattcttt gagctccgag 2520

aagacagaag ggagttgaaa ctctgaaaat gcgggcatgg actggttcct ggcgttggat 2580

tatgctcatt ctttttgcct gggggacctt attgttttat ataggtggtc atttggttcg 2640

agataatgac caccctgacc attctagcag agaactctcc aagattcttg caaagctgga 2700

gcgcttaaaa caacaaaatg aagacttgag gagaatggct gagtctctcc ggtaggtttg 2760

aaatactcaa ggatttgatg aaatactgtg cttgaccttt aggtataggg tctcagtctg 2820

ctgttgaaaa atataatttc tacaaaccgt ctttgtaaaa ttttaagtat tgtagcagac 2880

tttttaaaag tcagtgatac atctatatag tcaatatagg tttacatagt tgcaatctta 2940

ttttgcatat gaatcagtat atagaagcag tggcatttat atgcttatgt tgcatttaca 3000

attatgttta gacgaacaca aactttatgt gatttggatt agtgctcatt aaattttttt 3060

attctatgga ctacaacaga gacataaatt ttgaaaggct tagttactct taaattctta 3120

tgatgaaaag caaaaattca ttgttaaata gaacagtgca tccggaatgt gggtaattat 3180

tgccatattt ctagtctact aaaaattgtg gcataactgt tcaaagtcat cagttgtttg 3240

gaaagccaaa gtctgattta aatggaaaac ataaacaatg atatctattt ctagatacct 3300

ttaacttgca gttactgagt ttacaagttg tctgacaact ttggattctc ttacttcata 3360

tctaagaatg atcatgtgta cagtgcttac tgtcacttta aaaaactgca gggctagaca 3420

tgcagatatg aagactttga cattagatgt ggtaattggc actaccagca agtggtatta 3480

agatacagct gaatatatta ctttttgagg aacataattc atgaatggaa agtggagcat 3540

tagagaggat gccttctggc tctcccacac cactgtttgc atccattgca tttcacactg 3600

cttttagaac tcagatgttt catatggtat attgtgtaac tcaccatcag ttttatcttt 3660

aaatgtctat ggatgataat gttgtatgtt aacactttta caaaaacaaa tgaagccata 3720

tcctcggtgt gagttgtgat ggtggtaatt gtcacaatag gattattcag caaggaacta 3780

agtcagggac aagaagtggg cgatactttg ttggattaaa tcattttact ggaagttcat 3840

cagggagggt tatgaaagtt gtggtctttg aactgaaatt atatgtgatt cattattctt 3900

gatttaggcc ttgctaatag taactatcat ttattgggaa tttgtcatat gtgccaattt 3960

gtcatgggcc agacagcgtg ttttactgaa tttctagata tctttatgag attctagtac 4020

tgttttcagc cattttacag atgaagaatc ttaaaaaatg ttaaataatt tagtttgccc 4080

aagattatac gttaacaaat ggtagaacct tctttgaatt ctggcagtat ggctacacag 4140

tccgaactct tatcttccta agctgaaaac agaaaaagca atgacccaga aaattttatt 4200

taaaagtctc aggagagact tcccatcctg agaagatctc ttttcccttt tataatttag 4260

gctcctgaat aatcactgaa ttttctccat gttccatcta tagtactgtt atttctgttt 4320

tcctttttc ttaccacaaa gtatcttgtt tttgctgtat gaaagaaaat gtgttattgt 4380

aatgtgaaat tctctgtccc tgcagggtcc cacatccgcc tcaatcccaa ataaacacac 4440

agaggctgta ttaattatga aactgttggt cagttggcta gggcttctta ttggctagct 4500

ctgtcttaat tattaaacca taactactat tgtaagtatt tccatgtggt cttatcttac 4560

caaggaaagg gtccagggac ctcttactcc tctggcgtgt tggcagtgaa gaggagagag 4620

cgatttccta tttgtctctg cttattttct gattctgctc agctatgtca cttcctgcct 4680

ggccaatcag ccaatcagtg ttttattcat tagccaataa aagaaacatt tacacagaag 4740

gacttccccc atcatgttat ttgtatgagt tcttcagaaa atcatagtat cttttaatac 4800

taatttttat aaaaaattaa ttgtattgaa aattatgtgt atatgtgtct gtgtgtcgat 4860

ttgtgctcat aagtagcatg gagtgcagaa gagggaatca gatctttttt taagggacaa 4920

agagtttatt cagattacat tttaaggtga taatgtatga ttgcaaggtt atcaacatgg 4980

cagaaatgtg aagaagctgg tcacattaca tccagagtca agagtagaga gcaatgaatt 5040

gatgcatgca ttcctgtgct cagctcactt ttcctggagc tgagctgatt gtaagccatc 5100

tgatgtcttt gctgggaact aactcaaagg caagttcaaa acctgttctt aagtataagc 5160

catctctcca gtccctcata tggtctctta agacactttc tttatattct tgtacataga 5220

aattgaattc ctaacaactg cattcaaatt acaaaatagt ttttaaaagc tgatataata 5280

aatgtaaata caatctagaa catttttata aataagcata ttaactcagt aaaaataaat 5340

gcatggttat tttccttcat tagggaagta tgtctcccca ggctgttctc tagattctac 5400

tagtaatgct gtttgtacac catccacagg ggttttattt taaagctaag acatgaatga 5460

tggacatgct tgttagcatt tagacttttt tccttactat aattgagcta gtatttttgt 5520

gctcagtttg atatctgtta attcagataa atgtaatagt aggtaatttc tttgtgataa 5580

aggcatataa attgaagttg gaaaacaaaa gcctgaaatg acagttttta agattcagaa 5640

caataatttt caaaagcagt tacccaactt tccaaataca atctgcagtt ttcttgatat 5700

gtgataaatt tagacaaaga aatagcacat tttaaaatag ctatttactc ttgatttttt 5760

tttcaaattt aggctagttc actagttgtg tgtaaggtta tggctgcaaa catctttgac 5820

tcttggttag ggaatccagg atgatttacg tgtttggcca aaatcttgtt ccattctggg 5880

tttcttctct atctaggtag ctagcacaag ttaaaggtgt ggtagtattg gaaggctctc 5940

aggtatatat ttctatattc tgtatttttt tcctctgtca tatatttgct ttctgtttta 6000

ttgatttcta ctgttagttt gatacttact ttcttacact ttctttggga tttattttgc 6060

tgttctaaga tttcttagca agttcatatc actgatttta acagttgctt cttttgtaat 6120

atagactgaa tgccccttat ttgaaatgct tgggatcaga aactcagatt tgaacttttc 6180

tttttaata tttccatcaa gtttaccagc tgaatgtcct gatccaagaa tatgaaatct 6240

gaaatgcttt gaaatctgaa acttttagag tgataaagct tccctttaaa ttaatttgtg 6300

ttctatattt tttgacaatg tcaacctttc attgttatcc aatgagtgaa catattttca 6360

atttttttgt ttgatctgtt atattttgat ctgaccatat ttataaaatt ttatttaatt 6420

tgaatgttgt gctgttactt atctttatta ttatttttgc ttattttcta gccaaatgaa 6480

attatattct gtattatttt agtttgaatt ttactttgtg gcttagtaac tgccttttgt 6540

tggtgaatgc ttaagaaaaa cgtgtggtct actgatattg gttctaatct tatatagcat 6600

gttgtttgtt aggtagttga ttatgctggt cagattgtct tgagtttatg caaatgtaaa 6660

atatttagat gcttgttttg ttgtctaaga acaaagtatg cttgctgtct cctatcggtt 6720

ctggtttttc cattcatctc ttcaagctgt tttgtgtgtt gaatactaac tccgtactat 6780

cttgttttct gtgaattaac ccctttttcaa aggtttcttt tctttttttt tttaagggac 6840

aacaagttta ttcagattac attttaagct gataatgtat gattgcaagg ttatcaacat 6900

ggcagaaatg tgaagaagct aggcacatta catccacatg gagtcaagag cagagagcag 6960

tgaattaatg catgcattcc tgtggtcagc tcacttttcc tattcttaga tagtctagga 7020

tcataaacct ggggaatagt gctaccacaa tgggcatatc cacttacttc agttcatgca 7080

atcaaccaag gcacatccac aggaaaaact gatttagaca acctctcatt gagactcttc 7140

ccagatgatt agactgtgtc aagttgacaa ttaaaactat cacacctgaa gccatcacta 7200

gtaaatataa tgaaaatgtt gattatcacc ataattcatc tgtatccctt tgttattgta 7260

gattttgtga agttcctatt caagtccctg ttccttcctt aaaaacctgt tttttagtta 7320

aataggtttt ttagtgttcc tgtctgtaaa tacttttta aagttagata ttattttcaa 7380

gtatgttctc ccagtctttg gcttgtattt tcatcccttc aatacatata tttttgtaat 7440

ttatttttt tatttaaatt agaaacaaag ctgcttttac atgtcagtct cagttccctc 7500

tccctcccct cctcccctgc tccccaccta agccccaatt ccaactcctt tcttctcccc 7560

aggaagggtg aggccctcca tgggggaaat cttcaatgtc tgtcatatca tttggagcag 7620

ggcctagacc ctccccagtg tgtctaggct gagagagtat ccctctatgt ggagagggct 7680

cccaaagttc atttgtgtac taggggtaaa tactgatcca ctatcagtgg ccccatagat 7740

tgtccggacc tccaaactga cttcctcctt cagggagtct ggaacagttc tatgctggtt 7800

tcccagatat cagtctgggg tccatgagca accccttgtt caggtcagtt gtttctgtag 7860

gtttccccag cccggtcttg accccttttgc tcatcacttc tccctctctg caactggatt 7920

ccagagttca gctcagtgtt tagctgtggg tgtctgcatc tgcttccatc agctactgga 7980

tgagggctct aggatggcat ataaggtagt catcagtctc attatcagag aagggctttt 8040

aaggtagcct cttgattatt gcttagattg ttagttgggg tcaaccttgt aggtctctgg 8100

acagtgacag aattctcttt aaacctataa tggctccctc tgtggtggta tcccttttct 8160

tgctctcatc cgttcctccc ctgactagat cttcctgctc cctcatgtcc tcctctcccc 8220

tccccttctc cccttctctt tcttctaact ccctctcccc tccacccacg atccccatta 8280

gcttatgaga tcttgtcctt attttagcaa aacctttttg gctataaaat taattaattt 8340

aatatgctta tatcaggttt attttggcta gtatttgtat gtgtttggtt agtgttttta 8400

accttaattg acatgtatcc ttatatttag acacagattt aaatatttga agtttttttt 8460

tttttttttt ttaaagattt atttattttt tatgtcttct gcctgcatgc cagaagaggg 8520

caccagatct cattcaaggt ggttgtgagc caccatgtgg ttgctgggaa ttgaactcag 8580

gacctctgga agaacagtca gtgctcttaa ccgctgagcc atctctccag cccctgaagt 8640

gtttctttta aagaggatag cagtgcatca tttttccctt tgaccaatga ctcctacctt 8700

actgaattgt tttagccatt tatatgtaat gctgttacca ggtttacatt ttcttttatc 8760

ttgctaaatt tcttccctgt ttgtctcatc tcttattttt gtctgttgga ttatataggc 8820

ttttattttt ctgtttttac agtaagttat atcaaattaa aattatttta tggaatgggt 8880

gtgttgacta catgtatgtc tgtgcaccat gtgctgacct ggtcttggcc agaagaaggt 8940

gtcatattct ctgaaactgg tattgtggat gttacgaact gccatagggt gctaggaatc 9000

aaacccccagc tcctctggaa aagcagccac tgctctgagc cactgagtcc tctcttcaag 9060

caggtgatgc caacttttaa tggttaccag tggataagag tgcttgtatc tctagcaccc 9120

atgaaaattt atgcattgct atatgggctt gtcacttcag cattgtgtga cagagacagg 9180

aggatcccaa gagctc 9196

<210> 14
<211> 5
<212> PRT
<213> Mus musculus

<400> 14

Asn Tyr Asn Met Asp
1 5

<210> 15
<211> 17
<212> PRT
<213> Mus musculus

<400> 15

Tyr Ile Tyr Pro Asn Asn Gly Gly Thr Gly Tyr Asn Gln Lys Phe Lys
1 5 10 15

Ser

<210> 16
<211> 11
<212> PRT
<213> Mus musculus

<400> 16

Thr Gly His Tyr Tyr Gly Tyr Met Phe Ala Tyr
1 5 10

<210> 17
<211> 10
<212> PRT
<213> Mus musculus

<400> 17

Ser Ala Ser Ser Ser Val Ser Tyr Met His
1 5 10

<210> 18
<211> 7
<212> PRT
<213> Mus musculus

<400> 18
Ser Thr Ser Asn Leu Ala Ser

1 5

<210> 19
<211> 9
<212> PRT
<213> Mus musculus

<400> 19
Gln Gln Arg Ser Ser Tyr Pro Tyr Thr
1 5

<210> 20
<211> 120
<212> PRT
<213> Mus musculus

<400> 20
Glu Val Gln Leu Gln Gln Ser Gly Pro Glu Leu Val Lys Pro Gly Ala
1 5 10 15

Ser Val Lys Ile Ser Cys Lys Ala Ser Gly Tyr Thr Phe Thr Asp Tyr
20 25 30

Asn Met Asp Trp Val Lys Gln Ser His Gly Lys Ser Leu Glu Trp Ile
35 40 45

Gly Tyr Ile Tyr Pro Asn Asn Gly Gly Thr Gly Tyr Asn Gln Lys Phe
50 55 60

Lys Ser Lys Ala Thr Leu Thr Val Asp Lys Ser Ser Ser Thr Ala Tyr
65 70 75 80

Met Glu Leu His Ser Leu Thr Ser Glu Asp Ser Ala Val Tyr Tyr Cys
85 90 95

Ala Thr Tyr Gly His Tyr Tyr Gly Tyr Met Phe Ala Tyr Trp Gly Gln
100 105 110

Gly Thr Leu Val Thr Val Ser Ala
115 120

<210> 21
<211> 107
<212> PRT
<213> Mus musculus

<400> 21
Gln Ile Val Leu Thr Gln Ser Pro Ala Ile Met Ser Ala Ser Pro Gly
1 5 10 15

Glu Lys Val Thr Ile Thr Cys Ser Ala Ser Ser Ser Val Ser Tyr Met
20 25 30

His Trp Phe Gln Gln Lys Pro Gly Thr Ser Pro Lys Leu Trp Ile Tyr
35 40 45

Ser Thr Ser Asn Leu Ala Ser Gly Val Pro Ala Arg Phe Ser Gly Ser
50 55 60

Gly Ser Gly Thr Ser Tyr Ser Leu Thr Ile Ser Arg Met Glu Ala Glu
65 70 75 80

Asp Ala Ala Thr Tyr Tyr Cys Gln Gln Arg Ser Ser Tyr Pro Tyr Thr
85 90 95

Phe Gly Gly Gly Thr Lys Leu Glu Ile Lys Arg
100 105

<210> 22
<211> 125
<212> PRT
<213> Artificial sequence

<220>
<223> Description of Artificial Sequence: Amino Acid Sequence of Antibody Heavy Chain Variable Region

<400> 22
Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Asn Met Asp Trp Val Arg Gln Ala Pro Gly Gln Gly Leu Glu Trp Met
35 40 45

Gly Tyr Ile Tyr Pro Asn Asn Gly Gly Thr Gly Tyr Asn Gln Lys Phe
50 55 60

Lys Ser Lys Val Thr Ile Thr Val Asp Thr Ser Thr Ser Thr Ala Tyr
65 70 75 80

Met Glu Leu His Ser Leu Arg Ser Glu Asp Thr Ala Val Tyr Tyr Cys
85 90 95

Ala Thr Tyr Gly His Tyr Tyr Gly Tyr Met Phe Ala Tyr Trp Gly Gln
100 105 110

Gly Thr Leu Val Thr Val Ser Ser Ala Ser Thr Lys Gly
115 120 125

<210> 23
<211> 125
<212> PRT
<213> Artificial Sequence

<220>
<223> Description of Artificial Sequence: Amino Acid Sequence of Antibody Heavy Chain Variable Region

<400> 23

```
Glu Val Gln Leu Val Gln Ser Gly Ala Glu Val Lys Lys Pro Gly Ala
  1               5                  10                  15

Ser Val Lys Val Ser Cys Lys Ala Ser Gly Tyr Thr Phe Thr Asp Tyr
             20                  25                  30

Asn Met Asp Trp Val Arg Gln Ala Pro Gly Gln Gly Leu Glu Trp Met
         35                  40                  45

Gly Tyr Ile Tyr Pro Asn Asn Gly Gly Thr Gly Tyr Asn Gln Lys Phe
     50                  55                  60

Lys Ser Arg Val Thr Ile Thr Ala Asp Thr Ser Thr Ser Thr Ala Tyr
 65                  70                  75                  80

Met Glu Leu Ser Ser Leu Arg Ser Glu Asp Thr Ala Val Tyr Tyr Cys
                 85                  90                  95

Ala Arg Tyr Gly His Tyr Tyr Gly Tyr Met Phe Ala Tyr Trp Gly Gln
            100                 105                 110

Gly Thr Leu Val Thr Val Ser Ser Ala Ser Thr Lys Gly
        115                 120                 125
```

<210> 24
<211> 108

<212> PRT
<213> Artificial Sequence

<220>
<223> Description of Artificial Sequence: Amino Acid Sequence of Antibody Light Chain Variable Region

<400> 24
Asp Ile Gln Leu Thr Gln Ser Pro Ser Ser Leu Ser Ala Ser Val Gly
1               5                   10                  15

Asp Arg Val Thr Ile Thr Cys Ser Ala Ser Ser Ser Val Ser Tyr Met
            20                  25                  30

His Trp Tyr Gln Gln Lys Pro Gly Lys Ala Pro Lys Leu Leu Ile Tyr
        35                  40                  45

Ser Thr Ser Asn Leu Ala Ser Gly Val Pro Ser Arg Phe Ser Gly Ser
    50                  55                  60

Gly Ser Gly Thr Asp Phe Thr Phe Thr Ile Ser Ser Leu Gln Pro Glu
65                  70                  75                  80

Asp Ile Ala Thr Tyr Tyr Cys Gln Gln Arg Ser Ser Tyr Pro Tyr Thr
                85                  90                  95

Phe Gly Gly Gly Thr Lys Val Glu Ile Lys Arg Thr
            100                 105

<210> 25
<211> 108
<212> PRT
<213> Artificial Sequence

<220>
<223> Description of Artificial Sequence: Amino Acid Sequence of Antibody Light Chain Variable Region

<400> 25
Asp Ile Gln Met Thr Gln Ser Pro Ser Ser Leu Ser Ala Ser Val Gly
1 5 10 15

Asp Arg Val Thr Ile Thr Cys Ser Ala Ser Ser Ser Val Ser Tyr Met
20 25 30

His Trp Tyr Gln Gln Lys Pro Gly Lys Ala Pro Lys Leu Leu Ile Tyr
35 40 45

Ser Thr Ser Asn Leu Ala Ser Gly Val Pro Ser Arg Phe Ser Gly Ser
50 55 60

Gly Ser Gly Thr Asp Phe Thr Leu Thr Ile Ser Ser Leu Gln Pro Glu
65 70 75 80

Asp Phe Ala Thr Tyr Tyr Cys Gln Gln Arg Ser Ser Tyr Pro Tyr Thr
85 90 95

Phe Gly Gln Gly Thr Lys Val Glu Ile Lys Arg Thr
100 105

<210> 26
<211> 125
<212> PRT
<213> Artificial Sequence

<220>
<223> Description of Artificial Sequence: Amino Acid Sequence of Antibody Heavy Chain Variable Region

<400> 26
Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr

20 25 30

Asn Met Asp Trp Val Lys Gln Ser Pro Gly Gln Gly Leu Glu Trp Met
35 40 45

Gly Tyr Ile Tyr Pro Asn Asn Gly Gly Thr Gly Tyr Asn Gln Lys Phe
50 55 60

Lys Ser Lys Val Thr Ile Thr Val Asp Thr Ser Thr Ser Thr Ala Tyr
65 70 75 80

Met Glu Leu His Ser Leu Arg Ser Glu Asp Thr Ala Val Tyr Tyr Cys
85 90 95

Ala Thr Tyr Gly His Tyr Tyr Gly Tyr Met Phe Ala Tyr Trp Gly Gln
100 105 110

Gly Thr Leu Val Thr Val Ser Ser Ala Ser Thr Lys Gly
115 120 125

<210> 27
<211> 125
<212> PRT
<213> Artificial Sequence

<220>
<223> Description of Artificial Sequence: Amino Acid Sequence of Antibody Heavy Chain Variable Region

<400> 27
Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Asn Met Asp Trp Val Lys Gln Ser Pro Gly Lys Ser Leu Glu Trp Met

35 40 45

Gly Tyr Ile Tyr Pro Asn Asn Gly Gly Thr Gly Tyr Asn Gln Lys Phe
50 55 60

Lys Ser Lys Val Thr Ile Thr Val Asp Thr Ser Thr Ser Thr Ala Tyr
65 70 75 80

Met Glu Leu His Ser Leu Arg Ser Glu Asp Thr Ala Val Tyr Tyr Cys
85 90 95

Ala Thr Tyr Gly His Tyr Tyr Gly Tyr Met Phe Ala Tyr Trp Gly Gln
100 105 110

Gly Thr Leu Val Thr Val Ser Ser Ala Ser Thr Lys Gly
115 120 125

<210> 28
<211> 125
<212> PRT
<213> Artificial Sequence

<220>
<223> Description of Artificial Sequence: Amino Acid Sequence of Antibody Heavy Chain Variable Region

<400> 28
Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Asn Met Asp Trp Val Arg Gln Ala Pro Gly Gln Gly Leu Glu Trp Met
35 40 45

Gly Tyr Ile Tyr Pro Asn Asn Gly Gly Thr Gly Tyr Asn Gln Lys Phe

50 55 60

Lys Ser Lys Ala Thr Leu Thr Val Asp Thr Ser Thr Ser Thr Ala Tyr
65 70 75 80

Met Glu Leu His Ser Leu Arg Ser Glu Asp Thr Ala Val Tyr Tyr Cys
85 90 95

Ala Thr Tyr Gly His Tyr Tyr Gly Tyr Met Phe Ala Tyr Trp Gly Gln
100 105 110

Gly Thr Leu Val Thr Val Ser Ser Ala Ser Thr Lys Gly
115 120 125

<210> 29
<211> 125
<212> PRT
<213> Artificial Sequence

<220>
<223> Description of Artificial Sequence: Amino Acid Sequence of Antibody Heavy Chain Variable Region

<400> 29
Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Asn Met Asp Trp Val Lys Gln Ser Pro Gly Lys Ser Leu Glu Trp Met
35 40 45

Gly Tyr Ile Tyr Pro Asn Asn Gly Gly Thr Gly Tyr Asn Gln Lys Phe
50 55 60

Lys Ser Lys Ala Thr Leu Thr Val Asp Thr Ser Thr Ser Thr Ala Tyr

65 70 75 80

Met Glu Leu His Ser Leu Arg Ser Glu Asp Thr Ala Val Tyr Tyr Cys
85 90 95

Ala Thr Tyr Gly His Tyr Tyr Gly Tyr Met Phe Ala Tyr Trp Gly Gln
100 105 110

Gly Thr Leu Val Thr Val Ser Ser Ala Ser Thr Lys Gly
115 120 125

<210> 30
<211> 125
<212> PRT
<213> Artificial Sequence

<220>
<223> Description of Artificial Sequence: Amino Acid Sequence of Antibody Heavy Chain Variable Region

<400> 30
Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Asn Met Asp Trp Val Lys Gln Ser Pro Gly Gln Gly Leu Glu Trp Met
35 40 45

Gly Tyr Ile Tyr Pro Asn Asn Gly Gly Thr Gly Tyr Asn Gln Lys Phe
50 55 60

Lys Ser Lys Ala Thr Leu Thr Val Asp Thr Ser Thr Ser Thr Ala Tyr
65 70 75 80

Met Glu Leu His Ser Leu Arg Ser Glu Asp Thr Ala Val Tyr Tyr Cys

85 90 95

Ala Thr Tyr Gly His Tyr Tyr Gly Tyr Met Phe Ala Tyr Trp Gly Gln
100 105 110

Gly Thr Leu Val Thr Val Ser Ser Ala Ser Thr Lys Gly
115 120 125

<210> 31
<211> 108
<212> PRT
<213> Artificial Sequence

<220>
<223> Description of Artificial Sequence: Amino Acid Sequence of Antibody Light Chain Variable Region

<400> 31
Asp Ile Gln Leu Thr Gln Ser Pro Ser Ser Leu Ser Ala Ser Pro Gly
1 5 10 15

Asp Arg Val Thr Ile Thr Cys Ser Ala Ser Ser Ser Val Ser Tyr Met
20 25 30

His Trp Phe Gln Gln Lys Pro Gly Lys Ala Pro Lys Leu Trp Ile Tyr
35 40 45

Ser Thr Ser Asn Leu Ala Ser Gly Val Pro Ala Arg Phe Ser Gly Ser
50 55 60

Gly Ser Gly Thr Ser Tyr Ser Leu Thr Ile Ser Arg Leu Gln Pro Glu
65 70 75 80

Asp Ile Ala Thr Tyr Tyr Cys Gln Gln Arg Ser Ser Tyr Pro Tyr Thr
85 90 95

Phe Gly Gly Gly Thr Lys Val Glu Ile Lys Arg Thr

100 105

<210> 32
<211> 108
<212> PRT
<213> Artificial Sequence

<220>
<223> Description of Artificial Sequence: Amino Acid Sequence of Antibody Light Chain Variable Region

<400> 32
Asp Ile Gln Leu Thr Gln Ser Pro Ser Ser Leu Ser Ala Ser Pro Gly
1 5 10 15

Asp Arg Val Thr Ile Thr Cys Ser Ala Ser Ser Ser Val Ser Tyr Met
20 25 30

His Trp Phe Gln Gln Lys Pro Gly Lys Ala Pro Lys Leu Trp Ile Tyr
35 40 45

Ser Thr Ser Asn Leu Ala Ser Gly Val Pro Ser Arg Phe Ser Gly Ser
50 55 60

Gly Ser Gly Thr Ser Tyr Ser Leu Thr Ile Ser Arg Leu Gln Pro Glu
65 70 75 80

Asp Ile Ala Thr Tyr Tyr Cys Gln Gln Arg Ser Ser Tyr Pro Tyr Thr
85 90 95

Phe Gly Gly Gly Thr Lys Val Glu Ile Lys Arg Thr
100 105

<210> 33
<211> 108
<212> PRT

<213> Artificial Sequence

<220>
<223> Description of Artificial Sequence: Amino Acid Sequence of Antibody Light Chain Variable Region

<400> 33

```
Asp Ile Gln Leu Thr Gln Ser Pro Ser Ser Leu Ser Ala Ser Pro Gly
 1               5                  10                  15

Asp Arg Val Thr Ile Thr Cys Ser Ala Ser Ser Ser Val Ser Tyr Met
            20                  25                  30

His Trp Phe Gln Gln Lys Pro Gly Lys Ala Pro Lys Leu Trp Ile Tyr
        35                  40                  45

Ser Thr Ser Asn Leu Ala Ser Gly Val Pro Ser Arg Phe Ser Gly Ser
    50                  55                  60

Gly Ser Gly Thr Ser Tyr Ser Phe Thr Ile Ser Ser Leu Gln Pro Glu
65                  70                  75                  80

Asp Ile Ala Thr Tyr Tyr Cys Gln Gln Arg Ser Ser Tyr Pro Tyr Thr
                85                  90                  95

Phe Gly Gly Gly Thr Lys Val Glu Ile Lys Arg Thr

            100                 105
```

<210> 34
<211> 108
<212> PRT
<213> Artificial Sequence

<220>
<223> Description of Artificial Sequence: Amino Acid Sequence of Antibody Light Chain Variable Region

<400> 34
Asp Ile Gln Leu Thr Gln Ser Pro Ser Ser Leu Ser Ala Ser Pro Gly
1 5 10 15

Asp Arg Val Thr Ile Thr Cys Ser Ala Ser Ser Ser Val Ser Tyr Met
20 25 30

His Trp Tyr Gln Gln Lys Pro Gly Lys Ala Pro Lys Leu Trp Ile Tyr
35 40 45

Ser Thr Ser Asn Leu Ala Ser Gly Val Pro Ser Arg Phe Ser Gly Ser
50 55 60

Gly Ser Gly Thr Ser Tyr Ser Leu Thr Ile Ser Arg Leu Gln Pro Glu
65 70 75 80

Asp Ile Ala Thr Tyr Tyr Cys Gln Gln Arg Ser Ser Tyr Pro Tyr Thr
85 90 95

Phe Gly Gly Gly Thr Lys Val Glu Ile Lys Arg Thr
100 105

<210> 35
<211> 108
<212> PRT
<213> Artificial Sequence

<220>
<223> Description of Artificial Sequence: Amino Acid Sequence of Antibody Light Chain Variable Region

<400> 35
Asp Ile Gln Leu Thr Gln Ser Pro Ser Ser Met Ser Ala Ser Pro Gly
1 5 10 15

Asp Arg Val Thr Ile Thr Cys Ser Ala Ser Ser Ser Val Ser Tyr Met
20 25 30

His Trp Phe Gln Gln Lys Pro Gly Lys Ser Pro Lys Leu Trp Ile Tyr
35 40 45

Ser Thr Ser Asn Leu Ala Ser Gly Val Pro Ser Arg Phe Ser Gly Ser
50 55 60

Gly Ser Gly Thr Ser Tyr Ser Leu Thr Ile Ser Ser Met Gln Pro Glu
65 70 75 80

Asp Phe Ala Thr Tyr Tyr Cys Gln Gln Arg Ser Ser Tyr Pro Tyr Thr
85 90 95

Phe Gly Gln Gly Thr Lys Leu Glu Ile Lys Arg Thr
100 105

<210> 36
<211> 28
<212> DNA
<213> Artificial Sequence

<220>
<223> Description of Artificial Sequence: Synthetic DNA

<400> 36
gagacttcag cccacttcaa ttattggc 28

<210> 37
<211> 25
<212> DNA
<213> Artificial Sequence

<220>
<223> Description of Artificial Sequence: Synthetic DNA

<400> 37
cttgtgtgac tcttaactct cagag 25

<210> 38
<211> 25
<212> DNA
<213> Artificial Sequence

<220>
<223> Description of Artificial Sequence: Synthetic DNA

<400> 38
gaggccactt gtgtagcgcc aagtg 25

<210> 39
<211> 23
<212> DNA
<213> Artificial Sequence

<220>
<223> Description of Artificial Sequence: Synthetic DNA

<400> 39
ccctcgagat aacttcgtat agc 23

<210> 40
<211> 18
<212> DNA
<213> Artificial Sequence

<220>
<223> Description of Artificial Sequence : Synthetic DNA

<400> 40

ggtaggcctc actaactg 18

<210> 41
<211> 25
<212> DNA
<213> Artificial Sequence

<220>
<223> Description of Artificial Sequence : Synthetic DNA

<400> 41
catagaaaca agtaacaaca gccag 25

<210> 42
<211> 21
<212> DNA
<213> Artificial Sequence

<220>
<223> Description of Artificial Sequence: Synthetic DNA

<400> 42
gtgagtccat ggctgtcact g 21

<210> 43
<211> 20
<212> DNA
<213> Artificial Sequence

<220>
<223> Description of Artificial Sequence: Synthetic DNA

<400> 43
cctgacttgg ctattctcag 20

## Description

Technical Field

The present invention relates to an antibody composition comprising a recombinant antibody molecule which specifically binds to ganglioside GM2 and has complex type N-glycoside-linked sugar chains in the Fc region, wherein the complex type N-glycoside-linked sugar chains have a structure in which fucose is not bound to N-acetylglucosamine in the reducing end in the sugar chains; a transformant which produces the antibody composition; a process for producing the antibody composition; and a pharmaceutical composition comprising the antibody composition.

Background Art

Ganglioside which is one of glycolipids containing sialic acid is a constituent of animal cell membrane, and has a sugar chain as a hydrophilic side chain and a sphingosine and fatty acid as hydrophobic side chains. It is known that kinds and expression levels of ganglioside vary depending on the cell types, organ species, animal species and the like. It is also known that the expression of ganglioside changes quantitatively and qualitatively in the process of malignant transformation of cells [*Cancer Res.,* 45, 2405 (1985)].

For example, it has been reported that gangliosides GD2, GD3, GM2 and the like which are hardly found in normal cells are expressed in neuroectodermal tumors, such as neuroblastoma, small cell lung cancer and melanoma, which are considered to have a high grade of malignancy [*Cancer Res.,* 45, 2405 (1985), *J. Exp. Med.,* 155, 1133 (1982), *J. Biol. Chem.,* 257, 12752 (1982), *Cancer Res.,* 47, 225 (1987), *Cancer Res.,* 47, 1098 (1987), *Cancer Res.,* 45, 2642 (1985), *Proc. Natl. Acad. Sci. U.S.A,* 80, 5392 (1983)]. It is considered that antibodies against such ganglioside specific for tumor cells are useful for treatments of various human cancers.

It is generally known that when an antibody derived from a non-human animal is administered to human, it is recognized as a foreign substance, whereby side effects are induced [*J. Clin. Oncol.,* 2, 881 (1984), *Blood,* 65, 1349 (1985), *J. Natl. Cancer Inst.,* 80, 932 (1988), *Proc. Natl. Acad. Sci. U.S.A.,* 82, 1242 (1985)], disappearance of the antibody from the body is accelerated [*Blood,* 65, 1349 (1985), *J. Nucl. Med.,* 26, 1011 (1985), *J. Natl. Cancer Inst.,* 80, 937 (1988)] and thereby the therapeutic effects of the antibody is reduced [*J. Immunol.,* 135, 1530 (1985), *Cancer Res.,* 46, 6489 (1986)].

In order to solve these problems, attempts have been made to convert an antibody derived from a non-human animal into a humanized antibody such as a human chimeric antibody or a human CDR-grafted antibody utilizing recombinant DNA techniques [*Nature,* 321, 522 (1986)]. Humanized antibodies are reported to have reduced immunogenicity [*Proc. Natl. Acad Sci. U.S.A.,* 86, 4220 (1989)] and have prolonged therapeutic effects [*Cancer Res.,* 56, 1118 (1996), *Immunol.,* 85, 668 (1995)], as compared with antibodies derived from non-human animals.

It is shown that a humanized antibody against ganglioside GM2 is useful for treatment of human melanoma [*Lancet,* 1, 786 (1989)]. As humanized antibodies which specifically react with ganglioside GM2 and have cytotoxic activity such as antibody-dependent cell-mediated cytotoxic activity (hereinafter referred to as ADCC activity) or complement-dependent cytotoxic activity (hereinafter referred to as CDC activity), human chimeric antibodies and human CDR-grafted antibodies belonging to human IgG class have been obtained (WO00/61739, WO02/31140)

Also, since humanized antibodies are prepared utilizing recombinant DNA techniques, they can be prepared as molecules in various forms. For example, a humanized antibody having high effector function can be prepared [*Cancer Res.,* 56, 1118 (1996)].

In recent years, in the treatment of non Hodgkin's leukemia patients by Rituxan and the treatment of mammary cancer patients by Herceptin, when an antibody preparation induces high ADCC activity in effector cells of the patients, higher therapeutic effects can be obtained [*Blood,* 99, 754 (2002); *J. Clin. Oncol.,* 21, 3940 (2003); *Clin. Cancer Res.,* 10, 5650 (2004)].

Antibodies of the human IgG1 subclass express ADCC activity and CDC activity via the Fc region thereof and antibody receptors (hereinafter referred to as FcγR) or various complement components. It is suggested that in the binding of an antibody to FcγR, the hinge region of the antibody and a sugar chain bound to the second domain of the C region (hereinafter referred to as Cγ2 domain) are important [*Chemical Immunology,* 65, 88 (1997)].

It is known that there is diversity regarding the addition of galactose to the non-reducing end in a complex type N-glycoside-linked sugar chain bound to the Fc region of an IgG antibody molecule and the addition of fucose to N-acetylglucosamine at the reducing end [*Biochemistry,* 36, 130 (1997)]. In particular, it is reported that the addition of fucose to the N-acetylglucosamine in the reducing end in the sugar chain causes significant decrease of the ADCC activity of the antibody [WO00/61739, *J. Biol. Chem.,* 278, 3466 (2003)].

In general, most of the antibody compositions utilized as pharmaceutical compositions are prepared by recombinant DNA techniques using animal cells such as Chinese hamster ovary tissue-derived CHO cells as host cells, and the sugar chain structure of the expressed antibody compositions differs depending on host cells. Accordingly, in order

to offer high-quality medical services to patients, an antibody composition to which a sugar chain is added so as to exert suitable pharmacological activity must be appropriately prepared and provided.

It is possible to increase the ratio of sugar chains having a structure in which fucose is not bound to N-acetylglucosamine in the reducing end among the total complex type N-glycoside-linked sugar chains bound to the Fc region of antibody molecules in an antibody composition comprising antibody molecules having Fc region by decreasing or deleting the activity of α1,6-fucosyltransferase (FUT8), GDP-mannose 4,6-dehydratase (GMD) or GDP-4-keto-6-deoxy-D-mannose-3,5-epimerase (Fx) of antibody-producing cells (WO02/31140).

## Disclosure of the Invention

An object of the present invention is to provide an antibody composition comprising a recombinant antibody molecule which specifically binds to ganglioside GM2 and has complex type N-glycoside-linked sugar chains in the Fc region, wherein the complex type N-glycoside-linked sugar chains have a structure in which fucose is not bound to N-acetylglucosamine in the reducing end in the sugar chains; a transformant which produces the antibody composition; a process for producing the antibody composition; and a pharmaceutical composition comprising the antibody composition. Since the anti-ganglioside GM2 antibody composition of the present invention has high cytotoxic activity, it is effective for treatment to decrease ganglioside GM2-expressing cells from patients. The therapeutic use of the antibody having high cytotoxic activity is also expected to be effective for weakening side effects of patients because it does not require combination use of chemotherapeutic agents or radioisotope-labeled antibodies. Furthermore, reduction of burden on patients and the like are expected by decreasing the dose of the therapeutic agent to patients.

## Means to Solve the Problems

The present invention relates to the following (1) to (48).

(1) An antibody composition comprising a recombinant antibody molecule which specifically binds to ganglioside GM2 and has complex type N-glycoside-linked sugar chains in the Fc region, wherein the complex type N-glycoside-linked sugar chains have a structure in which fucose is not bound to N-acetylglucosamine in the reducing end in the sugar chains.

(2) The antibody composition according to (1), wherein the complex type N-glycoside-linked sugar chains are sugar chains in which 1-position of fucose is not bound to 6-position of N-acetylglucosamine in the reducing end through α-bond in the sugar chains.

(3) The antibody composition according to (1) or (2), which specifically binds to a ganglioside GM2-expressing cell.

(4) The antibody composition according to any one of (1) to (3), which has cytotoxic activity against a ganglioside GM2-expressing cell.

(5) The antibody composition according to any one of (1) to (4), which has higher cytotoxic activity against a ganglioside GM2-expressing cell than a monoclonal antibody produced by a non-human animal-derived hybridoma.

(6) The antibody composition according to (4) or (5), wherein the cytotoxic activity is antibody-dependent cell-mediated cytotoxic (ADCC) activity.

(7) The antibody composition according to (4) or (5), wherein the cytotoxic activity is complement-dependent cytotoxic (CDC) activity.

(8) The antibody composition according to any one of (1) to (7), which comprises complementarity determining region (CDR) 1, CDR 2 and CDR 3 of antibody molecule heavy chain (H chain) variable region (V region) consisting of the amino acid sequences represented by SEQ ID NOs:14, 15 and 16, respectively.

(9) The antibody composition according to any one of (1) to (7), which comprises complementarity determining region (CDR) 1, CDR 2 and CDR 3 of antibody molecule light chain (L chain) variable region (V region) consisting of the amino acid sequences represented by SEQ ID NOs:17, 18 and 19, respectively.

(10) The antibody composition according to any one of (1) to (9), which comprises complementarity determining region (CDR) 1, CDR 2 and CDR 3 of an antibody molecule heavy chain (H chain) variable region (V region) consisting of the amino acid sequences represented by SEQ ID NOs:14, 15 and 16, respectively; and CDR 1, CDR 2 and CDR 3 of antibody molecule light chain (L chain) V region consisting of the amino acid sequences represented by SEQ ID NOs:17, 18 and 19, respectively.

(11) The antibody composition according to any one of (1) to (10), wherein the recombinant antibody is a human chimeric antibody or a human CDR-grafted antibody.

(12) The antibody composition according to (11), wherein the human chimeric antibody comprises complementarity determining regions (CDRs) of heavy chain (H chain) variable region (V region) and light chain (L chain) V region of a monoclonal antibody which specifically binds to ganglioside GM2.

(13) The antibody composition according to (12), wherein the heavy chain (H chain) variable region (V region) of

the antibody molecule comprises the amino acid sequence represented by SEQ ID NO:20.
(14) The antibody composition according to (12), wherein the light chain (L chain) variable region (V region) of the antibody molecule comprises the amino acid sequence represented by SEQ ID NO:21.
(15) The human chimeric antibody composition according to any one of (12) to (14), wherein the heavy chain (H chain) variable region (V region) of the antibody molecule comprises the amino acid sequence represented by SEQ ID NO:20; and the light chain (L chain) V region of the antibody molecule comprises the amino acid sequence represented by SEQ ID NO:21.
(16) The antibody composition according to (11), wherein the human CDR-grafted antibody comprises complementarity determining regions (CDRs) of heavy chain (H chain) variable region (V region) and light chain (L chain) V region of a monoclonal antibody which specifically binds to ganglioside GM2.
(17) The antibody composition according to (16), which comprises complementarity determining region (CDRs) of heavy chain (H chain) variable region (V region) and light chain (L chain) V region of a monoclonal antibody which specifically binds to ganglioside GM2, and framework regions (FRs) of H chain V region and L chain V region of a human antibody.
(18) The antibody composition according to (16) or (17), which comprises complementarity determining region (CDRs) of heavy chain (H chain) variable region (V region) and light chain (L chain) V region of a monoclonal antibody which specifically binds to ganglioside GM2, framework regions (FRs) of H chain V region and L chain V region of a human antibody, and H chain constant region (C region) and L chain C region of a human antibody.
(19) The antibody composition according to any one of (16) to (18), wherein the heavy chain (H chain) variable region (V region) of the antibody molecule comprises the amino acid sequence represented by SEQ ID NO:22 or an amino acid sequence in which at least one amino acid residue selected from the group consisting of Arg at position 38, Ala at position 40, Gln at position 43 and Gly at position 44 is substituted with another amino acid residue in the amino acid sequence represented by SEQ ID NO:22.
(20) The antibody composition according to any one of (16) to (18), wherein the heavy chain (H chain) variable region (V region) of the antibody molecule comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23.
(21) The antibody composition according to any one of (16) to (18), wherein the light chain (L chain) variable region (V region) of the antibody molecule comprises the amino acid sequence represented by SEQ ID NO:24 or an amino acid sequence in which at least one amino acid residue selected from the group consisting of Val at position 15, Tyr at position 35, Leu at position 46, Ser at position 59, Asp at position 69, Phe at position 70, Thr at position 71, Phe at position 72 and Ser at position 76 is substituted with another amino acid residue in the amino acid sequence represented by SEQ ID NO:24.
(22) The antibody composition according to any one of (16) to (18), wherein the light chain (L chain) variable region (V region) of the antibody molecule comprises the amino acid sequence represented by SEQ ID NO:25 or an amino acid sequence in which at least one amino acid residue selected from the group consisting of Met at position 4, Leu at position 11, Val at position 15, Tyr at position 35, Ala at position 42, Leu at position 46, Asp at position 69, Phe at position 70, Thr at position 71, Leu at position 77 and Val at position 103 is substituted with another amino acid residue in the amino acid sequence represented by SEQ ID NO:25.
(23) The antibody composition according to any one of (16) to (19) or (21), wherein the heavy chain (H chain) variable region (V region) of the antibody molecule comprises the amino acid sequence represented by SEQ ID NO:22 or an amino acid sequence in which at least one amino acid residue selected from the group consisting of Arg at position 38, Ala at position 40, Gln at position 43 and Gly at position 44 is substituted with another amino acid residue in the amino acid sequence represented by SEQ ID NO:22; and the light chain (L chain) V region of the antibody molecule comprises the amino acid sequence represented by SEQ ID NO:24 or an amino acid sequence in which at least one amino acid residue selected from the group consisting of Val at position 15, Tyr at position 35, Leu at position 46, Ser at position 59, Asp at position 69, Phe at position 70, Thr at position 71, Phe at position 72 and Ser at position 76 is substituted with another amino acid residue in the amino acid sequence represented by SEQ ID NO:24.
(24) The antibody composition according to any one of (16) to (18), (20) or (21), wherein the heavy chain (H chain) variable region (V region) of the antibody molecule comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23; and the light chain (L chain) V region of the antibody molecule comprises the amino acid sequence represented by SEQ ID NO:24 or an amino acid sequence in which at least one amino acid residue selected from the group consisting of Val at position 15, Tyr at position 35, Leu at position 46, Ser at position 59, Asp at position 69, Phe at position 70, Thr at position 71, Phe at position 72

and Ser at position 76 is substituted with another amino acid residue in the amino acid sequence represented by SEQ ID NO:24.

(25) The antibody composition according to any one of (16) to (18), (20) or (22), wherein the heavy chain (H chain) variable region (V region) of the antibody molecule comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23; and the light chain (L chain) V region of the antibody molecule comprises the amino acid sequence represented by SEQ ID NO:25 or an amino acid sequence in which at least one amino acid residue selected from the group consisting of Met at position 4, Leu at position 11, Val at position 15, Tyr at position 35, Ala at position 42, Leu at position 46, Asp at position 69, Phe at position 70, Thr at position 71, Leu at position 77 and Val at position 103 is substituted with another amino acid residue in the amino acid sequence represented by SEQ ID NO:25.

(26) The antibody composition according to any one of (16) to (20) or (23) to (25), wherein the heavy chain (H chain) variable region (V region) of the antibody molecule comprises an amino acid sequence selected from the group consisting of the amino acid sequences represented by SEQ ID NOs:22, 23, 26, 27, 28, 29 and 30.

(27) The antibody composition according to any one of (16) to (18) or (21) to (25), wherein the light (L chain) variable region (V region) of the antibody molecule comprises an amino acid sequence selected from the group consisting of the amino acid sequences represented by SEQ ID NOs:24, 25, 31, 32, 33, 34 and 35.

(28) The antibody composition according to any one of (16) to (27), wherein the heavy chain (H chain) variable region (V region) of the antibody molecule comprises an amino acid sequence selected from the group consisting of the amino acid sequences represented by SEQ ID NOs:22, 23, 26, 27, 28, 29 and 30; and the light chain (L chain) V region of the antibody molecule comprises an amino acid sequence selected from the group consisting of the amino acid sequences represented by SEQ ID NOs:24, 25, 31, 32, 33, 34 and 35.

(29) The antibody composition according to any one of (16) to (19), (21), (23) or (26) to (28), wherein the heavy chain (H chain) variable region (V region) of the antibody molecule comprises the amino acid sequence represented by SEQ ID NO:26; and the light chain (L chain) V region of the antibody molecule comprises the amino acid sequence represented by SEQ ID NO: 31 or 32.

(30) The antibody composition according to any one of (16) to (19), (21) to (23) or (26) to (28), wherein the heavy chain (H chain) variable region (V region) of the antibody molecule comprises the amino acid sequence represented by SEQ ID NO:22; and the light chain (L chain) V region of the antibody molecule comprises the amino acid sequence represented by SEQ ID NO:32 or 35.

(31) A transformant producing the antibody composition according to any one of (1) to (30), which is obtainable by introducing a DNA encoding an antibody molecule which specifically binds to ganglioside GM2 into a host cell.

(32) The transformant according to (31), wherein the host cell is a cell in which genome is modified so as to have deleted activity of an enzyme relating to the synthesis of an intracellular sugar nucleotide, GDP-fucose, or an enzyme relating to the modification of a sugar chain in which 1-position of fucose is bound to 6-position of N-acetylglucosamine in the reducing end through α-bond in a complex type N-glycoside-linked sugar chain.

(33) The transformant according to (31), wherein the host cell is a cell in which all of alleles on a genome encoding an enzyme relating to the synthesis of an intracellular sugar nucleotide, GDP-fucose, or an enzyme relating to the modification of a sugar chain in which 1-position of fucose is bound to 6-position of N-acetylglucosamine in the reducing end through α-bond in a complex type N-glycoside-linked sugar chain are knocked out.

(34) The transformant according to (32) or (33), wherein the enzyme relating to the synthesis of an intracellular sugar nucleotide, GDP-fucose, is an enzyme selected from GDP-mannose 4,6-dehydratase (GMD) or GDP-4-keto-6-deoxy-D-mannose-3,5-epimerase (Fx).

(35) The transformant according to (34), wherein the GDP-mannose 4,6-dehydratase is a protein encoded by a DNA selected from the group consisting of the following (a) and (b):

(a) a DNA comprising the nucleotide sequence represented by SEQ ID NO:1;
(b) a DNA which hybridizes with the DNA consisting of the nucleotide sequence represented by SEQ ID NO: 1 under stringent conditions and which encodes a protein having GDP-mannose 4,6-dehydratase activity.

(36) The transformant according to (34), wherein the GDP-mannose 4,6-dehydratase is a protein selected from the group consisting of the following (a) to (c):

(a) a protein comprising the amino acid sequence represented by SEQ ID NO:2;
(b) a protein consisting of an amino acid sequence wherein one or more amino acid residue(s) is/are deleted, substituted, inserted and/or added in the amino acid sequence represented by SEQ ID NO:2 and having GDP-mannose 4,6-dehydratase activity;

(c) a protein consisting of an amino acid sequence which has 80% or more homology to the amino acid sequence represented by SEQ ID NO:2 and having GDP-mannose 4,6-dehydratase activity.

(37) The transformant according to (34), wherein the GDP-4-keto-6-deoxy-D-mannose-3,5-epimerase is a protein encoded by a DNA selected from the group consisting of the following (a) and (b):

(a) a DNA comprising the nucleotide sequence represented by SEQ ID NO:3;
(b) a DNA which hybridizes with the DNA consisting of the nucleotide sequence represented by SEQ ID NO:3 under stringent conditions and which encodes a protein having GDP-4-keto-6-deoxy-D-mannose-3,5-epimerase activity.

(38) The transformant according to (34), wherein the GDP-4-keto-6-deoxy-D-mannose-3,5-epimerase is a protein selected from the group consisting of the following (a) to (c):

(a) a protein comprising the amino acid sequence represented by SEQ ID NO:4;
(b) a protein consisting of an amino acid sequence wherein one or more amino acid residue(s) is/are deleted, substituted, inserted and/or added in the amino acid sequence represented by SEQ ID NO:4 and having GDP-4-keto-6-deoxy-D-mannose-3,5-epimerase activity;
(c) a protein consisting of an amino acid sequence which has 80% or more homology to the amino acid sequence represented by SEQ ID NO:4 and having GDP-4-keto-6-deoxy-D-mannose-3,5-epimerase activity.

(39) The transformant according to (32) or (33), wherein the enzyme relating to the modification of a sugar chain in which 1-position of fucose is bound to 6-position of N-acetylglucosamine in the reducing end through α-bond in a complex type N-glycoside-linked sugar chain is α1,6-fucosyltransferase.
(40) The transformant according to (39), wherein the α1,6-fucosyltransferase is a protein encoded by a DNA selected from the group consisting of the following (a) to (d):

(a) a DNA comprising the nucleotide sequence represented by SEQ ID NO:5;
(b) a DNA comprising the nucleotide sequence represented by SEQ ID NO:6;
(c) a DNA which hybridizes with the DNA consisting of the nucleotide sequence represented by SEQ ID NO:5 under stringent conditions and which encodes a protein having α1,6-fucosyltransferase activity;
(d) a DNA which hybridizes with the DNA consisting of the nucleotide sequence represented by SEQ ID NO:6 under stringent conditions and which encodes a protein having α1,6-fucosyltransferase activity.

(41) The transformant according to (39), wherein the α1,6-fucosyltransferase is a protein selected from the group consisting of the following (a) to (f):

(a) a protein comprising the amino acid sequence represented by SEQ ID NO:7;
(b) a protein comprising the amino acid sequence represented by SEQ ID NO:8;
(c) a protein consisting of an amino acid sequence wherein one or more amino acid residue(s) is/are deleted, substituted, inserted and/or added in the amino acid sequence represented by SEQ ID NO:7 and having α1,6-fucosyltransferase activity;
(d) a protein consisting of an amino acid sequence wherein one or more amino acid residue(s) is/are deleted, substituted, inserted and/or added in the amino acid sequence represented by SEQ ID NO:8 and having α1,6-fucosyltransferase activity;
(e) a protein consisting of an amino acid sequence which has 80% or more homology to the amino acid sequence represented by SEQ ID NO:7 and having α1,6-fucosyltransferase activity;
(f) a protein consisting of an amino acid sequence which has 80% or more homology to the amino acid sequence represented by SEQ ID NO:8 and having α1,6-fucosyltransferase activity.

(42) The transformant according to (41), wherein the transformant is FERM BP-8470.
(43) The transformant according to any one of (31) to (42), wherein the host cell is a cell selected from the group consisting of the following (a) to (i):

(a) a CHO cell derived from Chinese hamster ovary tissue;
(b) a rat myeloma cell line YB2/3HL.P2.G11.16Ag.20 cell;
(c) a mouse myeloma cell line NSO cell;
(d) a mouse myeloma cell line SP2/0-Ag14 cell;

(e) a BHK cell derived from Syrian hamster kidney tissue;
(f) an antibody-producing hybridoma cell;
(g) a human leukemia cell line Namalwa cell;
(h) an embryonic stem cell;
(i) a fertilized egg cell.

(44) A process for producing the antibody composition according to any one of (1) to (30), which comprises culturing the transformant according to any one of (31) to (43) in a medium to form and accumulate the antibody composition in the culture, and recovering and purifying the antibody composition from the culture.
(45) The antibody composition according to any one of (1) to (32), which is obtainable by the process according to (44).
(46) A pharmaceutical composition comprising the antibody composition according to any one of (1) to (30) and (45) as an active ingredient.
(47) A therapeutic agent for diseases relating to a ganglioside GM2, comprising the antibody composition according to any one of (1) to (30) and (45) as an active ingredient.
(48) The therapeutic agent according to (47), wherein the diseases relating to a ganglioside GM2 are cancer.

The present invention is described below in detail. This application is based on the priorities of Japanese patent application No. 2003-350168 filed on October 9, 2003 and Japanese patent application No. 2004-129431 filed on April 26, 2004, and the entire contents of the specification and the drawings in the patent application are incorporated hereinto by reference.

Best Mode for Carrying Out the Invention

An example of the antibody composition of the present invention comprising a recombinant antibody molecule which specifically binds to ganglioside GM2 and has complex type N-glycoside-linked sugar chains in the Fc region, wherein the complex type N-glycoside-linked sugar chains have a structure in which fucose is not bound to N-acetylglucosamine in the reducing end in the sugar chains, is an antibody composition wherein the complex type N-glycoside linked sugar chains have a structure in which 1-position of fucose is not bound to 6-position of N-acetylglucosamine in the reducing end through α-bond.
An antibody molecule has the Fc region, to which N-glycoside-linked sugar chains are bound. Therefore, two sugar chains are bound to one antibody molecule.
The N-glycoside-linked sugar chains include complex type sugar chains having one or multiple number of parallel galactose-N-acetylglucosamine (hereinafter referred to as Gal-GlcNAc) side chains in the non-reducing end of the core structure and having sialic acid, bisecting N-acetylglucosamine or the like in the non-reducing end of Gal-GlcNAc.
In the present invention, the complex type N-glycoside-linked sugar chain is represented by the following chemical formula 1.

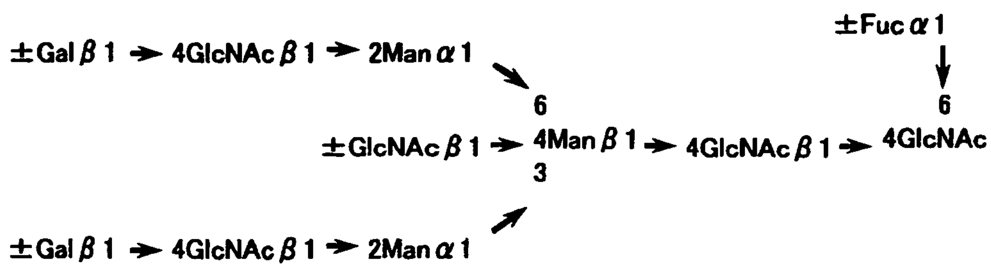

In the present invention, the sugar chain to which fucose is not bound includes a sugar chain represented by the above chemical formula in which fucose is not bound to N-acetylglucosamine in the reducing end. The sugar chain in the non-reducing end may have any structure.
Accordingly, the antibody composition of the present invention comprises an antibody molecule having the same sugar chain structure or antibody molecules having different sugar chain structures, so long as the antibody composition has the above sugar chain structure.
The expression "fucose is not bound to the N-acetylglucosamine in the reducing end in the sugar chains" as used herein means that fucose is not substantially bound thereto. The "antibody composition in which fucose is not

substantially bound" specifically refers to an antibody composition in which fucose is not substantially detected, i.e., the content of fucose is below the detection limit, when subjected to the sugar chain analysis described in 4 below. The antibody composition of the present invention in which fucose is not bound to the N-acetylglucosamine in the reducing end in the sugar chains has high ADCC activity.

The ratio of an antibody molecule having sugar chains in which fucose is not bound to the N-acetylglucosamine in the reducing end in an antibody composition comprising an antibody molecule having complex type N-glycoside-linked sugar chains in the Fc region can be determined by releasing the sugar chains from the antibody molecule by known methods such as hydrazinolysis and enzyme digestion [*Seibutsukagaku Jikkenho (Biochemical Experimentation Methods)* 23 - *Totanpakushitsu Tosa Kenkyuho (Methods of Studies on Glycoprotein Sugar Chains),* Gakkai Shuppan Center, edited by Reiko Takahashi (1989)], labeling the released sugar chains with a fluorescent substance or radioisotope, and separating the labeled sugar chains by chromatography. Alternatively, the released sugar chains may be analyzed by the HPAED-PAD method [*J. Liq. Chromatogr.,* 6, 1577 (1983)] to determine the ratio.

As the antibody composition of the present invention, an antibody composition having cytotoxic activity against ganglioside GM2-expressing cells is preferred.

The ganglioside GM2-expressing cells may be any cells, so long as they express ganglioside GM2.

The cytotoxic activity includes complement-dependent cytotoxic activity (hereinafter referred to as CDC activity), antibody-dependent cell-mediated cytotoxic activity (hereinafter referred to as ADCC activity), and the like.

The antibody composition having cytotoxic activity against ganglioside GM2-expressing cells injures ganglioside GM2-expressing cells by the cytotoxic activity possessed by the antibody composition to thereby treat diseases relating to the cells, such as small cell lung cancer, melanoma and neuroblastoma.

The antibody compositions of the present invention include compositions of human chimeric antibodies, compositions of human CDR-grafted antibodies, compositions of human antibodies and compositions of fragments of such antibodies.

The human chimeric antibody refers to an antibody comprising VH and VL of an antibody derived from a non-human animal, and CH and CL of a human antibody. As the non-human animal, any animal can be used, so long as hybridomas can be prepared from the animal. Suitable animals include mouse, rat, hamster, rabbit and the like.

The human chimeric antibody composition of the present invention can be produced by obtaining cDNAs encoding VH and VL of a non-human animal-derived antibody which specifically binds to ganglioside GM2, inserting the cDNAs into an expression vector for animal cells which carries genes encoding CH and CL of a human antibody to construct a human chimeric antibody expression vector, and introducing the vector into an animal cell to induce expression.

Examples of the antibody derived from a non-human animal used for producing the human chimeric antibody composition of the present invention include mouse monoclonal antibody KM690, mouse monoclonal antibody KM750 and mouse monoclonal antibody KM796 described in Japanese Published Unexamined Patent Application No. 311385/92, monoclonal antibody MoAb5-3 described in *Cancer Res.,* 46, 4116 (1986), monoclonal antibody MK1-16 and monoclonal antibody MK2-34 described in *Cancer Res.,* 48, 6154 (1988), monoclonal antibody DMAb-1 described in *J. Biol. Chem.,* 264, 12122 (1989) and the like. Also, as a human antibody, a monoclonal antibody belonging to IgM class described in *Proc. Natl. Acad. Sci. U.S.A,* 79, 7629 (1982) and the like can also be used for producing the human chimeric antibody composition of the present invention.

As the CH for the human chimeric antibody, any CH of antibodies belonging to human immunoglobulin (hereinafter referred to as hIg) may be used. Preferred are those of antibodies belonging to the hIgG class, which may be of any subclass, e.g., hIgG1, hIgG2, hIgG3 and hIgG4. As the CL for the human chimeric antibody, any CL of antibodies belonging to hIg, such as class $\kappa$ or class $\lambda$, may be used.

Examples of the human chimeric antibody composition of the present invention which specifically binds to ganglioside GM2 include: an anti-ganglioside GM2 chimeric antibody comprising CDR1, CDR2 and CDR3 of VH consisting of the amino acid sequences represented by SEQ ID NOs:14, 15 and 16, respectively, and/or CDR1, CDR2 and CDR3 of VL consisting of the amino acid sequences represented by SEQ ID NOs:17, 18 and 19, respectively; an anti-ganglioside GM2 chimeric antibody composition wherein the VH of the antibody comprises the amino acid sequence represented by SEQ ID NO:20 and/or the VL of the antibody comprises the amino acid sequence represented by SEQ ID NO:21; an anti-ganglioside GM2 chimeric antibody composition wherein the VH of the antibody consists of the amino acid sequence represented by SEQ ID NO:20, the CH of the human antibody consists of an amino acid sequence of the hIgG1 subclass, the VL of the antibody consists of the amino acid sequence represented by SEQ ID NO:21, and the CL of the human antibody consists of an amino acid sequence of the $\kappa$ class; and the like.

The amino acid sequence of the human chimeric antibody composition of the present invention which specifically binds to ganglioside GM2 includes the amino acid sequence of KM966 described in WO00/61739.

The human CDR-grafted antibody refers to an antibody in which CDRs of VH and VL of an antibody derived from a non-human animal are grafted into appropriate sites in VH and VL of a human antibody.

The human CDR-grafted antibody composition of the present invention can be produced by constructing cDNAs

encoding V regions in which CDRs of VH and VL of a non-human animal-derived antibody which specifically reacts with ganglioside GM2 are grafted into FRs of VH and VL of an arbitrary human antibody, inserting the resulting cDNAs into an expression vector for animal cells which has DNAs encoding the heavy chain constant region (hereinafter referred to as CH) and the light chain constant region (hereinafter referred to as CL) of a human antibody to construct a human CDR-grafted antibody expression vector, and introducing the expression vector into an animal cell to induce expression.

The antibody derived from a non-human animal used for preparing the human CDR-grafted antibody composition of the present invention includes mouse monoclonal antibody KM690, mouse monoclonal antibody KM750 and mouse monoclonal antibody KM796 described in Japanese Published Unexamined Patent Application No. 311385/92, monoclonal antibody MoAb5-3 described in *Cancer Res.,* 46, 4116 (1986), monoclonal antibody MK1-16 and monoclonal antibody MK2-34 described in *Cancer Res.,* 48, 6154 (1988), monoclonal antibody DB4Ab- described in *J. Biol. Chem.,* 264, 12122 (1989) and the like. Also, as a human antibody, a monoclonal antibody belonging to IgM class described in *Proc. Natl. Acad Sci. U.S.A,* 79, 7629 (1982) and the like can also be used for preparing the human CDR-grafted antibody composition of the present invention.

As the FR amino acid sequences of VH and VL of a human antibody, any of those derived from human antibodies can be used. Suitable sequences include the FR amino acid sequences of VH and VL of human antibodies registered in databases such as Protein Data Bank, and the amino acid sequences common to all FR subgroups of VH and VL of human antibodies [*Sequences of Proteins of Immunological Interest,* US Dept. Health and Human Services (1991)].

As the CH for the human CDR-grafted antibody of the present invention, any CH of antibodies belonging to human immunoglobulin (hereinafter referred to as hIg) may be used. Preferred are those of antibodies belonging to the hIgG class, which may be of any subclass, e.g., hIgG1, hIgG2, hIgG3 and hIgG4. As the CL for the human CDR-grafted antibody, any CL of antibodies belonging to hIg, e.g., class κ or class λ, may be used.

An example of the human CDR-grafted antibody composition of the present invention is a human CDR-grafted antibody composition comprising CDRs of VH and VL of an antibody derived from a non-human animal which specifically reacts with ganglioside GM2, and preferably a human CDR-grafted antibody composition or antibody fragment composition comprising CDR1, CDR2 and CDR3 of VH consisting of the amino acid sequences represented by SEQ ID NOs: 14, 15 and 16, respectively, and/or CDR1, CDR2 and CDR3 of VL consisting of the amino acid sequences represented by SEQ ID NOs:17, 18 and 19, respectively.

Among these human CDR-grafted antibody compositions, preferred human CDR-grafted antibody compositions include: a human CDR-grafted antibody composition, wherein the VH of the antibody comprises the amino acid sequence represented by SEQ ID NO:22 or an amino acid sequence in which at least one amino acid residue selected from the group consisting of Arg at position 38, Ala at position 40, Gln at position 43 and Gly at position 44 is substituted with another amino acid residue in the amino acid sequence represented by SEQ ID NO:22; a human CDR-grafted antibody composition, wherein the VH of the antibody comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23; a human CDR-grafted antibody composition, wherein the VL of the antibody comprises the amino acid sequence represented by SEQ ID NO:24 or an amino acid sequence in which at least one amino acid residue selected from the group consisting of Val at position 15, Tyr at position 35, Leu at position 46, Ser at position 59, Asp at position 69, Phe at position 70, Thr at position 71, Phe at position 72 and Ser at position 76 is substituted with another amino acid residue in the amino acid sequence represented by SEQ ID NO:24; and a human CDR-grafted antibody composition, wherein the VL of the antibody comprises the amino acid sequence represented by SEQ ID NO:25 or an amino acid sequence in which at least one amino acid residue selected from the group consisting of Met at position 4, Leu at position 11, Val at position 15, Tyr at position 35, Ala at position 42, Leu at position 46, Asp at position 69, Phe at position 70, Thr at position 71, Leu at position 77 and Val at position 103 is substituted with another amino acid residue in the amino acid sequence represented by SEQ ID NO:25. More preferred are the following antibody compositions: a human CDR-grafted antibody composition, wherein the VH of the antibody comprises the amino acid sequence represented by SEQ ID NO:22 or an amino acid sequence in which at least one amino acid residue selected from the group consisting of Arg at position 38, Ala at position 40, Gln at position 43 and Gly at position 44 is substituted with another amino acid residue in the amino acid sequence represented by SEQ ID NO:22, and the VL of the antibody comprises the amino acid sequence represented by SEQ ID NO:24 or an amino acid sequence in which at least one amino acid residue selected from the group consisting of Val at position 15, Tyr at position 35, Leu at position 46, Ser at position 59, Asp at position 69, Phe at position 70, Thr at position 71, Phe at position 72 and Ser at position 76 is substituted with another amino acid residue in the amino acid sequence represented by SEQ ID NO: 24; a human CDR-grafted antibody composition, wherein the VH of the antibody comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23, and the VL of the antibody comprises the amino acid sequence represented by SEQ ID NO:24 or an amino acid sequence in which at least one

amino acid residue selected from the group consisting of Val at position 15, Tyr at position 35, Leu at position 46, Ser at position 59, Asp at position 69, Phe at position 70, Thr at position 71, Phe at position 72 and Ser at position 76 is substituted with another amino acid residue in the amino acid sequence represented by SEQ ID NO:24; and a human CDR-grafted antibody composition, wherein the VH of the antibody comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23, and the VL of the antibody comprises the amino acid sequence represented by SEQ ID NO:25 or an amino acid sequence in which at least one amino acid residue selected from the group consisting of Met at position 4, Leu at position 11, Val at position 15, Tyr at position 35, Ala at position 42, Leu at position 46, Asp at position 69, Phe at position 70, Thr at position 71, Leu at position 77 and Val at position 103 is substituted with another amino acid residue in the amino acid sequence represented by SEQ ID NO:25.

Specific examples of the human CDR-grafted antibody composition are a human CDR-grafted antibody composition wherein the VH of the antibody comprises an amino acid sequence selected from the group consisting of the amino acid sequences represented by SEQ ID NOs:22, 23, 26, 27, 28, 29 and 30; a human CDR-grafted antibody composition wherein the VL of the antibody comprises an amino acid sequence selected from the group consisting of the amino acid sequences represented by SEQ ID NOs:24, 25, 31, 32, 33, 34 and 35; and a human CDR-grafted antibody composition wherein the VH of the antibody comprises an amino acid sequence selected from the group consisting of the amino acid sequences represented by SEQ ID NOs:22, 23, 26, 27, 28, 29 and 30, and the VL of the antibody comprises an amino acid sequence selected from the group consisting of the amino acid sequences represented by SEQ ID NOs:24, 25, 31, 32, 33, 34 and 35. More specific examples of the human CDR-grafted antibody composition are a human CDR-grafted antibody composition wherein the VH of the antibody comprises the amino acid sequences represented by SEQ ID NO:26, and the VL of the antibody comprises the amino acid sequence represented by SEQ ID NO:31 or 32; and a human CDR-grafted antibody composition wherein the VH of the antibody comprises the amino acid sequences represented by SEQ ID NO:22, and the VL of the antibody comprises the amino acid sequence represented by SEQ ID NO:32 or 35.

The human CDR-grated antibody composition of the present invention is most preferably a human CDR-grafted antibody composition wherein the VH of the antibody comprises the amino acid sequence represented by SEQ ID NO: 26, and the VL of the antibody comprises the amino acid sequence represented by SEQ ID NO:31; or a human CDR-grafted antibody composition wherein the VH of the antibody comprises the amino acid sequence represented by SEQ ID NO:22, and the VL of the antibody comprises the amino acid sequence represented by SEQ ID NO:32.

Examples of the amino acid sequence of the human CDR-grafted antibody composition of the present invention include amino acid sequences of KM8966 produced by transformant KM8966 (FERM BP-5105), KM8967 produced by transformant KM8967 (FERM BP-5106), KM8969 produced by transformant KM8969 (FERM BP-5527) and KM8970 produced by transformant KM8970 (FERM BP-5528) each described in Japanese Published Unexamined Patent Application No. 257893/98 and the like.

Also included within the scope of the present invention are antibodies and antibody fragments which specifically bind to ganglioside GM2, and consist of amino acid sequences wherein one or more amino acid residue(s) is/are deleted, added, substituted and/or inserted in the above amino acid sequences.

The number of amino acid residues which are deleted, substituted, inserted and/or added is one or more and is not specifically limited, but it is within the range where deletion, substitution or addition is possible by known methods such as site-directed mutagenesis described in *Molecular Cloning,* Second Edition; *Current Protocols in Molecular Biology; Nucleic Acids Research,* 10, 6487 (1982); *Proc. Natl. Acad Sci. USA,* 79, 6409 (1982); *Gene,* 34, 315 (1985); *Nucleic Acids Research,* 13, 4431 (1985); *Proc. Natl. Acad Sci. USA,* 82, 488 (1985), *etc.* The suitable number is 1 to dozens, preferably 1 to 20, more preferably 1 to 10, further preferably 1 to 5.

The expression "one or more amino acid residue(s) is/are deleted, substituted, inserted or added in the amino acid sequence of the antibody composition of the present invention" means that the amino acid sequence of the antibody composition contains deletion, substitution, insertion or addition of a single or plural amino acid residues at a single or plural residues at arbitrary positions therein. Deletion, substitution, insertion or addition may be simultaneously contained in one sequence, and amino acid residues to be substituted, inserted or added may be either natural or not. Examples of the natural amino acid residues are L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine and L-cysteine.

The followings are preferred examples of the amino acid residues capable of mutual substitution. The amino acid residues in the same group shown below can be mutually substituted.

Group A: leucine, isoleucine, norleucine, valine, norvaline, alanine, 2-aminobutanoic acid, methionine, O-methylserine, t-butylglycine, t-butylalanine, cyclohexylalanine

Group B: aspartic acid, glutamic acid, isoaspartic acid, isoglutamic acid, 2-aminoadipic acid, 2-aminosuberic acid

Group C: asparagine, glutamine
Group D: lysine, arginine, ornithine, 2,4-diaminobutanoic acid, 2,3-diaminopropionic acid
Group E: proline, 3-hydroxyproline, 4-hydroxyproline
Group F: serine, threonine, homoserine
Group G: phenylalanine, tyrosine

The recombinant antibody fragment compositions of the present invention include compositions of antibody fragments which specifically bind to ganglioside GM2 and which contain a part or the whole of the antibody Fc region in which fucose is not bound to the N-acetylglucosamine in the reducing end in complex type N-glycoside-linked sugar chains.

The antibody fragment compositions of the present invention include compositions of antibody fragments, e.g., Fab, Fab', $F(ab')_2$, scFv, diabody, dsFv and a peptide comprising CDR. When the antibody fragment composition does not contain a part or the whole of the antibody Fc region, the antibody fragment may be fused with a part or the whole of the Fc region of the antibody having sugar chains in which fucose is not bound to N-acetylglucosamine in the reducing end in the complex type N-glycoside-linked sugar chains as a fusion protein, or the antibody fragment may be fused with a protein to be a fused protein composition comprising a part or the whole of the Fc region.

A Fab is an antibody fragment having a molecular weight of about 50,000 and antigen binding activity, in which about a half of the N-terminal side of H chain and the entire L chain, among fragments obtained by treating IgG with a protease, papain (cut an amino acid residue at position of 224 of the H chain), are bound together through a disulfide bond.

The Fab of the present invention can be obtained by treating the antibody composition of the present invention which specifically binds to ganglioside GM2 with the protease, papain. Alternatively, the Fab may be produced by inserting DNA encoding the Fab of the antibody into an expression vector for prokaryote or eukaryote, and introducing the vector into a prokaryote or eukaryote to induce expression.

An $F(ab')_2$ is one of the fragments obtained by treatment of IgG with the protease, pepsin (cleavage at amino acid residue at position 234 of H chain). It is an antibody fragment with a molecular weight of approximately 100,000 having antigen-binding activity, which is slightly larger than the Fab fragments linked together by a disulfide bond at the hinge region.

The $F(ab')_2$ of the present invention can be obtained by treating the antibody composition of the present invention which specifically binds to ganglioside GM2 with the protease, pepsin. Alternatively, the $F(ab')_2$ may be prepared by binding Fab' fragments described below by a thioether bond or a disulfide bond.

An Fab' is an antibody fragment with a molecular weight of approximately 50,000 having antigen-binding activity, which is obtained by cleaving the disulfide bond at the hinge region of the above $F(ab')_2$.

The Fab' of the present invention can be obtained by treating the $F(ab')_2$ composition of the present invention which specifically binds to ganglioside GM2 with a reducing agent, dithiothreitol. Alternatively, the Fab' may be produced by inserting DNA encoding the Fab' of the antibody into an expression vector for prokaryote or eukaryote, and introducing the vector into a prokaryote or eukaryote to induce expression.

An scFv is a VH-P-VL or VL-P-VH polypeptide in which one VH and one VL are linked via an appropriate peptide linker (hereinafter referred to as P) and which has antigen-binding activity.

The scFv of the present invention can be produced by obtaining cDNAs encoding the VH and VL of the antibody composition of the present invention which specifically binds to ganglioside GM2, constructing DNA encoding the scFv, inserting the DNA into an expression vector for prokaryote or eukaryote, and introducing the expression vector into a prokaryote or eukaryote to induce expression.

A diabody is an antibody fragment which is an scFv dimer showing bivalent antigen binding activity, which may be either monospecific or bispecific.

The diabody of the present invention can be produced by obtaining cDNAs encoding the VH and VL of the antibody composition of the present invention which specifically binds to ganglioside GM2, constructing DNA encoding scFv fragments with P having an amino acid sequence of 8 or less amino acid residues, inserting the DNA into an expression vector for prokaryote or eukaryote, and introducing the expression vector into a prokaryote or eukaryote to induce expression.

A dsFv is an antibody fragment wherein polypeptides in which one amino acid residue of each of VH and VL is substituted with a cysteine residue are linked via a disulfide bond between the cysteine residues. The amino acid residue to be substituted with a cysteine residue can be selected based on antibody tertiary structure estimation according to the method proposed by Reiter, *et al.* [*Protein Engineering,* 7, 697-704 (1994)].

The dsFv of the present invention can be produced by obtaining cDNAs encoding the VH and VL of the antibody composition of the present invention which specifically binds to ganglioside GM2, constructing DNA encoding the dsFv, inserting the DNA into an expression vector for prokaryote or eukaryote, and introducing the expression vector into a prokaryote or eukaryote to induce expression.

A peptide comprising CDR comprises one or more region CDR of VH or VL. A peptide comprising plural CDRs

can be prepared by binding CDRs directly or via an appropriate peptide linker.

The peptide comprising CDR of the present invention can be produced by constructing DNA encoding CDR of VH and VL of the antibody composition of the present invention which specifically binds to ganglioside GM2, inserting the DNA into an expression vector for prokaryote or eukaryote, and introducing the expression vector into a prokaryote or eukaryote to induce expression.

The peptide comprising CDR can also be produced by chemical synthesis methods such as the Fmoc method (fluorenylmethyloxycarbonyl method) and the tBoc method (t-butyloxycarbonyl method).

The transformant of the present invention includes any transformant that is obtained by introducing DNA encoding an antibody molecule which specifically binds to ganglioside GM2 into a host cell and that produces the antibody composition of the present invention. Examples of such transformants include those obtained by introducing DNA encoding an antibody molecule which specifically binds to ganglioside GM2 into host cells such as the following (a) or (b):

(a) a cell in which genome is modified so as to have deleted activity of an enzyme relating to the synthesis of an intracellular sugar nucleotide, GDP-fucose;
(b) a cell in which genome is modified so as to have deleted activity of an enzyme relating to the modification of a sugar chain in which 1-position of fucose is bound to 6-position of N-acetylglucosamine in the reducing end through $\alpha$-bond in a complex type N-glycoside-linked sugar chain.

Specifically, the "modification of genome so as to have deleted activity of an enzyme" refers to introduction of mutation into an expression regulation region of a gene encoding the enzyme so as to delete the expression of the enzyme or introduction of mutation in the amino acid sequence of a gene encoding the enzyme so as to inactivate the enzyme. The "introduction of mutation" refers to carrying out modification of the nucleotide sequence on the genome such as deletion, substitution, insertion and/or addition in the nucleotide sequence. Complete suppression of the expression or activity of the thus modified genomic gene refers to "knock out of the genomic gene".

Examples of the enzymes relating to the synthesis of the intracellular sugar nucleotide, GDP-fucose include GDP-mannose 4,6-dehydratase (GMD), GDP-4-keto-6-deoxy-D-mannose-3,5-epimerase (Fx) and the like.

Examples of the GDP-mannose 4,6-dehydratase include proteins encoded by the DNAs of the following (a) and (b):

(a) a DNA comprising the nucleotide sequence represented by SEQ ID NO:1;
(b) a DNA which hybridizes with DNA consisting of the nucleotide sequence represented by SEQ ID NO: under stringent conditions and which encodes a protein having GDP-mannose 4,6-dehydratase activity.

Examples of the GDP-mannose 4,6-dehydratase also include proteins of the following (a) to (c):

(a) a protein comprising the amino acid sequence represented by SEQ ID NO:2;
(b) a protein consisting of an amino acid sequence wherein one or more amino acid residue(s) is/are deleted, substituted, inserted and/or added in the amino acid sequence represented by SEQ ID NO:2 and having GDP-mannose 4,6-dehydratase activity;
(c) a protein consisting of an amino acid sequence which has 80% or more homology to the amino acid sequence represented by SEQ ID NO:2 and having GDP-mannose 4,6-dehydratase activity.

Examples of the GDP-4-keto-6-deoxy-D-mannose-3,5-epimerase include proteins encoded by the DNAs of the following (a) and (b):

(a) a DNA comprising the nucleotide sequence represented by SEQ ID NO:3;
(b) a DNA which hybridizes with DNA consisting of the nucleotide sequence represented by SEQ ID NO:3 under stringent conditions and which encodes a protein having GDP-4-keto-6-deoxy-D-mannose-3,5-epimerase activity.

Examples of the GDP-4-keto-6-deoxy-D-mannose-3,5-epimerase also include proteins of the following (a) to (c):

(a) a protein comprising the amino acid sequence represented by SEQ ID NO:4;
(b) a protein consisting of an amino acid sequence wherein one or more amino acid residue(s) is/are deleted, substituted, inserted and/or added in the amino acid sequence represented by SEQ ID NO:4 and having GDP-4-keto-6-deoxy-D-mannose-3,5-epimerase activity;
(c) a protein consisting of an amino acid sequence which has 80% or more homology to the amino acid sequence represented by SEQ ID NO:4 and having GDP-4-keto-6-deoxy-D-mannose-3,5-epimerase activity.

An example of the enzyme relating to the modification of a sugar chain in which 1-position of fucose is bound to 6-position of N-acetylglucosamine in the reducing end through α-bond in a complex type N-glycoside-linked sugar chain is α1,6-fucosyltransferase.

In the present invention, examples of the α1,6-fucosyltransferase include proteins encoded by the DNAs of the following (a) to (d):

(a) a DNA comprising the nucleotide sequence represented by SEQ ID NO:5;
(b) a DNA comprising the nucleotide sequence represented by SEQ ID NO:6;
(c) a DNA which hybridizes with DNA consisting of the nucleotide sequence represented by SEQ ID NO:5 under stringent conditions and which encodes a protein having α1,6-fucosyltransferase activity;
(d) a DNA which hybridizes with DNA consisting of the nucleotide sequence represented by SEQ ID NO:6 under stringent conditions and which encodes a protein having α1,6-fucosyltransferase activity, or

proteins of the following (e) to (j):

(e) a protein comprising the amino acid sequence represented by SEQ ID NO:7;
(f) a protein comprising the amino acid sequence represented by SEQ ID NO: 8;
(g) a protein consisting of an amino acid sequence wherein one or more amino acid residue(s) is/are deleted, substituted, inserted and/or added in the amino acid sequence represented by SEQ ID NO:7 and having α1,6-fucosyltransferase activity;
(h) a protein consisting of an amino acid sequence wherein one or more amino acid residue(s) is/are deleted, substituted, inserted and/or added in the amino acid sequence represented by SEQ ID NO:8 and having α1,6-fucosyltransferase activity;
(i) a protein consisting of an amino acid sequence which has 80% or more homology to the amino acid sequence represented by SEQ ID NO:7 and having α1,6-fucosyltransferase activity;
(j) a protein consisting of an amino acid sequence which has 80% or more homology to the amino acid sequence represented by SEQ ID NO:8 and having α1,6-fucosyltransferase activity.

The DNAs encoding the amino acid sequences of the enzymes relating to the synthesis of an intracellular sugar nucleotide, GDP-fucose include a DNA comprising the nucleotide sequence represented by SEQ ID NO:1 or 3, and DNA which hybridizes with a DNA comprising the nucleotide sequence represented by SEQ ID NO:1 or 3 under stringent conditions and which encodes a protein having the enzyme activity relating to the synthesis of an intracellular sugar nucleotide, GDP-fucose.

The DNAs encoding the amino acid sequences of α1,6-fucosyltransferase include a DNA comprising the nucleotide sequence represented by SEQ ID NO: 5 or 6, and a DNA which hybridizes with DNA comprising the nucleotide sequence represented by SEQ ID NO:5 or 6 under stringent conditions and which encodes a protein having α1,6-fucosyltransferase activity.

In the present invention, the DNA which hybridizes under stringent conditions refers to a DNA which is obtained by colony hybridization, plaque hybridization, Southern hybridization or the like using, for example, a DNA consisting of the nucleotide sequence represented by SEQ ID NO:1, 3, 5 or 6 or a fragment thereof as a probe. A specific example of such DNA is a DNA which can be identified by performing hybridization at 65°C in the presence of 0.7 to 1.0 M sodium chloride using a filter with colony- or plaque-derived DNA immobilized thereon, and then washing the filter at 65°C with a 0.1 to 2-fold concentration SSC solution (1-fold concentration SSC solution: 150 mM sodium chloride and 15 mM sodium citrate). Hybridization can be carried out according to the methods described in *Molecular Cloning, A Laboratory Manual,* Second Edition, Cold Spring Harbor Laboratory Press (1989), *Current Protocols in Molecular Biology,* John Wiley & Sons (1987-1997), *DNA Cloning 1: Core Techniques, A Practical Approach,* Second Edition, Oxford University (1995), *etc.* Specifically, the DNA capable of hybridization under stringent conditions includes DNA having at least 60% or more homology, preferably 70% or more homology, more preferably 80% or more homology, further preferably 90% or more homology, particularly preferably 95% or more homology, most preferably 98% or more homology to the nucleotide sequence represented by SEQ ID NO:1, 3, 5 or 6.

In the present invention, the protein consisting of an amino acid sequence wherein one or more amino acid residue(s) is/are deleted, substituted, inserted and/or added in the amino acid sequence represented by SEQ ID NO:2 or 4 and having the activity of an enzyme relating to the synthesis of an intracellular sugar nucleotide, GDP-fucose, or the protein consisting of an amino acid sequence wherein one or more amino acid residue(s) is/are deleted, substituted, inserted and/or added in the amino acid sequence represented by SEQ ID NO:7 or 8 and having α1,6-fucosyltransferase activity can be obtained, for example, by introducing a site-directed mutation into DNA having the nucleotide sequence represented by SEQ ID NO:1, 3, 5 or 6 by site-directed mutagenesis described in *Molecular Cloning, A Laboratory Manual,* Second Edition, Cold Spring Harbor Laboratory Press (1989), *Current Protocols in Molecular Biology,* John

Wiley & Sons (1987-1997), *Nucleic Acids Research,* 10, 6487 (1982), *Proc. Natl. Acad Sci., USA,* 79, 6409 (1982), *Gene,* 34, 315 (1985), *Nucleic Acids Research,* 13, 4431 (1985), *Proc. Natl. Acad. Sci USA,* 82, 488 (1985), *etc.* The number of amino acid residues which are deleted, substituted, inserted and/or added is one or more, and is not specifically limited, but it is within the range where deletion, substitution or addition is possible by known methods such as the above site-directed mutagenesis. The suitable number is 1 to dozens, preferably 1 to 20, more preferably 1 to 10, further preferably 1 to 5.

The protein consisting of an amino acid sequence which has 80% or more homology to the amino acid sequence represented by SEQ ID NO:2, 4, 7 or 8 and having GDP-mannose 4,6-dehydratase activity, GDP-4-keto-6-deoxy-D-mannose-3,5-epimerase activity or α1,6-fucosyltransferase activity includes a protein having at least 80% or more homology, preferably 85% or more homology, more preferably 90% or more homology, further preferably 95% or more homology, particularly preferably 97% or more homology, most preferably 99% or more homology to the amino acid sequence represented by SEQ ID NO:2, 4, 7 or 8, respectively, when calculated by use of analysis software such as BLAST [*J. Mol. Biol.,* 215, 403 (1990)] or FASTA [*Methods in Enzymology,* 183, 63 (1990)].

The host cell used in the present invention, that is, the host cell in which the activity of an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is deleted may be obtained by any technique capable of deleting the above enzyme activity. For example, the following techniques can be employed for deleting the above enzyme activity:

(a) gene disruption targeting at a gene encoding the enzyme;
(b) introduction of a dominant-negative mutant of a gene encoding the enzyme;
(c) introduction of a mutation into the enzyme;
(d) suppression of transcription or translation of a gene encoding the enzyme;
(e) selection of a cell line resistant to a lectin which recognizes a sugar chain structure in which 1-position of fucose is bound to 6-position of N-acetylglucosamine in the reducing end through α-bond in a complex type N-glycoside-linked sugar chain.

As the lectin which recognizes a sugar chain structure in which 1-position of fucose is bound to 6-position of N-acetylglucosamine in the reducing end through α-bond in a complex type N-glycoside-linked sugar chain, any lectin capable of recognizing the sugar chain structure can be used. Specific examples include lentil lectin LCA (lentil agglutinin derived from *Lens culinaris*), pea lectin PSA (pea lectin derived from *Pisum sativum*), broad bean lectin VFA (agglutinin derived from *Yicia faba*), *Aleuria aurantia* lectin AAL (lectin derived from *Aleuria aurantia*) and the like.

The "cell resistant to a lectin" refers to a cell in which growth is not inhibited by the presence of a lectin at an effective concentration. The "effective concentration" is a concentration higher than the lowest concentration that does not allow the normal growth of a cell prior to the genome modification (hereinafter referred to also as parent cell line), preferably equal to the lowest concentration that does not allow the normal growth of a cell prior to the genome modification, more preferably 2 to 5 times, further preferably 10 times, most preferably 20 or more times the lowest concentration that does not allow the normal growth of a cell prior to the modification of the genomic gene.

The effective concentration of lectin that does not inhibit growth may be appropriately determined according to each cell line. It is usually 10 μg/ml to 10 mg/ml, preferably 0.5 mg/ml to 2.0 mg/ml.

The host cell for producing the antibody composition of the present invention may be any of the above host cells capable of expressing the antibody composition of the present invention. For example, yeast cells, animal cells, insect cells and plant cells can be used. Examples of the cells include those described in 1 below. Specifically, preferred among animal cells are CHO cell derived from Chinese hamster ovary tissue, rat myeloma cell line YB2/3HL.P2.G11.16Ag.20, mouse myeloma cell line NS0, mouse myeloma cell line SP2/0-Ag14 BHK cell derived from Syrian hamster kidney tissue, an antibody-producing hybridoma cell, human leukemia cell line Namalwa, an embryonic stem cell, and a fertilized egg cell.

A specific example of the transformant of the present invention is Ms705/GM2, which is a transformant derived from Chinese hamster ovary tissue-derived CHO cell line CHO/DG44 and carrying an introduced gene of the anti-ganglioside GM2 antibody of the present invention. The transformant Ms705/GM2 derived from CHO cell line CHO/DG44 was deposited with International Patent Organism Depositary, National Institute of Advanced Industrial Science and Technology, Central 6, 1-1, Higashi 1-chome, Tsukuba-shi, Ibaraki, Japan, on September 9, 2003 with accession No. FERM BP-8470.

Described below are the method for preparing a cell producing the antibody composition of the present invention, the method for producing the antibody composition of the present invention, the method for analyzing the antibody composition of the present invention and the method for utilizing the antibody composition of the present invention.

1. Preparation of a cell producing the antibody composition of the present invention

The cell producing the antibody composition of the present invention (hereinafter referred to as the cell of the present invention) can be prepared by preparing a host cell used for the production of the antibody composition of the present invention by the following techniques and then introducing a gene encoding the anti-ganglioside GM2 antibody into the host cell by the method described in 2 below.

(1) Gene disruption technique targeting at a gene encoding an enzyme

The host cell used for the production of the cell producing the antibody composition of the present invention (hereinafter referred to as the cell of the present invention) can be prepared by a gene disruption technique targeting a gene encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain. Examples of the enzymes relating to the synthesis of an intracellular sugar nucleotide, GDP-fucose include GDP-mannose 4,6-dehydratase (hereinafter referred to as GMD) and GDP-4-keto-6-deoxy-D-mannose-3,5-epimerase (hereinafter referred to as Fx). Examples of the enzymes relating to the modification of a sugar chain in which 1-position of fucose is bound to 6-position of N-acetylglucosamine in the reducing end through α-bond in a complex type N-glycoside-linked sugar chain include α1,6-fucosyltransferase and α-L-fucosidase.
The gene as used herein includes DNA and RNA.
The method of gene disruption may be any method capable of disrupting the gene encoding the target enzyme. Useful methods include the antisense method, the ribozyme method, the homologous recombination method, the RNA-DNA oligonucleotide method (hereinafter referred to as the RDO method), the RNA interference method (hereinafter referred to as the RNAi method), the method using a retrovirus and the method using a transposon. These methods are specifically described below.

(a) Preparation of the host cell for the production of the antibody composition of the present invention by the antisense method or the ribozyme method

The host cell used for the production of the antibody composition of the present invention can be prepared by the antisense method or the ribozyme method described in *Cell Technology,* 12, 239 (1993); *BIO/TECHNOLOGY,* 17**,** 1097 (1999); *Hum. Mol. Genet.,* 5*,* 1083 (1995); *Cell Technology,* 13, 255 (1994); *Proc. Natl. Acad Sci. U.S.A.,* 96, 1886 (1999); *etc.* targeting at a gene encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain, for example, in the following manner.
A cDNA or a genomic DNA encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is prepared.
The nucleotide sequence of the prepared cDNA or genomic DNA is determined.
Based on the determined DNA sequence, an antisense gene or a ribozyme of appropriate length is designed which comprises a DNA moiety encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, non-translated regions or introns.
In order to express the antisense gene or ribozyme in a cell, a recombinant vector is prepared by inserting a fragment or full-length of the prepared DNA into a site downstream of a promoter in an appropriate expression vector.
The recombinant vector is introduced into a host cell suited for the expression vector to obtain a transformant.
The host cell used for the production of the antibody composition of the present invention can be obtained by selecting a transformant using, as an index, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. The host cell used for the production of the antibody composition of the present invention can also be obtained by selecting a transformant using, as an index, the sugar chain structure of a glycoprotein on the cell membrane or the sugar chain structure of the produced antibody molecule.
As the host cell used for the production of the antibody composition of the present invention, any yeast cell, animal cell, insect cell, plant cell, or the like can be used so long as it has a gene encoding the target enzyme relating to the synthesis of an intracellular sugar nucleotide, GDP-fucose or the enzyme relating to the modification of a sugar

chain in which 1-position of fucose is bound to 6-position of N-acetylglucosamine in the reducing end through α-bond in a complex type N-glycoside-linked sugar chain. Examples of the host cells include those described in 2 below.

The expression vectors that can be employed are those capable of autonomous replication or integration into the chromosome in the above host cells and comprising a promoter at a position appropriate for the transcription of the designed antisense gene or ribozyme. Examples of the expression vectors include those described in 2 below.

Introduction of a gene into various host cells can be carried out by the methods suitable for introducing a recombinant vector into various host cells described in 2 below.

Selection of a transformant using, as an index, the activity of an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain can be carried out, for example, by the following methods.

Methods for selecting a transformant

A cell in which the activity of an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is deleted can be selected by measuring the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain using biochemical methods or genetic engineering techniques described in *Shin Seikagaku Jikken Koza (New Lectures on Experiments in Biochemistry) 3 - Saccharides I, Glycoprotein* (Tokyo Kagaku Dojin), edited by The Japanese Biochemical Society (1988); *Cell Technology, Extra Edition, Experimental Protocol Series, Glycobiology Experimental Protocol, Glycoprotein, Glycolipid and Proteoglycan* (Shujunsha), edited by Naoyuki Taniguchi, Akemi Suzuki, Kiyoshi Furukawa and Kazuyuki Sugawara (1996); *Molecular Cloning, A Laboratory Manual,* Second Edition, Cold Spring Harbor Laboratory Press (1989), *Current Protocols in Molecular Biology,* John Wiley & Sons (1987-1997); and the like. An example of the biochemical methods is a method in which the enzyme activity is evaluated using an enzyme-specific substrate. Examples of the genetic engineering techniques include Northern analysis and RT-PCR in which the amount of mRNA for a gene encoding the enzyme is measured.

Selection of a transformant using, as an index, the sugar chain structure of a glycoprotein on the cell membrane can be carried out, for example, by the method described in 1(5) below. Selection of a transformant using, as an index, the sugar chain structure of a produced antibody molecule can be carried out, for example, by the methods described in 4 or 5 below.

Preparation of a cDNA encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain can be carried out, for example, by the following method.

Preparation method of cDNA

Total RNA or mRNA is prepared from a various host cell tissue or cell.

A cDNA library is prepared from the total RNA or mRNA.

Degenerative primers are prepared based on the amino acid sequence of an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain, and a gene fragment encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain is obtained by PCR using the prepared cDNA library as a template.

A DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be obtained by screening the cDNA library using the obtained gene fragment as a probe.

As the mRNA of a human or non-human animal tissue or cell, commercially available one (for example, manufactured by Clontech) may be used, or it may be prepared from a human or non-human animal tissue or cell in the following manner.

The methods for preparing total RNA from a human or non-human animal tissue or cell include the guanidine thiocyanate-cesium trifluoroacetate method [*Methods in Enzymology,* 154, 3 (1987)], the acidic guanidine thiocyanate-phenol-chloroform (AGPC) method [*Analytical Biochemistry,* 162, 156 (1987); *Experimental Medicine,* 9, 1937 (1991)] and the like.

The methods for preparing mRNA as poly(A)$^+$RNA from the total RNA include the oligo (dT) immobilized cellulose column method [*Molecular Cloning, A Laboratory Manual,* Second Edition, Cold Spring Harbor Laboratory Press (1989)].

It is also possible to prepare mRNA by using a commercially available kit such as Fast Track mRNA Isolation Kit (manufactured by Invitrogen) or Quick Prep mRNA Purification Kit (manufactured by Pharmacia).

A cDNA library is prepared from the obtained mRNA of a human or non-human animal tissue or cell. The methods for preparing the cDNA library include the methods described in *Molecular Cloning, A Laboratory Manual,* Second Edition, Cold Spring Harbor Laboratory Press (1989), *Current Protocols in Molecular Biology,* John Wiley & Sons (1987-1997), *A Laboratory Manual,* 2nd Ed.(1989); *etc.,* and methods using commercially available kits such as SuperScript Plasmid System for cDNA Synthesis and Plasmid Cloning (manufactured by Life Technologies) and ZAP-cDNA Synthesis Kit (manufactured by STRATAGENE).

As the cloning vector for preparing the cDNA library, any vectors, e.g. phage vectors and plasmid vectors, can be used so long as they are autonomously replicable in *Escherichia coli* K12. Examples of suitable vectors include ZAP Express [manufactured by STRATAGENE; *Strategies,* 5, 58 (1992)], pBluescript II SK(+) *[Nucleic Acids Research,* 17, 9494 (1989)], λZAP II (manufactured by STRATAGENE), λgt10, λgt11 [*DNA Cloning, A Practical Approach,* 1, 49 (1985)], λTrip1Ex (manufactured by Clontech), λExCel1 (manufactured by Pharmacia), pT7T318U (manufactured by Pharmacia), pcD2 [*Mol. Cell. Biol.*, 3, 280 (1983)] and pUC18 [*Gene,* 33, 103 (1985)].

Any microorganism can be used as the host microorganism for preparing the cDNA library, but *Escherichia coli* is preferably used. Examples of suitable host microorganisms are *Escherichia coli* XL1-Blue MRF' [manufactured by STRATAGENE; *Strategies,* 5, 81 (1992)], *Escherichia coli* C600 [*Genetics,* 39, 440 (1954)], *Escherichia coli* Y1088 [*Science,* 222, 778 (1983)], *Escherichia coli* Y1090 [*Science,* 222, 778 (1983)], *Escherichia coli* NM522 [*J. Mol. Biol.,* 166, 1 (1983)], *Escherichia coli* K802 [*J. Mol. Biol.,* 16, 118 (1966)] and *Escherichia coli* JM105 [*Gene,* 38, 275 (1985)].

The cDNA library may be used as such in the following analysis. Alternatively, in order to efficiently obtain full-length cDNAs by decreasing the ratio of partial cDNAs, a cDNA library prepared using the oligo-cap method developed by Sugano, et al. [*Gene,* 138, 171 (1994); *Gene,* 200, 149 (1997); *Protein, Nucleic Acid and Enzyme,* 41, 603 (1996); *Experimental Medicine,* 11, 2491 (1993); *cDNA Cloning* (Yodosha) (1996); *Methods for Preparing Gene Libraries* (Yodosha) (1994)] may be used in the following analysis.

Degenerative primers specific for the 5'-terminal and 3'-terminal nucleotide sequences of a nucleotide sequence presumed to encode the amino acid sequence of an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain are prepared based on the amino acid sequence of the enzyme. A gene fragment encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be obtained by DNA amplification by PCR [*PCR Protocols,* Academic Press (1990)] using the prepared cDNA library as a template.

It can be confirmed that the obtained gene fragment is a DNA encoding the enzyme relating to the synthesis of an intracellular sugar nucleotide, GDP-fucose or the enzyme relating to the modification of a sugar chain in which 1-position of fucose is bound to 6-position ofN-acetylglucosamine in the reducing end through α-bond in a complex type N-glycoside-linked sugar chain by analyzing the nucleotide sequence by generally employed nucleotide sequence analyzing methods such as the dideoxy method of Sanger, et al. [*Proc. Natl. Acad. Sci. U.S.A.,* 74, 5463 (1977)] or by use of nucleotide sequence analyzers such as ABI PRISM 377 DNA Sequencer (manufactured by Applied Biosystems).

A DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be obtained from the cDNA or cDNA library synthesized from the mRNA contained in a human or non-human animal tissue or cell by colony hybridization or plaque hybridization [*Molecular Cloning, A Laboratory Manual,* Second Edition, Cold Spring Harbor Laboratory Press (1989)] using the above gene fragment as a probe.

A cDNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can also be obtained by amplification by PCR using the cDNA or cDNA library synthesized from the mRNA contained in a human or non-human animal tissue or cell as a template and using the primers used for obtaining the gene fragment encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain.

The nucleotide sequence of the obtained DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be determined by generally employed nucleotide sequence analyzing methods such as the dideoxy method of *Sanger, et al.* [*Proc. Natl. Acad Sci. U.S.A.,* 74, 5463 (1977)] or by use of nucleotide sequence analyzers such as ABI PRISM 377 DNA Sequencer (manufactured by Applied Biosystems).

By carrying out a search of nucleotide sequence databases such as GenBank, EMBL or DDBJ using a homology search program such as BLAST based on the determined nucleotide sequence of the cDNA, it can be confirmed that the obtained DNA is a gene encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain among the genes in the nucleotide sequence database.

Examples of the nucleotide sequences of the genes encoding the enzyme relating to the synthesis of an intracellular sugar nucleotide, GDP-fucose obtained by the above methods include the nucleotide sequences represented by SEQ ID NO:1 or 3.

Examples of the nucleotide sequences of the genes encoding the enzyme relating to the modification of a sugar chain in which 1-position of fucose is bound to 6-position of N-acetylglucosamine in the reducing end through α-bond in a complex type N-glycoside-linked sugar chain obtained by the above methods include the nucleotide sequence represented by SEQ ID NO: or 6.

The cDNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can also be obtained by chemical synthesis with a DNA synthesizer such as DNA Synthesizer Model 392 (manufactured by Perkin Elmer) utilizing the phosphoamidite method based on the determined nucleotide sequence of the DNA.

Preparation of a genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be carried out, for example, by the following method.

Method for preparing genomic DNA

The genomic DNA can be prepared by known methods described in *Molecular Cloning, A Laboratory Manual,* Second Edition, Cold Spring Harbor Laboratory Press (1989), *Current Protocols in Molecular Biology,* John Wiley & Sons (1987-1997); *etc.* In addition, the genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be obtained by using a kit such as Genomic DNA Library Screening System (manufactured by Genome Systems) or Universal GenomeWalker™ Kits (manufactured by CLONTECH).

The nucleotide sequence of the obtained DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be determined by generally employed nucleotide analyzing methods such as the dideoxy method of Sanger, *et al.* [*Proc. Natl. Acad. Sci. U.S.A.,* 74, 5463 (1977)] or by use of nucleotide sequence analyzers such as ABI PRISM 377 DNA Sequencer (manufactured by Applied Biosystems).

By carrying out a search of nucleotide sequence databases such as GenBank, EMBL or DDBJ using a homology search program such as BLAST based on the determined nucleotide sequence of the genomic DNA, it can be confirmed that the obtained DNA is a gene encoding the enzyme relating to the synthesis of an intracellular sugar nucleotide, GDP-fucose or the enzyme relating to the modification of a sugar chain in which 1-position of fucose is bound to 6-position ofN-acetylglucosamine in the reducing end through α-bond in a complex type N-glycoside-linked sugar chain among the genes in the nucleotide sequence database.

The genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can also be obtained by chemical synthesis with a DNA synthesizer such as DNA Synthesizer Model 392 (manufactured by Perkin Elmer) utilizing the phosphoamidite method based on the determined nucleotide sequence of the DNA.

Examples of the nucleotide sequences of the genomic DNAs encoding the enzyme relating to the synthesis of an intracellular sugar nucleotide, GDP-fucose obtained by the above methods include the nucleotide sequences represented by SEQ ID NOs:9, 10, 11 and 12.

An example of the nucleotide sequence of the genomic DNA encoding the enzyme relating to the modification of a sugar chain in which 1-position of fucose is bound to 6-position of N-acetylglucosamine in the reducing end through α-bond in a complex type N-glycoside-linked sugar chain obtained by the above methods is the nucleotide sequence represented by SEQ ID NO:13.

The host cell used for the production of the antibody composition of the present invention can also be obtained without using an expression vector by directly introducing into a host cell an antisense oligonucleotide or ribozyme designed based on the nucleotide sequence encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain.

The antisense oligonucleotide or ribozyme can be prepared by known methods or by using a DNA synthesizer. Specifically, based on the sequence information on an oligonucleotide having a sequence corresponding to 5 to 150, preferably 5 to 60, more preferably 10 to 40 contiguous nucleotides in the nucleotide sequence of the cDNA and genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, an oligonucleotide corresponding to the sequence complementary to the above oligonucleotide (antisense oligonucleotide) or a ribozyme comprising the oligonucleotide sequence can be synthesized.

The oligonucleotide includes oligo RNA and derivatives of the oligonucleotide (hereinafter referred to as oligonucleotide derivatives).

The oligonucleotide derivatives include an oligonucleotide derivative wherein the phosphodiester bond in the oligonucleotide is converted to a phosophorothioate bond, an oligonucleotide derivative wherein the phosphodiester bond in the oligonucleotide is converted to an N3'-P5' phosphoamidate bond, an oligonucleotide derivative wherein the ribose-phosphodiester bond in the oligonucleotide is converted to a peptide-nucleic acid bond, an oligonucleotide derivative wherein the uracil in the oligonucleotide is substituted with C-5 propynyluracil, an oligonucleotide derivative wherein the uracil in the oligonucleotide is substituted with C-5 thiazolyluracil, an oligonucleotide derivative wherein the cytosine in the oligonucleotide is substituted with C-5 propynylcytosine, an oligonucleotide derivative wherein the cytosine in the oligonucleotide is substituted with phenoxazine-modified cytosine, an oligonucleotide derivative wherein the ribose in the oligonucleotide is substituted with 2'-O-propylribose, and an oligonucleotide derivative wherein the ribose in the oligonucleotide is substituted with 2'-methoxyethoxyribose [*Cell Technology,* 16, 1463 (1997)].

(b) Preparation of the host cell for the production of the antibody composition of the present invention by the homologous recombination method

The host cell used for the production of the antibody composition of the present invention can be prepared by modifying a target gene on the chromosome by the homologous recombination method targeting a gene encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain.

Modification of the target gene on the chromosome can be carried out by using the methods described in *Manipulating the Mouse Embryo, A Laboratory Manual,* Second Edition, Cold Spring Harbor Laboratory Press (1994), *Gene Targeting, A Practical Approach,* IRL Press at Oxford University Press (1993); *Biomanual Series 8, Gene Targeting, Preparation of Mutant Mice Using ES Cells,* Yodosha (1995) (hereinafter referred to as *Preparation of Mutant Mice Using ES Cells*); *etc.,* for example, in the following manner.

A genomic DNA encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is prepared.

Based on the nucleotide sequence of the genomic DNA, a target vector is prepared for homologous recombination of a target gene to be modified (e.g., the structural gene or promoter gene for the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain).

The host cell used for the preparation of the cell of the present invention can be prepared by introducing the prepared target vector into a host cell and selecting a cell in which homologous recombination occurred between the target gene on the chromosome and the target vector.

As the host cell, any yeast cell, animal cell, insect cell, plant cell, or the like can be used so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine

in the reducing end through α-bond in a complex type N-glycoside-linked sugar chain. Examples of the host cells include those described in 2 below.

The genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be prepared by the methods for preparing a genomic DNA described in the above 1 (1) (a), *etc.*

Examples of the nucleotide sequences of the genomic DNAs encoding the enzyme relating to the synthesis of the intracellular sugar nucleotide, GDP-fucose obtained by the above methods include the nucleotide sequences represented by SEQ ID NOs:9, 10, 11 and 12.

An example of the nucleotide sequence of the genomic DNA encoding the enzyme relating to the modification of a sugar chain in which 1-position of fucose is bound to 6-position of N-acetylglucosamine in the reducing end through α-bond in a complex type N-glycoside-linked sugar chain obtained by the above methods is the nucleotide sequence represented by SEQ ID NO:13.

The target vector for use in the homologous recombination of the target gene on the chromosome can be prepared according to the methods described in *Gene Targeting, A Practical Approach,* IRL Press at Oxford University Press (1993); *Biomanual Series 8, Gene Targeting, Preparation of Mutant Mice Using ES Cells,* Yodosha (1995); *etc.* The target vector may be either a replacement-type or an insertion-type.

Introduction of the target vector into various host cells can be carried out by the methods suitable for introducing a recombinant vector into various host cells described in 3 below.

The methods for efficiently selecting a homologous recombinant include positive selection, promoter selection, negative selection and polyA selection described in *Gene Targeting, A Practical Approach,* IRL Press at Oxford University Press (1993); *Biomanual Series 8, Gene Targeting, Preparation of Mutant Mice Using ES Cells,* Yodosha (1995); *etc.* The methods for selecting the desired homologous recombinant from the selected cell lines include Southern hybridization *[Molecular Cloning, A Laboratory Manual,* Second Edition, Cold Spring Harbor Laboratory Press (1989)] and PCR [*PCR Protocols,* Academic Press (1990)] with the genomic DNA.

(c) Preparation of the host cell for the production of the antibody composition of the present invention by the RDO method

The host cell used for the production of the antibody composition of the present invention can be prepared by the RDO method targeting a gene encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain, for example, in the following manner.

A cDNA or a genomic DNA encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is prepared by the methods described in the above 1 (1) (a).

The nucleotide sequence of the prepared cDNA or genomic DNA is determined.

Based on the determined DNA sequence, an RDO construct of appropriate length which comprises a DNA moiety encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, non-translated regions or introns is designed and synthesized.

The host cell of the present invention can be obtained by introducing the synthesized RDO into a host cell and then selecting a transformant in which a mutation occurred in the target enzyme, that is, the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain.

As the host cell, any yeast cell, animal cell, insect cell, plant cell, or the like can be used so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. Examples of the host cells include those described in 2 below.

Introduction of the RDO into various host cells can be carried out by the methods suitable for introducing a recombinant vector into various host cells described in 2 below.

The cDNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be prepared

by the methods for preparing a cDNA described in the above 1 (1) (a) or the like.

The genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be prepared by the methods for preparing a genomic DNA described in the above 1 (1) (b) or the like.

After DNA is cleaved with appropriate restriction enzymes, the nucleotide sequence of the DNA can be determined by subcloning the DNA fragments into a plasmid such as pBluescript SK(-) (manufactured by Stratagene), subjecting the clones to the reaction generally used as a method for analyzing a nucleotide sequence such as the dideoxy method of Sanger *et al.* [*Proc. Natl. Acad. Sci. USA,* 74, 5463 (1977)] or the like, and then analyzing the clones by using an automatic nucleotide sequence analyzer such as ABI PRISM 377 DNA Sequencer (manufactured by Applied Biosystems) or the like.

The RDO can be prepared by conventional methods or by using a DNA synthesizer.

The methods for selecting a cell in which a mutation occurred by introducing the RDO into the host cell, in the gene encoding the target enzyme, that is, the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain include the methods for directly detecting mutations in chromosomal genes described in *Molecular Cloning, A Laboratory Manual,* Second Edition, Cold Spring Harbor Laboratory Press (1989), *Current Protocols in Molecular Biology,* John Wiley & Sons (1987-1997); *etc.*

For the selection of the transformant, the following methods can also be employed: the method using, as an index, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain described in the above 1 (1) (a); the method using, as an index, the sugar chain structure of a glycoprotein on the cell membrane described in 1 (5) below; and the method using, as an index, the sugar chain structure of a produced antibody molecule described in 4 or 5 below.

The RDO can be designed according to the descriptions in *Science,* 273, 1386 (1996); *Nature Medicine,* 4, 285 (1998); *Hepatology,* 25, 1462 (1997); *Gene Therapy,* 5, 1960 (1999); *J. Mol. Med.,* 75, 829 (1997); *Proc. Natl. Acad Sci. USA,* 96, 8774 (1999); *Proc. Natl. Acad Sci. USA,* 96, 8768 (1999); *Nuc. Acids Res.,* 27, 1323 (1999); *Invest. Dermatol.,* 111, 1172 (1998); *Nature Biotech.,* 16, 1343 (1998); *Nature Biotech.,* 18, 43 (2000); *Nature Biotech.,* 18, 555 (2000); *etc.*

(d) Preparation of the host cell for the production of the antibody composition of the present invention by the RNAi method

The host cell used for the production of the antibody composition of the present invention can be prepared by the RNAi method targeting a gene encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain, for example, in the following manner.

A cDNA encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is prepared by the methods described in the above 1 (1) (a).

The nucleotide sequence of the prepared cDNA is determined.

Based on the determined cDNA sequence, an RNAi gene of appropriate length is designed which comprises a moiety encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, or non-translated regions.

In order to express the RNAi gene in a cell, a recombinant vector is prepared by inserting a fragment or full-length of the prepared cDNA into a site downstream of a promoter in an appropriate expression vector.

The recombinant vector is introduced into a host cell suited for the expression vector to obtain a transformant.

The host cell used for the preparation of the cell of the present invention can be obtained by selecting a transformant using, as an index, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, or the sugar chain structure of a produced antibody molecule or a glycoprotein on the cell membrane.

As the host cell, any yeast cell, animal cell, insect cell, plant cell, or the like can be used so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine

in the reducing end through α-bond in a complex type N-glycoside-linked sugar chain. Examples of the host cells include those described in 2 below.

The expression vectors that can be employed are those capable of autonomous replication or integration into the chromosome in the above host cells and comprising a promoter at a position appropriate for the transcription of the designed RNAi gene. Examples of the expression vectors include those described in 2 below.

Introduction of a gene into various host cells can be carried out by the methods suitable for introducing a recombinant vector into various host cells described in 2 below.

The methods for selecting the transformant using, as an index, the activity of the enzyme relating to the synthesis of an intracellular sugar nucleotide, GDP-fucose or the activity of the enzyme relating to the modification of a sugar chain in which 1-position of fucose is bound to 6-position ofN-acetylglucosamine in the reducing end through α-bond in a complex type N-glycoside-linked sugar chain include the methods described in the above 1 (1) (a).

The methods for selecting the transformant using, as an index, the sugar chain structure of a glycoprotein on the cell membrane include the method described in 1 (5). The methods for selecting the transformant using, as an index, the sugar chain structure of a produced antibody molecule include the methods described in 4 or 5 below.

The methods for preparing cDNA encoding the enzyme relating to the synthesis of an intracellular sugar nucleotide, GDP-fucose or the enzyme relating to the modification of a sugar chain in which 1-position of fucose is bound to 6-position ofN-acetylglucosamine in the reducing end through α-bond in a complex type N-glycoside-linked sugar chain without using an expression vector include the methods for preparing a cDNA described in the above 1 (1) (a), *etc.*

The host cell used for the preparation of the cell of the present invention can also be obtained without using an expression vector by directly introducing into a host cell the RNAi gene designed based on the nucleotide sequence encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain.

The RNAi gene can be prepared by known methods or by using a DNA synthesizer. The RNAi gene construct can be designed according to the descriptions in *Nature,* 391, 806 (1998); *Proc. Natl. Acad. Sci. USA,* 95, 15502 (1998); *Nature,* 395, 854 (1998); *Proc. Natl. Acad Sci. USA,* 96, 5049 (1999); *Cell,* 95, 1017 (1998); *Proc. Natl. Acad* Sci. *USA,* 96, 1451 (1999); *Proc. Natl. Acad Sci. USA,* 95, 13959 (1998); *Nature Cell Biol.,* 2, 70 (2000); *etc.*

(e) Preparation of the host cell for the production of the antibody composition of the present invention by the method using a transposon

The host cell used for the production of the antibody composition of the present invention can be prepared by using the transposon system described in *Nature Genet.,* 25, 35 (2000), *etc.,* and then selecting a mutant using, as an index, the activity of the enzyme relating to the synthesis of an intracellular sugar nucleotide, GDP-fucose or the activity of the enzyme relating to the modification of a sugar chain in which 1-position of fucose is bound to 6-position of N-acetylglucosamine in the reducing end through α-bond in a complex type N-glycoside-linked sugar chain, or the sugar chain structure of a produced antibody molecule or a glycoprotein on the cell membrane.

The transposon system is a system for inducing a mutation by random insertion of an exogenous gene into the chromosome, wherein usually an exogenous gene inserted into a transposon is used as a vector for inducing a mutation and a transposase expression vector for randomly inserting the gene into the chromosome is introduced into the cell at the same time.

Any transposase can be used so long as it is suitable for the sequence of the transposon to be used.

As the exogenous gene, any gene can be used so long as it can induce a mutation in the DNA of a host cell.

As the host cell, any yeast cell, animal cell, insect cell, plant cell, or the like can be used so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. Examples of the host cells include those described in 2 below. Introduction of the gene into various host cells can be carried out by the methods suitable for introducing a recombinant vector into various host cells described in 2 below.

The methods for selecting the mutant using, as an index, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain include the methods described in the above 1 (1) (a).

The methods for selecting the mutant using, as an index, the sugar chain structure of a glycoprotein on the cell membrane include the method described in 1 (5). The methods for selecting the mutant using, as an index, the sugar chain structure of a produced antibody molecule include the methods described in 4 or 5 below.

(2) Technique of introducing a dominant-negative mutant of a gene encoding an enzyme

The host cell used for the production of the antibody composition of the present invention can be prepared by using the method of introducing a dominant-negative mutant of a target gene, i.e., a gene encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain. Examples of the enzymes relating to the synthesis of the intracellular sugar nucleotide, GDP-fucose include GMD and Fx. Examples of the enzymes relating to the modification of a sugar chain in which 1-position of fucose is bound to 6-position of N-acetylglucosamine in the reducing end through α-bond in a complex type N-glycoside-linked sugar chain include α1,6-fucosyltransferase and α-L-fucosidase.

These enzymes have substrate specificity and catalyze specific reactions. By disrupting the active center of such enzymes having substrate specificity and catalytic action, their dominant-negative mutants can be prepared. Preparation of a dominant-negative mutant is described in detail below, using for an example GMD among the target enzymes.

As a result of the analysis of the tertiary structure of GMD derived from *Escherichia coli,* it has been revealed that four amino acids (threonine at position 133, glutamic acid at position 135, tyrosine at position 157 and lysine at position 161) have an important function for the enzyme activity (*Structure,* 8, 2, 2000). That is, the mutants prepared by substituting the above four amino acids by other amino acids based on the tertiary structure information all showed significantly decreased enzyme activity. On the other hand, little change was observed in the ability of the mutants to bind to the GMD coenzyme NADP or the substrate GDP-mannose. Accordingly, a dominant-negative mutant can be prepared by substituting the four amino acids which are responsible for the enzyme activity of GMD. On the basis of the result of preparation of a dominant-negative mutant of GMD derived from *Escherichia coli,* dominant-negative mutants can be prepared by performing homology comparison and tertiary structure prediction using the amino acid sequence information. For example, in the case of GMD derived from CHO cell (SEQ ID NO:2), a dominant-negative mutant can be prepared by substituting threonine at position 155, glutamic acid at position 157, tyrosine at position 179 and lysine at position 183 by other amino acids. Preparation of such a gene carrying introduced amino acid substitutions can be carried out by site-directed mutagenesis described in *Molecular Cloning, A Laboratory Manual,* Second Edition, Cold Spring Harbor Laboratory Press (1989), *Current Protocols in Molecular Biology,* John Wiley & Sons (1987-1997); *etc.*

The host cell used for the production of the antibody composition of the present invention can be prepared according to the method of gene introduction described in *Molecular Cloning, A Laboratory Manual,* Second Edition, Cold Spring Harbor Laboratory Press (1989), *Current Protocols in Molecular Biology,* John Wiley & Sons (1987-1997), *Manipulating the Mouse Embryo A Laboratory Manual,* Second Edition, Cold Spring Harbor Laboratory Press (1994); *etc.* using a gene encoding a dominant-negative mutant of a target enzyme (hereinafter abbreviated as dominant-negative mutant gene) prepared as above, for example, in the following manner.

A dominant-negative mutant gene encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain is prepared.

Based on the full-length DNA of the prepared dominant-negative mutant gene, a DNA fragment of appropriate length containing a region encoding the protein is prepared according to need.

A recombinant vector is prepared by inserting the DNA fragment or full-length DNA into a site downstream of a promoter in an appropriate expression vector.

The recombinant vector is introduced into a host cell suited for the expression vector to obtain a transformant.

The host cell used for the preparation of the cell of the present invention can be obtained by selecting a transformant using, as an index, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, or the sugar chain structure of a produced antibody molecule or a glycoprotein on the cell membrane.

As the host cell, any yeast cell, animal cell, insect cell, plant cell, or the like can be used so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. Examples of the host cells include those described in 2 below.

The expression vectors that can be employed are those capable of autonomous replication or integration into the chromosome in the above host cells and comprising a promoter at a position appropriate for the transcription of the DNA encoding the desired dominant-negative mutant. Examples of the expression vectors include those described in 2 below.

Introduction of a gene into various host cells can be carried out by the methods suitable for introducing a recombinant vector into various host cells described in 2 below.

The methods for selecting the transformant using, as an index, the activity of the enzyme relating to the synthesis of an intracellular sugar nucleotide, GDP-fucose or the activity of the enzyme relating to the modification of a sugar chain in which 1-position of fucose is bound to 6-position ofN-acetylglucosamine in the reducing end through α-bond in a complex type N-glycoside-linked sugar chain include the methods described in the above 1 (1) (a).
The methods for selecting the transformant using, as an index, the sugar chain structure of a glycoprotein on the cell membrane include the method described in 1 (5) below. The methods for selecting the transformant using, as an index, the sugar chain structure of a produced antibody molecule include the methods described in 4 or 5 below.

(3) Technique of introducing a mutation into an enzyme

The host cell used for the production of the antibody composition of the present invention can be prepared by introducing a mutation into a gene encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain, and then selecting a desired cell line in which the mutation occurred in the enzyme.
Examples of the enzymes relating to the synthesis of the intracellular sugar nucleotide, GDP-fucose include GMD and Fx. Examples of the enzymes relating to the modification of a sugar chain in which 1-position of fucose is bound to 6-position of N-acetylglucosamine in the reducing end through α-bond in a complex type N-glycoside-linked sugar chain include α1,6-fucosyltransferase and α-L-fucosidase.
The methods for introducing a mutation into the enzyme include: 1) a method in which a desired cell line is selected from mutants obtained by subjecting a parent cell line to mutagenesis or by spontaneous mutation using, as an index, the activity of the enzyme relating to the synthesis of an intracellular sugar nucleotide, GDP-fucose or the activity of the enzyme relating to the modification of a sugar chain in which 1-position of fucose is bound to 6-position of N-acetylglucosamine in the reducing end through α-bond in a complex type N-glycoside-linked sugar chain; 2) a method in which a desired cell line is selected from mutants obtained by subjecting a parent cell line to mutagenesis or by spontaneous mutation using, as an index, the sugar chain structure of a produced antibody molecule; and 3) a method in which a desired cell line is selected from mutants obtained by subjecting a parent cell line to mutagenesis or by spontaneous mutation using, as an index, the sugar chain structure of a glycoprotein on the cell membrane.
Mutagenesis may be carried out by any method capable of inducing a point mutation, a deletion mutation or a frameshift mutation in DNA of a cell of a parent cell line.
Suitable methods include treatment with ethyl nitrosourea, nitrosoguanidine, benzopyrene or an acridine dye and radiation treatment. Various alkylating agents and carcinogens are also useful as mutagens. A mutagen is allowed to act on a cell by the methods described in *Soshiki Baiyo no Gijutsu (Tissue Culture Techniques),* Third Edition (Asakura Shoten), edited by The Japanese Tissue Culture Association (1996); *Nature Genet.,* 24, 314 (2000); *etc.*
Examples of the mutants generated by spontaneous mutation include spontaneous mutants obtained by continuing subculture under usual cell culture conditions without any particular treatment for mutagenesis.
The methods for measuring the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain include the methods described in the above 1 (1) (a). The methods for determining the sugar chain structure of a produced antibody molecule include the methods described in 4 or 5 below. The methods for determining the sugar chain structure of a glycoprotein on the cell membrane include the method described in 1 (5).

(4) Technique of suppressing transcription or translation of a gene encoding an enzyme

The host cell used for the production of the antibody composition of the present invention can be prepared by suppressing transcription or translation of a target gene, i.e., a gene encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain using the antisense RNA/DNA technique [*Bioscience and Industry,* 50, 322 (1992); *Chemistry,* 46, 681 (1991); *Biotechnology,* 9, 358 (1992); *Trends in Biotechnology,* 10, 87 (1992); *Trends in Biotechnology,* 10, 152 (1992); *Cell Technology,* 16, 1463 (1997)], the triple helix technique [*Trends in Biotechnology,* 10, 132 (1992)], *etc.*
Examples of the enzymes relating to the synthesis of the intracellular sugar nucleotide, GDP-fucose include GMD and Fx. Examples of the enzymes relating to the modification of a sugar chain in which 1-position of fucose is bound to 6-position of N-acetylglucosamine in the reducing end through α-bond in a complex type N-glycoside-linked sugar chain include α1,6-fucosyltransferase and α-L-fucosidase.
The methods for measuring the activity of the enzyme relating to the synthesis of an intracellular sugar nucleotide, GDP-fucose or the activity of the enzyme relating to the modification of a sugar chain in which 1-position of fucose is bound to 6-position of N-acetylglucosamine in the reducing end through α-bond in a complex type N-glycoside-linked sugar chain include the methods described in the above 1 (1) (a).

The methods for determining the sugar chain structure of a glycoprotein on the cell membrane include the method described in 1 (5). The methods for determining the sugar chain structure of a produced antibody molecule include the methods described in 4 or 5 below.

(5) Technique of selecting a cell line resistant to a lectin which recognizes a sugar chain structure in which 1-position of fucose is bound to 6-position of N-acetylglucosamine in the reducing end through α-bond in a complex type N-glycoside-linked sugar chain

The host cell used for the production of the antibody composition of the present invention can be prepared by selecting a cell line resistant to a lectin which recognizes a sugar chain structure in which 1-position of fucose is bound to 6-position of N-acetylglucosamine in the reducing end through α-bond in a complex type N-glycoside-linked sugar chain.

Selection of a cell line resistant to a lectin which recognizes a sugar chain structure in which 1-position of fucose is bound to 6-position of N-acetylglucosamine in the reducing end through α-bond in a complex type N-glycoside-linked sugar chain can be carried out, for example, by the method using a lectin described in *Somatic Cell Mol. Genet.,* 12, 51 (1986), *etc.*

As the lectin, any lectin can be used so long as it recognizes a sugar chain structure in which 1-position of fucose is bound to 6-position of N-acetylglucosamine in the reducing end through α-bond in a complex type N-glycoside-linked sugar chain. Specific examples include lentil lectin LCA (lentil agglutinin derived from *Lens culinaris*), pea lectin PSA (pea lectin derived from *Pisum sativum*), broad bean lectin VFA (agglutinin derived from *Vicia faba*) and *Aleuria aurantia* lectin AAL (lectin derived from *Aleuria aurantia*).

Specifically, the cell line of the present invention resistant to a lectin which recognizes a sugar chain structure in which 1-position of fucose is bound to 6-position of N-acetylglucosamine in the reducing end through α-bond in a complex type N-glycoside-linked sugar chain can be selected by culturing cells in a medium containing the above lectin at a concentration of 1 μg/ml to 1 mg/ml for one day to 2 weeks, preferably one day to one week, subculturing surviving cells or picking up a colony and transferring it into a culture vessel, and subsequently continuing the culturing using the medium containing the lectin.

2. Process for producing the antibody composition

The antibody composition of the present invention can be obtained by expressing it in a host cell using the methods described in *Molecular Cloning, A Laboratory Manual,* Second Edition, Cold Spring Harbor Laboratory Press (1989), *Current Protocols in Molecular Biology,* John Wiley & Sons (1987-1997), *Antibodies, A Laboratory manual,* Cold Spring Harbor Laboratory (1988), *Monoclonal Antibodies: Principles and Practice,* Third Edition, Acad. Press (1993), *Antibody Engineering, A Practical Approach,* IRL Press at Oxford University Press (1996) *etc.,* for example, in the following manner.

A full-length cDNA encoding an anti-ganglioside GM2 antibody molecule is prepared, and a DNA fragment of appropriate length comprising a region encoding the antibody molecule is prepared.

A recombinant vector is prepared by inserting the DNA fragment or full-length DNA into a site downstream of a promoter in an appropriate expression vector.

The recombinant vector is introduced into a host cell suited for the expression vector to obtain a transformant producing the antibody composition.

As the host cell, any yeast cells, animal cells, insect cells, plant cells, *etc.* that are capable of expressing the antibody can be used.

Also useful as the host cell are cells obtained by selecting cells in which the activity of an enzyme relating to the modification of an N-glycoside-linked sugar chain bound to the Fc region of an antibody molecule, i.e., an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is deleted, or cells obtained by various artificial techniques described in the above 1.

The expression vectors that can be employed are those capable of autonomous replication or integration into the chromosome in the above host cells and comprising a promoter at a position appropriate for the transcription of the DNA encoding the desired antibody molecule.

The cDNA can be prepared from a human or non-human animal tissue or cell according to the methods for preparing a cDNA described in the above 1 (1) (a) using, e.g., a probe or primers specific for cDNA encoding the desired

antibody molecule.

When yeast is used as the host cell, YEP13 (ATCC 37115), YEp24 (ATCC 37051), YCp50 (ATCC 37419), *etc.* can be used as the expression vector.

As the promoter, any promoters capable of expressing in yeast strains can be used. Suitable promoters include promoters of genes of the glycolytic pathway such as hexosekinase, PHO5 promoter, PGK promoter, GAP promoter, ADH promoter, gal 1 promoter, gal 10 promoter, heat shock protein promoter, MFα1 promoter and CUP 1 promoter.

Examples of suitable host cells are microorganisms belonging to the genera *Saccharomyces, Schizosaccharomyces, Kluyveromyces, Trichosporon* and *Schwanniomyces,* and specifically, *Saccharomyces cerevisiae, Schizosaccharomyces pombe, Kluyveromyces lactis, Trichosporon pullulans* and *Schwanniomyces alluvius.*

Introduction of the recombinant vector can be carried out by any of the methods for introducing DNA into yeast, for example, electroporation [*Methods Enzymol.,* 194, 182 (1990)], the spheroplast method [*Proc. Natl. Acad Sci. USA,* 84, 1929 (1978)], the lithium acetate method [*J. Bacteriology,* 153, 163 (1983)] and the method described in *Proc. Natl. Acad. Sci. USA,* 75, 1929 (1978).

When an animal cell is used as the host cell, pcDNAI, pcDM8 (commercially available from Funakoshi Co., Ltd.), pAGE107 [Japanese Published Unexamined Patent Application No. 22979/91; *Cytotechnology,* 3, 133 (1990)], pAS3-3 (Japanese Published Unexamined Patent Application No. 227075/90), pCDM8 [*Nature,* 329, 840 (1987)], pcDNAI/Amp (manufactured by Invitrogen Corp.), pREP4 (manufactured by Invitrogen Corp.), pAGE103 [*J. Biochemistry,* 101, 1307 (1987)], pAGE210, *etc.* can be used as the expression vector.

As the promoter, any promoters capable of expressing in animal cells can be used. Suitable promoters include the promoter of IE (immediate early) gene of cytomegalovirus (CMV), SV40 early promoter, the promoter of a retrovirus, metallothionein promoter, heat shock promoter, SRα promoter, *etc.* The enhancer of IE gene of human CMV may be used in combination with the promoter.

Examples of suitable host cells are human-derived Namalwa cells, monkey-derived COS cells, Chinese hamster-derived CHO cells, HBT5637 (Japanese Published Unexamined Patent Application No. 299/88), rat myeloma cells, mouse myeloma cells, cells derived from Syrian hamster kidney, embryonic stem cells and fertilized egg cells.

Introduction of the recombinant vector can be carried out by any of the methods for introducing DNA into animal cells, for example, electroporation [*Cytotechnology,* 3, 133 (1990)], the calcium phosphate method (Japanese Published Unexamined Patent Application No. 227075/90), lipofection [*Proc. Natl. Acad Sci. USA,* 84, 7413 (1987)], the injection method [*Manipulating the Mouse Embryo, A Laboratory Manual,* Second Edition, Cold Spring Harbor Laboratory Press (1994)], the method using particle gun (gene gun) (Japanese Patent Nos. 2606856 and 2517813), the DEAE-dextran method [*Biomanual Series 4 - Methods of Gene Transfer, Expression and Analysis* (Yodosha), edited by Takashi Yokota and Kenichi Arai (1994)] and the virus vector method [*Manipulating the Mouse Embryo, A Laboratory Manual,* Second Edition, Cold Spring Harbor Laboratory Press (1994)].

When an insect cell is used as the host cell, the protein can be expressed by the methods described in *Current Protocols in Molecular Biology; Baculovirus Expression Vectors, A Laboratory Manual,* W. H. Freeman and Company, New York (1992); Bio/Technology, 6, 47 (1988), *etc.*

That is, the expression vector and a baculovirus are cotransfected into insect cells to obtain a recombinant virus in the culture supernatant of the insect cells, and then insect cells are infected with the recombinant virus, whereby the protein can be expressed.

The gene introducing vectors useful in this method include pVL1392, pVL1393 and pBlueBacIII (products of Invitrogen Corp.).

An example of the baculovirus is Autographa californica nuclear polyhedrosis virus, which is a virus infecting insects belonging to the family *Barathra.*

Examples of the insect cells are *Spodoptera frugiperda* ovarian cells Sf9 and Sf21 [*Current Protocols in Molecular Biology; Baculovirus Expression Vectors, A Laboratory Manual,* W.H. Freeman and Company, New York (1992)] and *Trichoplusia ni* ovarian cell High 5 (manufactured by Invitrogen Corp.).

Cotransfection of the above expression vector and the above baculovirus into insect cells for the preparation of the recombinant virus can be carried out by the calcium phosphate method (Japanese Published Unexamined Patent Application No. 227075/90), lipofection *[Proc. Natl. Acad Sci. USA,* 84, 7413 (1987)], *etc.*

When a plant cell is used as the host cell, Ti plasmid, tobacco mosaic virus vector, *etc.* can be used as the expression vector.

As the promoter, any promoters capable of expressing in plant cells can be used. Suitable promoters include 35S promoter of cauliflower mosaic virus (CaMV), rice actin 1 promoter, *etc.*

Examples of suitable host cells are cells of plants such as tobacco, potato, tomato, carrot, soybean, rape, alfalfa, rice, wheat and barley.

Introduction of the recombinant vector can be carried out by any of the methods for introducing DNA into plant cells, for example, the method using *Agrobacterium* (Japanese Published Unexamined Patent Application Nos. 140885/84 and 70080/85, WO94/00977) electroporation (Japanese Published Unexamined Patent Application No.

251887/85) and the method using particle gun (gene gun) (Japanese Patent Nos. 2606856 and 2517813).

Expression of the antibody composition can be carried out not only by direct expression but also by secretory production, expression of a fusion protein of the Fc region and another protein, *etc.* according to the methods described in *Molecular Cloning, A Laboratory Manual,* Second Edition, Cold Spring Harbor Laboratory Press (1989), *etc.*

When the gene is expressed in a yeast cell, an animal cell, an insect cell or a plant cell carrying an introduced gene relating to the synthesis of a sugar chain, an antibody molecule to which a sugar or a sugar chain is added by the introduced gene can be obtained.

The antibody composition can be produced by culturing the transformant obtained as above in a medium, allowing the antibody molecules to form and accumulate in the culture, and recovering them from the culture. Culturing of the transformant in a medium can be carried out by conventional methods for culturing the host cell.

For the culturing of the transformant obtained by using a eucaryote such as yeast as the host, any of natural media and synthetic media can be used insofar as it is a medium suitable for efficient culturing of the transformant which contains carbon sources, nitrogen sources, inorganic salts, *etc.* which can be assimilated by the host used.

As the carbon sources, any carbon sources that can be assimilated by the host can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of organic or inorganic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged spinner culture under aeration. The culturing temperature is preferably 15 to 40°C, and the culturing period is usually 16 hours to 7 days. The pH is maintained at 3 to 9 during the culturing. The pH adjustment is carried out by using an organic or inorganic acid, an alkali solution, urea, calcium carbonate, ammonia, *etc.*

If necessary, antibiotics such as ampicillin and tetracycline may be added to the medium during the culturing.

When a microorganism transformed with a recombinant vector comprising an inducible promoter is cultured, an inducer may be added to the medium, if necessary. For example, in the case of a microorganism transformed with a recombinant vector comprising *lac* promoter, isopropyl-β-D-thiogalactopyranoside or the like may be added to the medium; and in the case of a microorganism transformed with a recombinant vector comprising *trp* promoter, indoleacrylic acid or the like may be added.

For the culturing of the transformant obtained by using an animal cell as the host cell, generally employed media such as RPMI1640 medium [*The Journal of the American Medical Association,* 199, 519 (1967)], Eagle's MEM medium [*Science,* 122, 501 (1952)], Dulbecco's modified MEM medium [*Virology,* 8, 396 (1959)], 199 medium [*Proceeding* of *the Society for the Biological Medicine,* 73, 1 (1950)] and Whitten's medium [*Developmental Engineering Experimentation Manual - Preparation of Transgenic Mice* (Kodansha), edited by Motoya Katsuki (1987)], media prepared by adding fetal calf serum or the like to these media, *etc.* can be used as the medium.

Culturing is usually carried out under conditions of pH 6 to 8 at 30 to 40°C for 1 to 7 days in the presence of 5% $CO_2$.

If necessary, antibiotics such as kanamycin and penicillin may be added to the medium during the culturing.

For the culturing of the transformant obtained by using an insect cell as the host cell, generally employed media such as TNM-FH medium (manufactured by Pharmingen, Inc.), Sf-900 II SFM medium (manufactured by Life Technologies, Inc.), ExCell 400 and ExCell 405 (manufactured by JRH Biosciences, Inc.) and Grace's Insect Medium [*Nature,* 195, 788 (1962)] can be used as the medium.

Culturing is usually carried out under conditions of pH 6 to 7 at 25 to 30°C for 1 to 5 days.

If necessary, antibiotics such as gentamicin may be added to the medium during the culturing.

The transformant obtained by using a plant cell as the host cell may be cultured in the form of cells as such or after differentiation into plant cells or plant organs. For the culturing of such transformant, generally employed media such as Murashige-Skoog (MS) medium and White medium, media prepared by adding phytohormones such as auxin and cytokinin to these media, *etc.* can be used as the medium.

Culturing is usually carried out under conditions of pH 5 to 9 at 20 to 40°C for 3 to 60 days.

If necessary, antibiotics such as kanamycin and hygromycin may be added to the medium during the culturing.

As described above, the antibody composition can be produced by culturing, according to a conventional culturing method, the transformant derived from an animal cell or a plant cell and carrying an expression vector into which DNA encoding the antibody molecule has been inserted, allowing the antibody composition to form and accumulate, and recovering the antibody composition from the culture.

Expression of the antibody composition can be carried out not only by direct expression but also by secretory production, fusion protein expression, *etc.* according to the methods described in *Molecular Cloning, A Laboratory Manual,* Second Edition, Cold Spring Harbor Laboratory Press (1989).

The antibody composition may be produced intracellularly on host cells, may be secrated extracellularly on host cells or may be produced on outer membranes of host cells. A desirable production method can be adopted by changing the kind of the host cells used or the structure of the antibody molecule to be produced.

When the antibody composition is produced in host cells or on outer membranes of host cells, it is possible to force the antibody composition to be secreted outside the host cells by applying the method of Paulson, *et al.* [*J. Biol. Chem.,* 264, 17619 (1989)], the method of Lowe, *et al.* [*Proc. Natl. Acad. Sci. USA,* 86, 8227 (1989); *Genes Develop.,* 4, 1288 (1990)], or the methods described in Japanese Published Unexamined Patent Application No. 336963/93, WO94/23021, *etc.*

That is, it is possible to force the desired antibody molecule to be secreted outside the host cells by inserting DNA encoding the antibody molecule and DNA encoding a signal peptide suitable for the expression of the antibody molecule into an expression vector, introducing the expression vector into the host cells, and then expressing the antibody molecule by use of recombinant DNA techniques.

It is also possible to increase the production of the antibody composition by utilizing a gene amplification system using a dihydrofolate reductase gene or the like according to the method described in Japanese Published Unexamined Patent Application No. 227075/90.

Further, the antibody composition can be produced using an animal individual having an introduced gene (non-human transgenic animal) or a plant individual having an introduced gene (transgenic plant) constructed by redifferentiation of animal or plant cells carrying the introduced gene.

When the transformant is an animal individual or plant individual, the antibody composition can be produced by raising or culturing the animal or plant in a usual manner, allowing the antibody composition to form and accumulate therein, and recovering the antibody composition from the animal individual or plant individual.

Production of the antibody composition using an animal individual can be carried out, for example, by producing the desired antibody composition in an animal constructed by introducing the gene according to known methods [*American Journal of Clinical Nutrition,* 63, 639S (1996); *American Journal of Clinical Nutrition,* 63, 627S (1996); *Bio/Technology,* 9, 830 (1991)].

In the case of an animal individual, the antibody composition can be produced, for example, by raising a non-human transgenic animal carrying the introduced DNA encoding the antibody molecule, allowing the antibody composition to form and accumulate in the animal, and recovering the antibody composition from the animal. The places where the antibody composition is formed and accumulated include milk (Japanese Published Unexamined Patent Application No. 309192/88), egg, *etc.* of the animal. As the promoter in this process, any promoters capable of expressing in an animal can be used. Preferred promoters include mammary gland cell-specific promoters such as $\alpha$ casein promoter, $\beta$ casein promoter, $\beta$ lactoglobulin promoter and whey acidic protein promoter.

Production of the antibody composition using a plant individual can be carried out, for example, by culturing a transgenic plant carrying the introduced DNA encoding the antibody molecule according to known methods *[Soshiki Baiyo (Tissue Culture),* 20 (1994); *Soshiki Baiyo (Tissue Culture),* 21 (1995); *Trends in Biotechnology,* 15, 45 (1997)], allowing the antibody composition to form and accumulate in the plant, and recovering the antibody composition from the plant.

When the antibody composition produced by the transformant into which the DNA encoding the antibody molecule is introduced is expressed in a soluble form in cells, the cells are recovered by centrifugation after the completion of culturing and suspended in an aqueous buffer, followed by disruption using a sonicator, French press, Manton Gaulin homogenizer, Dynomill or the like to obtain a cell-free extract. A purified preparation of the antibody composition can be obtained by centrifuging the cell-free extract to obtain the supernatant and then subjecting the supernatant to ordinary means for isolating and purifying enzymes, e.g., extraction with a solvent, salting-out with ammonium sulfate, *etc.,* desalting, precipitation with an organic solvent, anion exchange chromatography using resins such as diethylaminoethyl (DEAE)-Sepharose and DIAION HPA-75 (manufactured by Mitsubishi Chemical Corporation), cation exchange chromatography using resins such as S-Sepharose FF (manufactured by Pharmacia), hydrophobic chromatography using resins such as butyl Sepharose and phenyl Sepharose, gel filtration using a molecular sieve, affinity chromatography, chromatofocusing, and electrophoresis such as isoelectric focusing, alone or in combination.

When the antibody composition is expressed as an inclusion body in cells, the cells are similarly recovered and disrupted, followed by centrifugation to recover the inclusion body of the antibody composition as a precipitate fraction. The recovered inclusion body of the antibody composition is solubilized with a protein-denaturing agent. The solubilized antibody solution is diluted or dialyzed, whereby the antibody composition is renatured to have normal tertiary structure. Then, a purified preparation of the antibody composition can be obtained by the same isolation and purification steps as described above.

When the antibody composition is extracellularly secreted, the antibody composition or its derivative can be

recovered in the culture supernatant. That is, the culture is treated by the same means as above, e.g., centrifugation, to obtain the culture supernatant. A purified preparation of the antibody composition can be obtained from the culture supernatant by using the same isolation and purification methods as described above.

As an example of the methods for obtaining the antibody composition of the present invention, the method for producing a humanized antibody composition is specifically described below. Other antibody compositions can also be obtained in a similar manner.

(1) Construction of a vector for expression of humanized antibody

A vector for expression of humanized antibody is an expression vector for animal cells carrying inserted genes encoding CH and CL of a human antibody, which can be constructed by cloning each of the genes encoding CH and CL of a human antibody into an expression vector for animal cells.

The C regions of a human antibody may be CH and CL of any human antibody. Examples of the C regions include the C region of IgG1 subclass human antibody H chain (hereinafter referred to as hCγl) and the C region of κ class human antibody L chain (hereinafter referred to as hCκ).

As the genes encoding CH and CL of a human antibody, a chromosome DNA comprising exons and introns can be used. Also useful is a cDNA prepared by reverse transcription of an mRNA.

As the expression vector for animal cells, any vector for animal cells can be used so long as it is capable of inserting and expressing the gene encoding the C region of a human antibody. Suitable vectors include pAGE107 [*Cytotechnology,* 3, 133 (1990)], pAGE103 [*J. Biochem.,* 101, 1307 (1987)], pHSG274 [*Gene,* 27, 223 (1984)], pKCR [*Proc. Natl. Acad. Sci. USA,* 78, 1527 (1981)] and pSG1βd2-4 [*Cytotechnology,* 4, 173 (1990)]. Examples of the promoter and enhancer for use in the expression vector for animal cells include SV40 early promoter and enhancer [*J. Biochem.,* 101, 1307 (1987)], LTR of Moloney mouse leukemia virus [*Biochem. Biophys. Res. Commun.,* 149, 960 (1987)] and immunoglobulin H chain promoter [*Cell,* 41, 479 (1985)] and enhancer [*Cell,* 33, 717 (1983)].

The vector for expression of humanized antibody may be either of the type in which the genes encoding antibody H chain and L chain exist on separate vectors or of the type in which both genes exist on the same vector (hereinafter referred to as tandem-type). The tandem-type ones are preferred in view of the easiness of construction of the humanized antibody expression vector, the easiness of introduction into animal cells, the balance between the expression of antibody H chain and that of antibody L chain in animal cells, *etc.* [*J. Immunol. Methods,* 167, 271 (1994)]. Examples of the tandem-type humanized antibody expression vectors include pKANTEX93 [*Mol. Immunol.,* 37, 1035 (2000)] and pEE18 [*Hybridoma,* 17, 559 (1998)].

The constructed vector for expression of humanized antibody can be used for the expression of a human chimeric antibody and a human CDR-grafted antibody in animal cells.

(2) Obtaining of cDNA encoding V region of an antibody derived from a non-human animal

cDNAs encoding VH and VL of an antibody derived from a non-human animal, e.g., a mouse antibody can be obtained in the following manner.

A cDNA is synthesized using, as a template, an mRNA extracted from a hybridoma cell producing an antibody which specifically binds to ganglioside GM2. The synthesized cDNA is inserted into a vector such as a phage or a plasmid to prepare a cDNA library. A recombinant phage or recombinant plasmid carrying a cDNA encoding the H chain V region and a recombinant phage or recombinant plasmid carrying a cDNA encoding the L chain V region are isolated from the cDNA library using DNA encoding the C region or V region of a known mouse antibody as a probe. The entire nucleotide sequences of VH and VL of the desired mouse antibody on the recombinant phages or recombinant plasmids are determined, and the whole amino acid sequences of VH and VL are deduced from the nucleotide sequences.

Hybridoma cells producing a non-human animal-derived antibody which specifically binds to ganglioside GM2 can be obtained by immunizing a non-human animal with ganglioside GM2, preparing hybridomas from antibody-producing cells of the immunized animal and myeloma cells according to a known method (*Antibodies: A Laboratory Manual,* Cold Spring Harbor Laboratory, Chapter 14, 1998), selecting cloned hybridomas, culturing the selected hybridomas and purifying cells from the culture supernatant.

As the non-human animal, any animal can be used so long as hybridoma cells can be prepared from the animal. Suitable animals include mouse, rat, hamster and rabbit.

The methods for preparing total RNA from a hybridoma cell include the guanidine thiocyanate-cesium trifluoroacetate method [*Methods in Enzymol.,* 154, 3 (1987)], and the methods for preparing mRNA from the total RNA include the oligo (dT) immobilized cellulose column method [*Molecular Cloning, A Laboratory Manual,* Second Edition, Cold Spring Harbor Laboratory Press (1989)]. Examples of the kits for preparing mRNA from a hybridoma cell include Fast Track mRNA Isolation Kit (manufactured by Invitrogen) and Quick Prep mRNA Purification Kit (manufactured by Pharmacia).

The methods for synthesizing the cDNA and preparing the cDNA library include conventional methods [*Molecular Cloning, A Laboratory Manual,* Second Edition, Cold Spring Harbor Laboratory Press (1989), *Current Protocols in Molecular Biology,* Supplement 1-34], or methods using commercially available kits such as SuperScript™ Plasmid System for cDNA Synthesis and Plasmid Cloning (manufactured by GIBCO BRL) and ZAP-cDNA Synthesis Kit (manufactured by STRATAGENE).

In preparing the cDNA library, the vector for inserting the cDNA synthesized using the mRNA extracted from a hybridoma cell as a template may be any vector so long as the cDNA can be inserted. Examples of suitable vectors include ZAP Express [*Strategies, 5,* 58 (1992)], pBluescript II SK(+) [*Nucleic Acids Research,* 17, 9494 (1989)], λZAP II (manufactured by STRATAGENE), λgt10, λgt11 [*DNA Cloning: A Practical Approach,* I, 49 (1985)], Lambda BlueMid (manufactured by Clontech), λExCell, pT7T3 18U (manufactured by Pharmacia), pcD2 [*Mol.* Cell. *Biol.,* 3, 280 (1983)] and pUC18 [*Gene,* 33, 103 (1985)].

As *Escherichia coli* for introducing the cDNA library constructed with a phage or plasmid vector, any *Escherichia coli* can be used so long as the cDNA library can be introduced, expressed and maintained. Examples of suitable *Escherichia coli* include XL1-Blue MRF' [*Strategies,* 5, 81 (1992)], C600 [*Genetics,* 39, 440 (1954)], Y1088, Y1090 [*Science,* 222, 778 (1983)], NM522 [*J. Mol. Biol.,* 166, 1 (1983)], K802 [*J. Mol. Biol.,* 16, 118 (1966)] and JM105 [*Gene,* 38, 275 (1985)].

The methods for selecting the cDNA clones encoding VH and VL of a non-human animal-derived antibody from the cDNA library include colony hybridization or plaque hybridization [*Molecular Cloning, A Laboratory Manual,* Second Edition, Cold Spring Harbor Laboratory Press (1989)] using an isotope- or fluorescence-labeled probe. It is also possible to prepare the cDNAs encoding VH and VL by preparing primers and carrying out PCR [*Molecular Cloning, A Laboratory Manual,* Second Edition, Cold Spring Harbor Laboratory Press (1989), *Current Protocols in Molecular Biology,* Supplement 1-34] using the cDNA or cDNA library as a template.

The nucleotide sequences of the cDNAs selected by the above methods can be determined by cleaving the cDNAs with appropriate restriction enzymes, cloning the fragments into a plasmid such as pBluescript SK(-) (manufactured by STRATAGENE), and then analyzing the sequences by generally employed nucleotide sequence analyzing methods such as the dideoxy method of *Sanger, et al.* [*Proc. Natl. Acad Sci. USA,* 74, 5463 (1977)] or by use of nucleotide sequence analyzers such as ABI PRISM 377 DNA Sequencer (manufactured by Applied Biosystems).

The entire amino acid sequences of VH and VL are deduced from the determined nucleotide sequences and compared with the entire amino acid sequences of VH and VL of a known antibody [*Sequences of Proteins of Immunological Interest,* US Dept. Health and Human Services (1991)], whereby it can be confirmed that the obtained cDNAs encode amino acid sequences which completely comprise VH and VL of the antibody including secretory signal sequences.

Further, when the amino acid sequence of an antibody variable region or the nucleotide sequence of DNA encoding the variable region is already known, the DNA can be obtained by the following methods.

When the amino acid sequence is known, the desired DNA can be obtained by designing a DNA sequence encoding the variable region taking into consideration the frequency of codon usage [*Sequences of Proteins of Immunological Interest,* US Dept. Health and Human Services (1991)], synthesizing several synthetic DNAs constituting approximately 100-nucleotides based on the designed DNA sequence, and carrying out PCR using the synthetic DNAs. When the nucleotide sequence is known, the desired DNA can be obtained by synthesizing several synthetic DNAs constituting approximately 100-nucleotides based on the nucleotide sequence information and carrying out PCR using the synthetic DNAs.

(3) Analysis of the amino acid sequence of the V region of an antibody derived from a non-human animal

By comparing the entire amino acid sequences of VH and VL of the antibody including secretory signal sequences with the amino acid sequences of VH and VL of a known antibody [*Sequences of Proteins of Immunological Interest,* US Dept. Health and Human Services (1991)], it is possible to deduce the length of the secretory signal sequences and the N-terminal amino acid sequences and further to know the subgroup to which the antibody belongs. In addition, the amino acid sequences of CDRs of VH and VL can be deduced in a similar manner.

(4) Construction of a human chimeric antibody expression vector

A human chimeric antibody expression vector can be constructed by inserting the cDNAs encoding VH and VL of an antibody derived from a non-human animal into sites upstream of the genes encoding CH and CL of a human antibody in the vector for expression of humanized antibody described in the above 2 (1). For example, a human chimeric antibody expression vector can be constructed by ligating the cDNAs encoding VH and VL of an antibody derived from a non-human animal respectively to synthetic DNAs comprising the 3'-terminal nucleotide sequences of VH and VL of an antibody derived from a non-human animal and the 5'-terminal nucleotide sequences of CH and CL of a human

antibody and also having recognition sequences for appropriate restriction enzymes at both ends, and inserting them into sites upstream of the genes encoding CH and CL of a human antibody in the vector for humanized antibody expression described in the above 2 (1) so as to express them in an appropriate form.

(5) Construction of cDNA encoding V region of a human CDR-grafted antibody

cDNAs encoding VH and VL of a human CDR-grafted antibody can be constructed in the following manner. First, amino acid sequences of FRs of VH and VL of a human antibody for grafting CDRs of VH and VL of a non-human animal-derived antibody are selected. The amino acid sequences of FRs of VH and VL of a human antibody may be any of those derived from human antibodies. Suitable sequences include the amino acid sequences of FRs of VHs and VLs of human antibodies registered at databases such as Protein Data Bank, and the amino acid sequences common to subgroups of FRs of VHs and VLs of human antibodies [*Sequences of Proteins of Immunological Interest,* US Dept. Health and Human Services (1991)]. In order to prepare a human CDR-grafted antibody having a sufficient activity, it is preferred to select amino acid sequences having as high a homology as possible (at least 60% or more) with the amino acid sequences of FRs of VH and VL of the non-human animal-derived antibody of interest.
Next, the amino acid sequences of CDRs of VH and VL of the non-human animal-derived antibody of interest are grafted to the selected amino acid sequences of FRs of VH and VL of a human antibody to design amino acid sequences of VH and VL of a human CDR-grafted antibody. The designed amino acid sequences are converted into DNA sequences taking into consideration the frequency of codon usage in the nucleotide sequences of antibody genes [*Sequences of Proteins of Immunological Interest,* US Dept. Health and Human Services (1991)], and DNA sequences encoding the amino acid sequences of VH and VL of the human CDR-grafted antibody are designed. Several synthetic DNAs constituting approximately 100-nucleotides are synthesized based on the designed DNA sequences, and PCR is carried out using the synthetic DNAs. It is preferred to design 4 to 6 synthetic DNAs for each of the H chain and the L chain in view of the reaction efficiency of PCR and the lengths of DNAs that can be synthesized.
Cloning into the vector for humanized antibody expression constructed in the above 2 (1) can be easily carried out by introducing recognition sequences for appropriate restriction enzymes to the 5'-terminals of synthetic DNAs present on both ends. After the PCR, the amplification products are cloned into a plasmid such as pBluescript SK(-) (manufactured by STRATAGENE) and the nucleotide sequences are determined by the method described in the above 2 (2) to obtain a plasmid carrying DNA sequences encoding the amino acid sequences of VH and VL of the desired human CDR-grafted antibody.

(6) Modification of the amino acid sequence of V region of a human CDR-grafted antibody

It is known that a human CDR-grafted antibody prepared merely by grafting CDRs of VH and VL of a non-human animal-derived antibody to FRs of VH and VL of a human antibody has a lower antigen-binding activity compared with the original non-human animal-derived antibody [*BIO/TECHNOLOGY,* 9, 266 (1991)]. This is probably because in VH and VL of the original non-human animal-derived antibody, not only CDRs but also some of the amino acid residues in FRs are involved directly or indirectly in the antigen-binding activity, and such amino acid residues are replaced by amino acid residues derived from FRs of VH and VL of the human antibody by CDR grafting. In order to solve this problem, attempts have been made in the preparation of a human CDR-grafted antibody to raise the lowered antigen-binding activity by identifying the amino acid residues in the amino acid sequences of FRs of VH and VL of a human antibody which are directly relating to the binding to an antigen or which are indirectly relating to it through interaction with amino acid residues in CDRs or maintenance of the tertiary structure of antibody, and modifying such amino acid residues to those derived from the original non-human animal-derived antibody [*BIO/TECHNOLOGY,* 9, 266 (1991)].
In the preparation of a human CDR-grafted antibody, it is most important to efficiently identify the amino acid residues in FR which are relating to the antigen-binding activity. For the efficient identification, construction and analyses of the tertiary structures of antibodies have been carried out by X ray crystallography [*J. Mol. Biol.,* 112, 535 (1977)], computer modeling [*Protein* Engineering, 7, 1501 (1994)], *etc.* Although these studies on the tertiary structures of antibodies have provided much information useful for the preparation of human CDR-grafted antibodies, there is no established method for preparing a human CDR-grafted antibody that is adaptable to any type of antibody. That is, at present, it is still necessary to make trial-and-error approaches, e.g., preparation of several modifications for each antibody and examination of each modification for the relationship with the antigen-binding activity.
Modification of the amino acid residues in FRs of VH and VL of a human antibody can be achieved by PCR as described in the above 2 (5) using synthetic DNAs for modification. The nucleotide sequence of the PCR amplification product is determined by the method described in the above 2 (2) to confirm that the desired modification has been achieved.

(7) Construction of a human CDR-grafted antibody expression vector

A human CDR-grafted antibody expression vector can be constructed by inserting the cDNAs encoding VH and VL of the human CDR-grafted antibody constructed in the above 2 (5) and (6) into sites upstream of the genes encoding CH and CL of a human antibody in the vector for humanized antibody expression described in the above 2 (1). For example, a human CDR-grafted antibody expression vector can be constructed by introducing recognition sequences for appropriate restriction enzymes to the 5'-terminals of synthetic DNAs present on both ends among the synthetic DNAs used for constructing VH and VL of the human CDR-grafted antibody in the above 2 (5) and (6), and inserting them into sites upstream of the genes encoding CH and CL of a human antibody in the vector for humanized antibody expression described in the above 2 (1) so as to express them in an appropriate form.

(8) Stable production of a humanized antibody

Transformants capable of stably producing a human chimeric antibody and a human CDR-grafted antibody (hereinafter collectively referred to as humanized antibody) can be obtained by introducing the humanized antibody expression vectors described in the above 2 (4) and (7) into appropriate animal cells.
Introduction of the humanized antibody expression vector into an animal cell can be carried out by electroporation [Japanese Published Unexamined Patent Application No. 257891/90; *Cytotechnology,* 3, 133 (1990)], *etc.*
As the animal cell for introducing the humanized antibody expression vector, any animal cell capable of producing a humanized antibody can be used.
Examples of the animal cells include mouse myeloma cell lines NSO and SP2/0, Chinese hamster ovary cells CHO/dhfr- and CHO/DG44, rat myeloma cell lines YB2/0 and IR983F, Syrian hamster kidney-derived BHK cell, and human myeloma cell line Namalwa. Preferred are Chinese hamster ovary cell CHO/DG44 and rat myeloma cell line YB2/0.
After the introduction of the humanized antibody expression vector, the transformant capable of stably producing the humanized antibody can be selected using a medium for animal cell culture containing an agent such as G418 sulfate (hereinafter referred to as G418; manufactured by SIGMA) according to the method described in Japanese Published Unexamined Patent Application No. 257891/90. Examples of the media for animal cell culture include RPMI1640 medium (manufactured by Nissui Pharmaceutical Co., Ltd.), GIT medium (manufactured by Nihon Pharmaceutical Co., Ltd.), EX-CELL 302 medium (manufactured by JRH), IMDM medium (manufactured by GIBCO BRL), Hybridoma-SFM medium (manufactured by GIBCO BRL), and media prepared by adding various additives such as fetal calf serum (hereinafter referred to as FCS) to these media. By culturing the obtained transformant in the medium, the humanized antibody can be formed and accumulated in the culture supernatant. The amount and the antigen-binding activity of the humanized antibody produced in the culture supernatant can be measured by enzyme-linked immunosorbent assay [hereinafter referred to as ELISA; *Antibodies: A Laboratory Manual,* Cold Spring Harbor Laboratory, Chapter 14 (1998); *Monoclonal Antibodies: Principles and Practice,* Academic Press Limited (1996)] or the like. The production of the humanized antibody by the transformant can be increased by utilizing a DHFR gene amplification system or the like according to the method described in Japanese Published Unexamined Patent Application No. 257891/90.
The humanized antibody can be purified from the culture supernatant of the transformant using a protein A column [*Antibodies: A Laboratory Manual,* Cold Spring Harbor Laboratory, Chapter 8 (1988); *Monoclonal Antibodies: Principles and Practice,* Academic Press Limited (1996)]. In addition, purification methods generally employed for the purification of proteins can also be used. For example, the purification can be carried out by combinations of gel filtration, ion exchange chromatography, ultrafiltration and the like. The molecular weight of the H chain, L chain or whole antibody molecule of the purified humanized antibody can be measured by SDS-denatured polyacrylamide gel electrophoresis [hereinafter referred to as SDS-PAGE; *Nature,* 227, 680 (1970)], Western blotting [*Antibodies: A Laboratory Manual,* Cold Spring Harbor Laboratory, Chapter 12 (1988); *Monoclonal Antibodies: Principles and Practice,* Academic Press Limited (1996)], *etc.*
Shown above is the method for producing the antibody composition using an animal cell as the host. As described above, the antibody composition can also be produced using yeast, an insect cell, a plant cell, an animal individual or a plant individual by similar methods.
When a host cell inherently has the ability to express the antibody molecule, the antibody composition of the present invention can be produced by preparing a cell expressing the antibody composition using the method described in the above 1, culturing the cell, and then purifying the desired antibody composition from the culture.

3. Evaluation of the activity of the antibody composition

The protein amount, antigen-binding activity and cytotoxic activity of the purified antibody composition can be measured using the known methods described in *Monoclonal Antibodies: Principles and Practice,* Academic Press Limited (1996), *Antibody Engineering, A Practical Approach,* IRL Press at Oxford University Press (1996), *etc.*

Specifically, when the antibody composition is a humanized antibody, the activity to bind to an antigen or an antigenically positive cultured cell line can be measured by ELISA, the fluorescent antibody technique [*Cancer Immunol. Immunother.,* 36, 373 (1993)], *etc.* The cytotoxic activity against an antigenically positive cultured cell line can be evaluated by measuring CDC activity, ADCC activity, *etc.* [*Cancer Immunol. Immunother.,* 36, 373 (1993)].
The safety and therapeutic effect of the antibody composition in human can be evaluated using an appropriate animal model of a species relatively close to human, e.g., cynomolgus monkey.

4. Analysis of sugar chains in the antibody composition

The sugar chain structure of the antibody compositions expressed in various cells can be analyzed according to general methods of analysis of the sugar chain structure of glycoprotein compositions. For example, a sugar chain bound to an IgG molecule consists of neutral sugars such as galactose, mannose and fucose, amino sugars such as N-acetylglucosamine, and acidic sugars such as sialic acid, and can be analyzed by techniques such as sugar composition analysis and sugar chain structure analysis using two-dimensional sugar chain mapping.

(1) Analysis of neutral sugar and amino sugar compositions

The sugar chain composition of an antibody composition can be analyzed by carrying out acid hydrolysis of sugar chains with trifluoroacetic acid or the like to release neutral sugars or amino sugars and analyzing the composition ratio.
Specifically, the analysis can be carried out by a method using a carbohydrate analysis system (BioLC; product of Dionex). BioLC is a system for analyzing the sugar composition by HPAEC-PAD (high performance anion-exchange chromatography-pulsed amperometric detection) [*J. Liq. Chromatogr.,* 6, 1577 (1983)].
The composition ratio can also be analyzed by the fluorescence labeling method using 2-aminopyridine. Specifically, the composition ratio can be calculated by fluorescence labeling an acid-hydrolyzed sample by 2-aminopyridylation according to a known method [*Agric. Biol. Chem.,* 55(1), 283 (1991)] and then analyzing the composition by HPLC.

(2) Analysis of sugar chain structure

The sugar chain structure of an antibody composition can be analyzed by two-dimensional sugar chain mapping [*Anal. Biochem.,* 171, 73 (1988); *Seibutsukagaku Jikkenho* (*Biochemical Experimentation Methods) 23 - Totanpakushitsu Tosa Kenkyuho (Methods of Studies on Glycoprotein Sugar Chains),* Gakkai Shuppan Center, edited by Reiko Takahashi (1989)]. The two-dimensional sugar chain mapping is a method of deducing a sugar chain structure, for example, by plotting the retention time or elution position of a sugar chain by reversed phase chromatography as the X axis and the retention time or elution position of the sugar chain by normal phase chromatography as the Y axis, respectively, and comparing them with the results on known sugar chains.
Specifically, a sugar chain is released from an antibody composition by hydrazinolysis of the antibody composition and subjected to fluorescence labeling with 2-aminopyridine (hereinafter referred to as PA) [*J. Biochem.,* 95, 197 (1984)]. After being separated from an excess PA-treating reagent by gel filtration, the sugar chain is subjected to reversed phase chromatography. Then, each peak of the sugar chain is subjected to normal phase chromatography. The sugar chain structure can be deduced by plotting the obtained results on a two-dimensional sugar chain map and comparing them with the spots of a sugar chain standard (manufactured by Takara Shuzo Co., Ltd.) or those in the literature [*Anal. Biochem.,* 171, 73 (1988)].
The structure deduced by the two-dimensional sugar chain mapping can be confirmed by carrying out mass spectrometry, e.g., MALDI-TOF-MS, of each sugar chain.

5. Immunoassay for determining the sugar chain structure of an antibody molecule

An antibody composition comprises an antibody molecule having different sugar chain structures binding to the Fc region of antibody. The antibody composition of the present invention, in which the ratio of a sugar chain in which fucose is not bound to the N-acetylglucosamine in the reducing end to the total complex type N-glycoside-linked sugar chains bound to the Fc region is 100%, has high ADCC activity. Such an antibody composition can be determined using the method for analyzing the sugar chain structure of an antibody composition described in the above 4. Further, it can also be determined by immunoassays using lectins.
Determination of the sugar chain structure of an antibody composition by immunoassays using lectins can be made according to the immunoassays such as Western staining, RIA (radioimmunoassay), VIA (viroimmunoassay), EIA (enzymeimmunoassay), FIA (fluoroimmunoassay) and MIA (metalloimmunoassay) described in the literature [*Monoclonal Antibodies: Principles and Applications,* Wiley-Liss, Inc. (1995); *Enzyme Immunoassay,* 3rd Ed., Igaku Shoin

(1987); *Enzyme Antibody Technique,* Revised Edition, Gakusai Kikaku (1985); *etc.*], for example, in the following manner.

A lectin recognizing the sugar chain structure of an antibody molecule constituting an antibody composition is labeled, and the labeled lectin is subjected to reaction with a sample antibody composition, followed by measurement of the amount of a complex of the labeled lectin with the antibody molecule.

Examples of lectins useful for determining the sugar chain structure of an antibody molecule include WGA (wheat-germ agglutinin derived from *T. vulgaris*), ConA (concanavalin A derived from *C. ensiformis*), RIC (toxin derived from *R. communis*), L-PHA (leukoagglutinin derived from *P. vulgaris*), LCA (lentil agglutinin derived from *L. culinaris*), PSA (pea lectin derived from *P. sativum*), AAL (*Aleuria aurantia* lectin), ACL (*Amaranthus caudatus* lectin), BPL (*Bauhinia purpurea* lectin), DSL (*Datura stramonium* lectin), DBA (*Dolichos biflorus* agglutinin), EBL (Elderberry balk lectin), ECL (*Erythrina cristagalli* lectin), EEL (*Euonymus europaeus* lectin), GNL (*Galanthus nivalis* lectin), GSL (*Griffonia simplicifolia* lectin), HPA (*Helix pomatia* agglutinin), HHL (*Hippeastrum* hybrid lectin), Jacalin, LTL (*Lotus tetragonolobus* lectin), LEL (*Lycopersicon esculentum* lectin), MAL (*Maackia amurensis* lectin), MPL (*Maclura pomifera* lectin), NPL (*Narcissus pseudonarcissus* lectin), PNA (peanut agglutinin), E-PHA (*Phaseolus vulgaris* erythroagglutinin), PTL (*Psophocarpus tetragonolobus* lectin), RCA (*Ricinus communis* agglutinin), STL (*Solanum tuberosum* lectin), SJA (*Sophora japonica* agglutinin), SBA (soybean agglutinin), UEA (*Ulex europaeus* agglutinin), VVL (*Vicia villosa* lectin) and WFA (*Wisteria floribunda* agglutinin).

It is preferred to use lectins specifically recognizing a sugar chain structure wherein fucose is bound to the N-acetylglucosamine in the reducing end in complex type N-glycoside-linked sugar chains. Examples of such lectins include lentil lectin LCA (lentil agglutinin derived from *Lens culinaris*), pea lectin PSA (pea lectin derived from *Pisum sativum*), broad bean lectin VFA (agglutinin derived from *Vicia faba*) and *Aleuria aurantia* lectin AAL (lectin derived from *Aleuria aurantia*).

6. Utilization of the antibody composition of the present invention

Since the antibody composition of the present invention specifically binds to ganglioside GM2 and has high ADCC activity and CDC activity, it is useful for the prevention and treatment of various diseases in which ganglioside GM2-expressing cells are concerned, including cancer.

In the present invention, the diseases relating to a ganglioside GM2 may be any diseases, so long as they are related to ganglioside GM2-expressing cells. Examples include cancer and the like.

The cancer in the present invention includes neuroectodermal tumors such as neuroblastoma, small cell lung cancer and melanoma.

The ganglioside GM2 is present at a slight amount in normal cells, but is present at a large amount in cancer cells such as neuroblastoma, small cell lung cancer and melanoma, and a monoclonal antibody against GM2 is considered to be useful for treatment of these cancers [*Lancet.,* 48, 6154 (1988)]. Ordinary anti-tumor agents are characterized by the suppression of the proliferation of these cancer cells. In contrast, antibodies having high ADCC activity and CDC activity can induce cell death of cancer cells, and therefore, they are more effective as therapeutic agents for cancer than the ordinary anti-tumor agents. At present, therapeutic antibodies used as therapeutic agents for cancer have only insufficient anti-tumor effect when used alone and thus are used in combination with chemotherapy [*Science,* 280, 1197 (1998)]. Since the antibody composition of the present invention has potent anti-tumor effect by itself, the dependency on chemotherapy will be decreased and side effects will be reduced as well.

Since the antibody composition of the present invention specifically binds to ganglioside GM2 and has high cytotoxic activity against ganglioside GM2-expressing cells, the cells expressing ganglioside GM2 can be selectively eliminated.

Furthermore, since the antibody composition of the present invention has high cytotoxic activity, it can treat patients of the above cancers mentioned above which cannot be cured by the conventional antibody compositions. Moreover, in the case of the cancer, since it is difficult to deliver a drug to the infiltration region of the cancer cells, it is preferable that therapeutic effects can be obtained by a small amount of drug. Since the antibody composition of the present invention has high ADCC activity at a small amount, it is effective for treatment of the above diseases.

A pharmaceutical composition comprising the antibody composition of the present invention may be administered alone as a therapeutic agent. However, it is preferably mixed with one or more pharmaceutically acceptable carriers and provided as a pharmaceutical preparation produced by an arbitrary method well known in the technical field of pharmaceutics.

It is desirable to administer the pharmaceutical composition by the route that is most effective for the treatment. Suitable administration routes include oral administration and parenteral administration such as intraoral administration, intratracheal administration, intrarectal administration, subcutaneous administration, intramuscular administration and intravenous administration. In the case of an antibody preparation, intravenous administration is preferable.

The pharmaceutical preparation may be in the form of spray, capsules, tablets, granules, syrup, emulsion, suppository, injection, ointment, tape, and the like.

The pharmaceutical preparations suitable for oral administration include emulsions, syrups, capsules, tablets, powders and granules.

Liquid preparations such as emulsions and syrups can be prepared using, as additives, water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates), flavors (e.g., strawberry flavor and peppermint), and the like.

Capsules, tablets, powders, granules, *etc*, can be prepared using, as additives, excipients (e.g., lactose, glucose, sucrose and mannitol), disintegrators (e.g., starch and sodium alginate), lubricants (e.g., magnesium stearate and talc), binders (e.g., polyvinyl alcohol, hydroxypropyl cellulose and gelatin), surfactants (e.g., fatty acid esters), plasticizers (e.g., glycerin), and the like.

The pharmaceutical preparations suitable for parenteral administration include injections, suppositories and sprays.

Injections can be prepared using carriers comprising a salt solution, a glucose solution, or a mixture thereof, *etc.* It is also possible to prepare powder injections by freeze-drying the antibody composition according to a conventional method and adding sodium chloride thereto.

Suppositories can be prepared using carriers such as cacao butter, hydrogenated fat and carboxylic acid.

The antibody composition may be administered as such in the form of spray, but sprays may be prepared using carriers which do not stimulate the oral or airway mucous membrane of a recipient and which can disperse the antibody composition as fine particles to facilitate absorption thereof.

Suitable carriers include lactose and glycerin. It is also possible to prepare aerosols, dry powders, *etc.* according to the properties of the antibody composition and the carriers used. In preparing these parenteral preparations, the above-mentioned additives for the oral preparations may also be added.

The dose and administration frequency will vary depending on the desired therapeutic effect, the administration route, the period of treatment, the patient's age and body weight, *etc.* However, an appropriate dose of the active ingredient for an adult person is generally 10 μg/kg to 20 mg/kg per day.

The anti-tumor effect of the antibody composition against various tumor cells can be examined by in vitro tests such as CDC activity measurement and ADCC activity measurement and *in vivo* tests such as anti-tumor experiments using tumor systems in experimental animals (e.g., mice).

The CDC activity and ADCC activity measurements and anti-tumor experiments can be carried out according to the methods described in the literature [*Cancer Immunology Immunotherapy,* 36, 373 (1993); *Cancer Research,* 54, 1511 (1994); *etc.*].

Brief Description of the Drawings

Fig. 1 shows the steps for constructing plasmid pKOFUT8Neo.

Fig. 2 shows the result of genomic Southern analysis of a hemi-knockout clone wherein one copy of the FUT8 allele was disrupted in CHO/DG44 cell. The lanes respectively show the following, from left to right: molecular weight marker, hemi-knockout clone 50-10-104, and parent cell CHO/DG44.

Fig. 3 shows the result of genomic Southern analysis of double-knockout clone WK704 wherein both FUT8 alleles were disrupted in CHO/DG44 cell. The arrow indicates the detection spot of a positive fragment resulting from homologous recombination.

Fig. 4 shows the result of genomic Southern analysis of a clone obtained by removing a drug-resistant gene from a double-knockout clone wherein both FUT8 alleles were disrupted in CHO/DG44 cell. The lanes respectively show the following, from left to right: molecular weight marker, drug resistant gene-removed double-knockout clone 4-5-C3, double-knockout clone WK704, hemi-knockout clone 50-10-104, and parent cell CHO/DG44.

Fig. 5 shows the reactivity of purified Ms705/GM2 antibody and DG44/GM2 antibody at varied concentrations to ganglioside GM2 measured by ELISA. The numbers on the abscissa indicate the antibody concentration and those on the ordinate indicate the absorbance at each antibody concentration. □ corresponds to the DG44/GM2 antibody, and ■ corresponds to the Ms705/GM2 antibody.

Fig. 6 shows the ADCC activity of purified Ms705/GM2 antibody and DG44/GM2 antibody at varied concentrations to human small cell lung cancer cell line SBC-3. The numbers on the abscissa indicate the antibody concentration and those on the ordinate indicate the cytotoxic activity at each antibody concentration. • corresponds to the DG44/GM2 antibody, and o corresponds to the Ms705/GM2 antibody.

The present invention is described below based on Examples; however, the present invention is not limited thereto.

Examples

Example 1

Construction of CHO/DG44 cell line in which both alleles of α1,6-fucosyltransferase (hereinafter referred to as FUT8) on the genome have been disrupted:

The CHO/DG44 cell line comprising the deletion of a genome region for both alleles of FUT8 including the translation initiation codons was constructed according to the following steps.

1. Construction of targeting vector pKOFUT8Neo comprising exon 2 of Chinese hamster FUT8 gene

pKOFUT8Neo was constructed in the following manner using targeting vector pKOFUT8Puro comprising exon 2 of Chinese hamster FUT8 gene constructed by the method described in Example 13-1 of WO02/31140, and pKOSelectNeo (manufactured by Lexicon).

pKOSelectNeo (manufactured by Lexicon) was digested with the restriction enzyme *Asc*I (manufactured by New England Biolabs) and subjected to agarose gel electrophoresis, and approximately 1.6 Kb *Asc*I fragment comprising the neomycin resistant gene expression unit was recovered using GENECLEAN Spin Kit (manufactured by BIO101).

After pKOFUT8Puro was digested with the restriction enzyme *Asc*I (manufactured by New England Biolabs), the end of the DNA fragment with alkaline phosphatase derived from *Escherichia coli* C15 (manufactured by Takara Shuzo Co., Ltd.) was dephosphorylated. After the reaction, the DNA fragment was purified by phenol/chloroform extraction and ethanol precipitation.

Sterilized water was added to 0.1 μg of the pKOSelectNeo-derived *Asc*I fragment (approximately 1.6 Kb) and 0.1 μg of the pKOFUT8Puro-derived *Asc*I fragment (approximately 10.1 Kb) obtained above to make up to 5 μl, and 5 μl of Ligation High (manufactured by Toyobo Co., Ltd.) was added thereto. The ligation reaction was carried out at 16°C for 30 minutes. *Escherichia coli* DH5α was transformed using the resulting reaction mixture, and a plasmid DNA was prepared from each of the obtained ampicillin-resistant clones. The plasmid DNA was subjected to reaction using BigDye Terminator Cycle Sequencing Ready Reaction Kit v2.0 (manufactured by Applied Biosystems) according to the attached instructions, and the nucleotide sequence was analyzed using DNA Sequencer ABI PRISM 377 (manufactured by Applied Biosystems). The thus obtained plasmid pKOFUT8Neo shown in Fig. 1 was used as a targeting vector for the subsequent preparation of FUT8 gene-hemi-knockout CHO cell line.

2. Preparation of hemi-knockout cell line in which one copy of the FUT8 gene on the genome has been disrupted

(1) Obtaining of a cell line in which the targeting vector pKOFUT8Neo has been introduced

The Chinese hamster FUT8 genome region targeting vector pKOFUT8Neo constructed in Example 1-1 was introduced into Chinese hamster ovary-derived CHO/DG44 cells deficient in the dihydrofolate reductase gene (*dhfr*) [*Somataic Cell and Molecular Genetics,* 12, 555 (1986)] in the following manner.

pKOFUT8Neo was digested with the restriction enzyme *Sal*I (manufactured by New England Biolabs) for linearization, and 4 μg of the linearized pKOFUT8Neo was introduced into $1.6 \times 10^6$ CHO/DG44 cells by electroporation [*Cytotechnology,* 3, 133 (1990)]. The resulting cells were suspended in IMDM-dFBS (10)-HT(1) [IMDM medium (manufactured by Invitrogen) containing 10% dialysis FBS (manufactured by Invitrogen) and 1-fold concentration HT supplement (manufactured by Invitrogen)] and then inoculated on a 10-cm dish for adherent cell culture (manufactured by Falcon). After culturing in a 5% $CO_2$ incubator at 37°C for 24 hours, the medium was replaced with 10 ml of IMDM-dFBS (10) (IMDM medium containing 10% dialysis FBS) containing 600 μg/ml G418 (manufactured by Nacalai Tesque, Inc.). Culturing was carried out in a 5% $CO_2$ incubator at 37°C for 15 days during which the above medium replacement was repeated every 3 to 4 days to obtain G418-resistant clones.

(2) Confirmation of homologous recombination by genomic PCR

Confirmation of the homologous recombination in the G418-resistant clones obtained in the above (1) was carried out by PCR using genomic DNA in the following manner.

The C418-resistant clones on a 96-well plate were subjected to trypsinization, and a 2-fold volume of a medium to be frozen (20% DMSO, 40% fetal calf serum and 40% IMDM) was added to each well to suspend the cells. One half of the cell suspension in each well was inoculated on a flat-bottomed 96-well plate for adherent cells (manufactured by Asahi Techno Glass) to prepare a replica plate, while the other half was stored by cryopreservation as a master plate.

The neomycin-resistant clones on the replica plate were cultured using IMDM-dFBS(10) containing 600 μg/ml

G418 in a 5% $CO_2$ incubator at 37°C for one week, followed by recovery of cells. The genomic DNA of each clone was prepared from the recovered cells according to a known method *[Analytical Biochemistry,* 201, 331 (1992)] and then dissolved overnight in 30 μl of TE-RNase buffer (pH 8.0) (10 mmol/l Tris-HCL, 1 mmol/l EDTA, 200 μg/ml RNase A).

Primers used in the genomic PCR were designed as follows. Primers respectively having the sequences represented by SEQ ID NOs:39 and 40, which are contained in the sequence of the FUT8 genome region obtained by the method described in Example 12 of WO03/31140 (SEQ ID NO:13), were employed as forward primers. Primers respectively having the sequences represented by SEQ ID NOs:41 and 42 which specifically bind to the loxP sequence of the targeting vector were employed as reverse primers. The above primers were used in the following polymerase chain reaction (PCR). A reaction mixture [25 μl; DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above primers (a combination of a forward primer and a reverse primer)] containing 10 μl of each genomic DNA solution prepared above was prepared, and PCR was carried out, after heating at 94°C for 3 minutes, by cycles, one cycle consisting of reaction at 94°C for one minute, reaction at 60°C for one minute and reaction at 72°C for 2 minutes.

After the PCR, the reaction mixture was subjected to 0.8% (w/v) agarose gel electrophoresis, and cell lines with which a specific amplification product (approximately 1.7 Kb) resulting from the homologous recombination was observed were determined to be positive clones.

(3) Confirmation of homologous recombination by genomic Southern blotting

Confirmation of the homologous recombination in the positive clones obtained in the above (2) was carried out by Southern blotting using genomic DNA in the following manner.

From the master plates stored by cryopreservation in the above (2), a 96-well plate containing the positive clones found in (2) was selected. After the plate was allowed to stand in a 5% $CO_2$ incubator at 37°C for 10 minutes, the cells in the wells corresponding to the positive clones were inoculated on a flat-bottomed 24-well plate for adherent cells (manufactured by Greiner). After culturing using IMDM-dFBS(10) containing 600 μg/ml G418 in a 5% $CO_2$ incubator at 37°C for one week, the cells were inoculated on a flat-bottomed 6-well plate for adherent cells (manufactured by Greiner). The plate was subjected to culturing in a 5% $CO_2$ incubator at 37°C and the cells were recovered. The genomic DNA of each clone was prepared from the recovered cells according to a known method [*Nucleic Acids Research,* 3, 2303 (1976)] and then dissolved overnight in 150 μl of TE-RNase buffer (pH 8.0).

The genomic DNA prepared above (12 μg) was digested with the restriction enzyme *Bam*HI (manufactured by New England Biolabs), and a DNA fragment recovered by ethanol precipitation was dissolved in 20 μl of TE buffer (pH 8.0) (10 mmol/l Tris-HCL, 1 mmol/l EDTA) and then subjected to 0.6% (w/v) agarose gel electrophoresis. After the electrophoresis, the genomic DNA was transferred to a nylon membrane according to a known method [*Proc. Natl. Acad Sci. USA,* 76, 3683 (1979)], followed by heat treatment of the nylon membrane at 80°C for 2 hours for immobilization.

In the meantime, a probe used in the Southern blotting was prepared in the following manner. Primers respectively having the sequences represented by SEQ ID NOs:43 and 44, which are contained in the sequence of the FUT8 genome region obtained by the method described in Example 12 of WO03/31140 (SEQ ID NO:13), were prepared and used in the following PCR. A reaction mixture [20 μl; DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above primers] containing 4.0 ng of pFUT8fgE2-2 described in Example 12 of WO02/31140 as a template was prepared, and PCR was carried out, after heating at 94°C for one minute, by 25 cycles, one cycle consisting of reaction at 94°C for 30 seconds, reaction at 55°C for 30 seconds and reaction at 74°C for one minute.

After the PCR, the reaction mixture was subjected to 1.75% (w/v) agarose gel electrophoresis, and approximately 230 bp probe DNA fragment was recovered using GENECLEAN Spin Kit (manufactured by BIO101). A 5-μl portion of the obtained probe DNA solution was subjected to radiolabeling using [$\alpha$-$^{32}$P] dCTP 1.75 MBq and Megaprime DNA Labelling system, dCTP (manufactured by Amersham Pharmacia Biotech).

Hybridization was carried out in the following manner. The above nylon membrane to which the genomic DNA digestion product had been transferred was put into a roller bottle and 15 ml of a hybridization solution [5 × SSPE, 50 × Denhaldt's solution, 0.5% (w/v) SDS, 100 μg/ml salmon sperm DNA] was added thereto. Prehybridization was carried out at 65°C for 3 hours. Then, the $^{32}$P-labeled probe DNA was heat-denatured and put into the bottle, and hybridization was carried out at 65°C overnight.

After the hybridization, the nylon membrane was immersed in 50 ml of a primary washing solution [2 × SSC - 0.1% (w/v) SDS] and washed by heating at 65°C for 15 minutes. After this washing step was repeated twice, the nylon membrane was immersed in 50 ml of a secondary washing solution [0.2 × SSC - 0.1% (w/v) SDS] and washed by heating at 65°C for 15 minutes. Then, the nylon membrane was exposed to an X-ray film at -80°C for development.

Fig. 2 shows the results of the analysis of the genomic DNAs of the parent cell line CHO/DG44 and the 50-10-104 cell line, which is the positive clone obtained in the above (2), according to the present method. In the CHO/DG44 cell line, only approximately 25.5 Kb fragment derived from the wild-type FUT8 allele was detected. On the other hand, in

the positive clone, i.e. 50-10-104 cell line, approximately 20.0 Kb fragment peculiar to the allele which underwent homologous recombination was detected in addition to approximately 25.5 Kb fragment derived from the wild-type FUT8 allele. The quantitative ratio of these two kinds of fragments was 1:1, whereby it was confirmed that the 50-10-104 cell line was a hemi-knockout clone wherein one copy of the FUT8 allele was disrupted.

3. Preparation of CHO/DG44 cell line in which the FUT8 gene on the genome has been double-knocked out

(1) Preparation of a cell line in which targeting vector pKOFUT8Puro has been introduced

In order to disrupt the other FUT8 allele in the FUT8 gene-hemi-knockout clone obtained in the above 2, the Chinese hamster FUT8 gene exon 2 targeting vector pKOFUT8Puro described in Example 13-1 of WO02/31140 was introduced into the clone in the following manner.

pKOFUT8Puro was digested with the restriction enzyme *Sal*I (manufactured by New England Biolabs) for linearization, and 4 μg of the linearized pKOFUT8Puro was introduced into 1.6 × $10^6$ cells of the FUT8 gene-hemi-knockout clone by electroporation [*Cytotechnology,* 3, 133 (1990)]. The resulting cells were suspended in IMDM-dFBS (10)-HT(1) and then inoculated on a 10-cm dish for adherent cell culture (manufactured by Falcon). After culturing in a 5% $CO_2$ incubator at 37°C for 24 hours, the medium was replaced with 10 ml of IMDM-dFBS(10)-HT(1) containing 15 μg/ml puromycin (manufactured by SIGMA). Culturing was carried out in a 5% $CO_2$ incubator at 37°C for 15 days during which the above medium replacement was repeated every 7 days to obtain puromycin-resistant clones.

5. Confirmation of homologous recombination by genomic Southern blotting

Confirmation of the homologous recombination in the drug-resistant clones obtained in the above (1) was carried out by Southern blotting using genomic DNA in the following manner.

The puromycin-resistant clones were recovered into a flat-bottomed plate for adherent cells (manufactured by Asahi Techno Glass) according to a known method [*Gene Targeting,* Oxford University Press (1993)], followed by culturing using IMDM-dFBS(10)-HT(1) containing 15 μg/ml puromycin (manufactured by SIGMA) in a 5% $CO_2$ incubator at 37°C for one week.

After the culturing, each clone on the above plate was subjected to trypsinization and the resulting cells were inoculated on a flat-bottomed 24-well plate for adherent cells (manufactured by Greiner). After culturing using IMDM-dFBS(10)-HT(1) containing 15 μg/ml puromycin (manufactured by SIGMA) in a 5% $CO_2$ incubator at 37°C for one week, the cells were subjected to trypsinization again and then inoculated on a flat-bottomed 6-well plate for adherent cells (manufactured by Greiner). The plate was subjected to culturing in a 5% $CO_2$ incubator at 37°C and the cells were recovered. The genomic DNA of each clone was prepared from the recovered cells according to a known method [Nucleic Acids Research, 3, 2303 (1976)] and then dissolved overnight in 150 μl of TE-RNase buffer (pH 8.0).

The genomic DNA prepared above (12 μg) was digested with the restriction enzyme *Bam*HI (manufactured by New England Biolabs), and a DNA fragment recovered by ethanol precipitation was dissolved in 20 μl of TE buffer (pH 8.0) and then subjected to 0.6% (w/v) agarose gel electrophoresis. After the electrophoresis, the genomic DNA was transferred to a nylon membrane according to a known method [*Proc. Natl. Acad Sci. USA,* 76, 3683 (1979)], followed by heat treatment of the nylon membrane at 80°C for 2 hours for immobilization.

Separately, a probe used in the Southern blotting was prepared in the following manner. Primers respectively having the sequences represented by SEQ ID NOs:45 and 46, which specifically bind to the sequences closer to the 5'-terminal than the FUT8 genome region contained in the targeting vector, were prepared and used in the following PCR. A reaction mixture [20 μl; DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above primers] containing 4.0 ng of the plasmid pFUT8fgE2-2 described in Example 12 of WO02/31140 as a template was prepared, and PCR was carried out, after heating at 94°C for one minute, by 25 cycles, one cycle consisting of reaction at 94°C for 30 seconds, reaction at 55°C for 30 seconds and reaction at 74°C for one minute.

After the PCR, the reaction mixture was subjected to 1.75% (w/v) agarose gel electrophoresis, and approximately 230 bp probe DNA fragment was purified using GENECLEAN Spin Kit (manufactured by BIO101). A 5-μl portion of the obtained probe DNA solution was subjected to radiolabeling using [α-$^{32}$P] dCTP 1.75 MBq and Megaprime DNA Labelling system, dCTP (manufactured by Amersham Pharmacia Biotech).

Hybridization was carried out in the following manner. The above nylon membrane to which the genomic DNA digestion product had been transferred was put into a roller bottle and 15 ml of a hybridization solution [5 × SSPE, 50 × Denhaldt's solution, 0.5% (w/v) SDS, 100 μg/ml salmon sperm DNA] was added thereto. Prehybridization was carried out at 65°C for 3 hours. Then, the $^{32}$P-labeled probe DNA was heat-denatured and put into the bottle, and hybridization was carried out at 65°C overnight.

After the hybridization, the nylon membrane was immersed in 50 ml of a primary washing solution [2 × SSC -

0.1% (w/v) SDS] and washed by heating at 65°C for 15 minutes. After this washing step was repeated twice, the nylon membrane was immersed in 50 ml of a secondary washing solution [0.2 × SSC - 0.1% (w/v) SDS] and washed by heating at 65°C for 15 minutes. Then, the nylon membrane was exposed to an X-ray film at -80°C for development.

Fig. 3 shows the result of the analysis of the genomic DNA of the WK704 cell line, which is one of the puromycin-resistant clones obtained from the 50-10-104 cell line by the method described in the above (1), according to the present method. In the WK704 cell line, approximately 25.5 Kb fragment derived from the wild-type FUT8 allele was deleted and only approximately 20.0 Kb fragment specific to the allele which underwent homologous recombination (indicated by arrow in the figure) was detected. From this result, it was confirmed that the WK704 cell line was a clone wherein both FUT8 alleles were disrupted.

4. Removal of the drug resistant genes from FUT8 gene-double-knockout cells

(1) Introduction of Cre recombinase expression vector

For the purpose of removing the drug resistant genes from the FUT8 gene-double-knockout clone obtained in the above item 3, the Cre recombinase expression vector pBS185 (manufactured by Life Technologies) was introduced into the clone in the following manner.

pBS185 (4 μg) was introduced into 1.6 × $10^6$ cells of the FUT8 gene-double-knockout clone by electroporation [*Cytotechnology,* 3, 133 (1990)]. The resulting cells were suspended in 10 ml of IMDM-dFBS(10)-HT(1) and the suspension was diluted 20000-fold with the same medium. The diluted suspension was inoculated on seven 10-cm dishes for adherent cell culture (manufactured by Falcon), followed by culturing in a 5% $CO_2$ incubator at 37°C for 10 days to form colonies.

(2) Obtaining of a cell line in which the Cre recombinase expression vector has been introduced

Clones arbitrarily selected from the colonies obtained in the above (1) were recovered into a flat-bottomed plate for adherent cells (manufactured by Asahi Techno Glass) according to a known method [*Gene Targeting,* Oxford University Press (1993)], followed by culturing using IMDM-dFBS(10)-HT(I) in a 5% $CO_2$ incubator at 37°C for one week.

After the culturing, each clone on the above plate was subjected to trypsinization, and a 2-fold volume of a medium to be frozen (20% DMSO, 40% fetal calf serum and 40% IMDM) was added to each well to suspend the cells. One half of the cell suspension in each well was inoculated on a flat-bottomed 96-well plate for adherent cells (manufactured by Asahi Techno Glass) to prepare a replica plate, while the other half was stored by cryopreservation as a master plate.

The cells on the replica plate were cultured using IMDM-dFBS(10)-HT(1) containing 600 μg/ml G418 and 15 μg/ml puromycin in a 5% $CO_2$ incubator at 37°C for one week. Positive clones in which the drug resistant genes inserted between loxP sequences has been removed by the expression of Cre recombinase have died in the presence of G418 and puromycin. The positive clones were selected in this manner.

(3) Confirmation of removal of the drug resistant genes by genomic Southern blotting

Confirmation of the removal of the drug resistant genes in the positive clones selected in the above (2) was carried out by genomic Southern blotting in the following manner.

From the master plates stored by cryopreservation in the above (2), a 96-well plate containing the above positive clones was selected. After the plate was allowed to stand in a 5% $CO_2$ incubator at 37°C for 10 minutes, the cells in the wells corresponding to the above clones were inoculated on a flat-bottomed 24-well plate for adherent cells (manufactured by Greiner). After culturing using IMDM-dFBS(10)-HT(1) for one week, the cells were subjected to trypsinization and then inoculated on a flat-bottomed 6-well plate for adherent cells (manufactured by Greiner). The plate was subjected to culturing in a 5% $CO_2$ incubator at 37°C and the proliferated cells were recovered. The genomic DNA of each clone was prepared from the recovered cells according to a known method [*Nucleic Acids Research,* 3, 2303 (1976)] and then dissolved overnight in 150 μl of TE-RNase buffer (pH 8.0).

The genomic DNA prepared above (12 μg) was digested with the restriction enzyme *Nhe*I (manufactured by New England Biolabs), and a DNA fragment recovered by ethanol precipitation was dissolved in 20 μl of TE buffer (pH 8.0) and then subjected to 0.6% (w/v) agarose gel electrophoresis. After the electrophoresis, the genomic DNA was transferred to a nylon membrane according to a known method [*Proc. Natl. Acad. Sci. USA,* 76, 3683 (1979)], followed by heat treatment of the nylon membrane at 80°C for 2 hours for immobilization.

Separately, a probe used in the Southern blotting was prepared in the following manner. PCR was carried out using primers respectively having the sequences represented by SEQ ID NOs:45 and 46, which specifically bind to the sequences similar to the 5'-terminal than the FUT8 genome region contained in the targeting vector. That is, a reaction

mixture [20 μl; DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above primers] containing 4.0 ng of the plasmid pFUT8fgE2-2 described in Example 12 of WO02/31140 as a template was prepared, and PCR was carried out, after heating at 94°C for one minute, by 25 cycles, one cycle consisting of reaction at 94°C for 30 seconds, reaction at 55°C for 30 seconds and reaction at 74°C for one minute.

After the PCR, the reaction mixture was subjected to 1.75% (w/v) agarose gel electrophoresis, and approximately 230 bp probe DNA fragment was purified using GENECLEAN Spin Kit (manufactured by BIO101). A 5-μl portion of the obtained probe DNA solution was subjected to radiolabeling using [$\alpha$-$^{32}$P] dCTP 1.75 MBq and Megaprime DNA Labelling system, dCTP (manufactured by Amersham Pharmacia Biotech).

Hybridization was carried out in the following manner. The above nylon membrane to which the genomic DNA digestion product had been transferred was put into a roller bottle and 15 ml of a hybridization solution [5 × SSPE, 50 × Denhaldt's solution, 0.5% (w/v) SDS, 100 μg/ml salmon sperm DNA] was added thereto. Prehybridization was carried out at 65°C for 3 hours. Then, the $^{32}$P-labeled probe DNA was heat-denatured and put into the bottle, and hybridization was carried out at 65°C overnight.

After the hybridization, the nylon membrane was immersed in 50 ml of a primary washing solution [2 × SSC - 0.1% (w/v) SDS] and washed by heating at 65°C for 15 minutes. After this washing step was repeated twice, the nylon membrane was immersed in 50 ml of a secondary washing solution [0.2 × SSC - 0.1% (w/v) SDS] and washed by heating at 65°C for 15 minutes. Then, the nylon membrane was exposed to an X-ray film at -80°C for development.

Fig. 4 shows the results of the analysis of the genomic DNAs of the parent cell line CHO/DG44, the 50-10-104 cell line described in the above item 2, the WK704 cell line described in the above item 3, and the 4-5-C3 cell line, which is one of the drug-sensitive clones obtained from the WK704 cell line by the method described in the above (2), according to the present method. In the CHO/DG44 cell line, only approximately 8.0 Kb DNA fragment derived from the wild-type FUT8 allele was detected. In the 50-10-104 cell line and the WK704 cell line, approximately 9.5 Kb DNA fragment derived from the allele which underwent homologous recombination was observed. On the other hand, in the 4-5-C3 cell line, only approximately 8.0 Kb DNA fragment resulting from the removal of the neomycin resistant gene (approximately 1.6 Kb) and the puromycin resistant gene (approximately 1.5 Kb) from the allele which underwent homologous recombination was detected. From the above results, it was confirmed that the drug resistant genes had been removed by Cre recombinase in the 4-5-C3 cell line.

Besides the 4-5-C3 cell line, plural FUT8 gene-double-knockout clones in which the drug-resistant gene had been removed (hereinafter referred to as FUT8 gene-double-knockout cells) were obtained.

Example 2

Expression of an anti-ganglioside GM2 human CDR-grafted antibody composition in FUT8 gene-double-knockout cell

1. Stable expression in FUT8 gene-double-knockout cell

By introducing an anti- ganglioside GM2 human CDR-grafted antibody expression vector, pKANTEX796HM2Lm-28No.1 described in Japanese Published Unexamined Patent Application No. 257893/98 into the FUT8 gene double knockout cell described in Example 1-4 and its parent cell line CHO/DG44 cell, a stable producing cell of the anti-ganglioside GM2 human CDR-grafted antibody composition was prepared in the following manner.

The pKANTEX796HM2Lm-28No.1 was linearized by digesting it with a restriction enzyme *Aat*II (manufactured by New England Biolabs), 10 μg of the linearized pKANTEX1259HV3LV0 was introduced into 1.6 × $10^6$ cells of the FUT8 gene double knockout cell or its parent cell line CHO/DG44 cell by electroporation [*Cytotechnology,* 3, 133 (1990)], and then the cells were suspended in 10 ml of IMDM-dFBS(10)-HT(1) [IMDM medium (manufactured by Invitrogen) containing 10% dialyzed FBS (manufactured by Invitrogen) and 1 × concentration of HT supplement (manufactured by Invitrogen)] and inoculated into a 75 $cm^2$ flask (manufactured by Greiner). After culturing at 37°C for 24 hours in a 5% $CO_2$ incubator, the medium was exchanged with IMDM-dFBS(10) [IMDM medium containing 10% dialyzed FBS] containing G418 (manufactured by Nacalai Tesque) in a concentration of 500 μg/ml, followed by culturing for 1 to 2 weeks. Transformants capable of growing in the IMDM-dFBS(10) medium containing G418 in a concentration of 500 μg/ml and of producing the anti-GM2 human CDR-grafted antibody were finally obtained. The transformant obtained from the parent CHO/DG44 cell line was designated DG44/GM2 cell line, and the transformant obtained from the FUT8 gene double knockout cell was designated Ms705/GM2 cell line.

2. Measurement of the human IgG antibody concentration in culture supernatant (ELISA)

Goat anti-human IgG (manufactured by H & L) antibody (manufactured by American Qualex) was diluted with Phosphate Buffered Saline (hereinafter referred to as PBS) (manufactured by Invitrogen) to a concentration of 1 μg/ml

and dispensed into wells of a 96-well plate for ELISA (manufactured by Greiner) in an amount of 50 μl/well, followed by standing at 4°C overnight for adsorption. After washing with PBS, PBS containing 1% BSA (hereinafter referred to as 1% BSA-PBS) (manufactured by Wako Pure Chemical Industries, Ltd.) was added to the wells in an amount of 100 μl/ well, followed by reaction at room temperature for one hour to block the remaining active groups. Then, the 1% BSA-PBS was discarded, and 50 μl each of the culture supernatant of transformant or variously diluted solutions of an antibody purified from the culture supernatant were respectively added to the wells, followed by reaction at room temperature for one hour. After the reaction, the wells were washed with PBS containing 0.05% Tween 20 (hereinafter referred to as Tween-PBS) (manufactured by Wako Pure Chemical Industries, Ltd.). To each well was added 50 μl of peroxidase-labeled goat anti-human IgG (manufactured by H & L) antibody solution (manufactured by American Qualex) diluted 2000-fold with 1% BSA-PBS as a secondary antibody solution, followed by reaction at room temperature for one hour. After the reaction, the wells were washed with Tween-PBS, and 50 μl of ABTS substrate solution [a solution prepared by dissolving 0.55 g of 2,2'-azino-bis(3-ethylbenzothiazoline-6-sulfonic acid) ammonium (manufactured by Wako Pure Chemical Industries, Ltd.) in 1 liter of 0.1 M citrate buffer (pH 4.2) and adding thereto, just before use, 1 μl/ml hydrogen peroxide (manufactured by Wako Pure Chemical Industries, Ltd.)] was added to each well to develop color. Then, the absorbance at 415 nm (hereinafter referred to as OD 415) was measured.

3. Purification of anti-ganglioside GM2 human CDR-grafted antibody compositions

Anti-ganglioside GM2 human CDR-grafted antibody compositions produced by the transformants DG44/GM2 cell line and Ms705/GM2 cell line obtained in Example 2-1 were purified in the following manner.

Each transformant was suspended in IMDM-dFBS(10) containing 500 μg/ml G418 and 30 ml of the suspension was incubated to a 182-$cm^2$ flask (manufactured by Greiner), followed by culturing in a 5% $CO_2$ incubator at 37°C for several days. When the cells became confluent, the culture supernatant was removed and the cells were washed with 25 ml of PBS, followed by addition of 30 ml of EXCELL301 medium (manufactured by JRH Biosciences). After culturing in a 5% $CO_2$ incubator at 37°C for 7 days, the cell suspension was recovered and subjected to centrifugation at 3000 rpm at 4°C for 5 minutes to recover the supernatant. The supernatant was filtered through Millex GV filter (pore size: 0.22 μm, manufactured by Millipore) for sterilization. The anti-ganglioside GM2 human CDR-grafted antibody composition was purified from the culture supernatant thus obtained using Mab Select (manufactured by Amersham Biosciences) column according to the attached instructions. The purified anti-ganglioside GM2 human CDR-grafted antibody compositions obtained from the DG44/GM2 cell line and the Ms705/GM2 cell line were designated DG44/GM2 antibody and Ms705/GM2 antibody, respectively.

Example 3

Biological activities of anti-ganglioside GM2 human CDR-grafted antibody compositino produced by FUT8 gene-double-knockout cell

1. Binding activity of anti-ganglioside GM2 human CDR-grafted antibody to ganglioside GM2 (ELISA)

The binding activity of the DG44/GM2 antibody and the Ms705/GM2 antibody purified in Example 2-3 to ganglioside GM2 was measured in the following manner.

Firstly, 57.5 ng of ganglioside GM2 (manufactured by SIGMA) was dissolved in an ethanol solution of 2 ml containing 10 ng of phosphatidyl choline (manufactured by SIGMA) and 5 ng of cholesterol (manufactured by SIGMA). A 20 μl portion of the solution was dispensed into each well of a 96-well ELISA plate (manufactured by Greiner), followed by air drying, and 1% BSA-PBS solution was added thereto in an amount of 100 μl/well, followed by reaction at room temperature for 1 hour to block the remaining active groups. After the 1% BSA-PBS was discarded, each of variously diluted solutions of the DG44/GM2 antibody or the Ms705/GM2 antibody prepared in Example 2-3 was added thereto in an amount of 50 μl/well, followed by reaction at room temperature for 1 hour. After the reaction, the wells were washed with Tween-PBS, and a peroxidase-labeled goat anti-human IgG (H&L) antibody solution (manufactured by Amercian Qualex) diluted 2000-fold with 1% BSA-PBS was added thereto in an amount of 50 μl/well as a secondary antibody solution, followed by reaction at room temperature for 1 hour. After the reaction, the wells were washed with Tween-PBS, and an ABTS substrate solution was added in an amount of 50 μl/well to develop color, followed by measurement of OD415.

Fig. 5 shows the binding activity of the DG44/GM2 antibody and the Ms705/GM2 antibody to ganglioside GM2. Each antibody had equal binding activity to ganglioside GM2.

2. *In vitro* cytotoxic activity (ADCC activity) of anti-ganglioside GM2 human CDR-grafted antibody composition

The *in vitro* cytotoxic activity of the DG44/GM2 antibody and the Ms705/GM2 antibody obtained in Example 2-3 was measured in the following manner.

(1) Preparation of a target cell suspension

Human small cell lung cancer cell line SBC-3 (JCRB 0818) cultured in RPMI 1640-FCS(10) medium (RPMI 1640 medium (manufactured by Invitrogen) containing 10% FCS) was washed with RPMI 1640-FCS(5) medium (RPMI 1640 medium (manufactured by Invitrogen) containing 5% FCS) by centrifugation and suspension and then adjusted to a density of $2 \times 10^5$ cells/ml by using RPMI 1640-FCS(5) medium and used as the target cell suspension.

(2) Preparation of an effector cell suspension

Venous blood (50 ml) was collected from a healthy donor and gently mixed with 0.5 ml of heparin sodium (manufactured by Shimizu Pharmaceutical Co., Ltd.). The monocyte layer was separated from this mixture using Lymphoprep (manufactured by AXIS SHIELD) according to the attached instructions. After being washed three times with RPMI1640-FCS(5) medium by centrifugation, the cells were suspended in the same medium at a density of $5 \times 10^6$ cells/ml to give an effector cell suspension.

(3) Measurement of ADCC activity

A 50 μl portion of the target cell suspension prepared in the above (1) was dispensed into each well of a 96-well U-shaped bottom plate (manufactured by Falcon) ($1 \times 10^4$ cells/well). Then, 50 μl of the effector cell suspension prepared in (2) was added to each well ($2.5 \times 10^5$ cells/well; the ratio of effector cells to target cells becomes 25:1). Subsequently, each of the anti-ganglioside GM2 human CDR-grafted antibodies was added to give a final concentration of 0.1 to 1000 ng/ml and to make a total volume of 150 μl, followed by reaction at 37°C for 4 hours. After the reaction, the plate was subjected to centrifugation, and the lactate dehydrogenase (LDH) activity of the supernatant was measured by obtaining absorbance data using CytoTox96 Non-Radioactive Cytotoxicity Assay (manufactured by Promega) according to the attached instructions. The absorbance data for target cell spontaneous release were obtained by the same procedure as above using only the medium instead of the effector cell suspension and the antibody solution, and those for effector cell spontaneous release were obtained by the same procedure using only the medium instead of the target cell suspension and the antibody solution. The absorbance data for target cell total release were obtained by the same procedure as above using the medium instead of the antibody solution and the effector cell suspension, adding 15 μl of 9% Triton X-100 solution 45 minutes before the completion of the reaction, and measuring the LDH activity of the supernatant. The ADCC activity was calculated according to the following equation.

Cytotoxic activity = {[absorbance of sample] - [absorbance for effector cell spontaneous release] - [absorbance for target cell spontaneous release]} / {[absorbance for target cell total release] - [absorbance for target cell spontaneous release]}

Fig. 6 shows the cytotoxic activity of the DG44/GM2 antibody and the Ms705/GM2 antibody against the human small cell lung cancer cell line SBC-3. The Ms705/GM2 antibody showed higher ADCC activity than the DG44/GM2 antibody at any antibody concentration and also showed highest cytotoxic activity.

Example 4

Analysis of monosaccharide composition of anti-ganglioside GM2 human CDR-grafted antibody composition produced by FUT8 gene-double-knockout cell

Analysis of the neutral sugar and amino sugar composition of the DG44/GM2 antibody and the Ms705/GM2 antibody purified in Example 1-3 was carried out in the following manner.

After the antibody was dried under reduced pressure using a centrifugal concentrator, 2.0 to 4.0 mM trifluoroacetic acid solution was added thereto and acid hydrolysis was carried out at 100°C for 2 to 4 hours to release neutral sugars and amino sugars from the protein. The trifluoroacetic acid solution was removed with a centrifugal concentrator,

and the sugars were redissolved in deionized water and subjected to analysis using a carbohydrate analysis system (DX-500, manufactured by Dionex). The analysis was carried out according to the elution program shown in Table 1 using CarboPac PA-1 column and CarboPac PA-1 guard column (manufactured by Dionex), 10 to 20 mM solution of sodium hydroxide in deionized water as an eluting solution and 500 mM solution of sodium hydroxide in deionized water as a washing solution.

Table 1

| Elution program for neutral sugar and amino sugar composition analysis | | | | | | |
|---|---|---|---|---|---|---|
| Time (min.) | 0 | 35 | 35.1 | 45 | 45.1 | 58 |
| Eluting solution (%) | 100 | 100 | 0 | 0 | 100 | 100 |
| Washing solution (%) | 0 | 0 | 100 | 100 | 0 | 0 |

From the peak areas of neutral and amino sugar components in the obtained elution profile, the composition ratio of components (fucose, galactose and mannose) was calculated, regarding the value ofN-acetylglucosamine as 4.

Table 2 shows the ratio of sugar chains having a structure in which fucose is not bound to the N-acetylglucosamine in the reducing end among the total complex type N-glycoside-linked sugar chains as calculated from the monosaccharide composition ratio of each antibody. In the DG44/GM2 antibody, the ratio of sugar chains having a structure in which fucose is not bound was 4%. On the other hand, in the Ms705/GM2 antibody, the peak of fucose was below the detection limit, whereby the ratio of sugar chains having a structure in which fucose is not bound was estimated to be nearly 100%.

The above result indicates that fucose is not bound to the N-acetylglucosamine in the reducing end in complex type N-glycoside-linked sugar chains in the Ms705/GM2 antibody.

Table 2

| Ratio of sugar chains to which fucose is not bound in anti-GM2 human CDR-grafted antibody compositions | |
|---|---|
| Antibody | Ratio of sugar chains to which fucose is not bound |
| DG44/GM2 antibody | 4% |
| Ms705/GM2 antibody | ~100% |

Example 5

Analysis of biological activity of anti-ganglioside GM2 human CDR-grafted antibody composition having sugar chains to which fucose is not bound

In Example 3-2, it was shown that the Ms705/GM2 antibody has higher ADCC activity than the DG44/GM2 antibody (Fig. 6). In this example, in order to further clarify superiority of the anti-ganglioside GM2 human CDR-grafted antibody composition of the present invention having sugar chains to which fucose is not bound, the biological activity was compared with an antibody composition mixed with anti-ganglioside GM2 human CDR-grafted antibody having sugar chains to which fucose is bound as follows.

Changes in the cytotoxic activity were examined in the case of adding the anti-ganglioside GM2 human CDR-grafted antibody having sugar chains to which fucose is bound, in the Ms705/GM2 antibody composition having sugar chains to fucose is not bound. ADCC activity of the anti-ganglioside GM2 human CDR-grafted antibody was measured in the following manner.

1. Preparation of target cell suspension

The preparation was carried out according to the method described in Example 3-2(1).

2. Preparation of effector cell suspension

A layer of monocytes was separated according to the method described in Example 3-2(2) and the monocytes were suspended by using RPMI 1640-FCS(5) medium to a density of $4 \times 10^6$ cells/ml to give the effector cell suspension.

3. Measurement of ADCC activity

The target cell suspension prepared in the above (1) was dispensed at 50 μl into each well of a 96-well U bottom plate (manufactured by Falcon) (1 × $10^4$ cells/well). Next, the effector cell suspension prepared in the above (2) was added at 50 μl (2 × $10^5$ cells/well, the ratio of effector cells and target cells becomes 20:1). Subsequently, the Ms705/GM2 antibody and the DG44/GM2 antibody were added independently or as a mixture of both of them, adjusted to a total volume of 150 μl and then allowed to react at 37°C for 4 hours. After the reaction, the plate was centrifuged, and lactate dehydrogenase (LDH) activity in the supernatant was measured using LDH-Cytotoxic Test Wako (manufactured by Wako Pure Chemical Industries) in accordance with the instructions attached thereto. The ADCC activity was calculated in accordance with the method described in Example 3-2.

By adding the DG44/GM2 antibody to a predetermined amount of the Ms705/GM2 antibody, anti-ganglioside GM2 human CDR-grafted antibody compositions containing a predetermined amount of an antibody to which fucose was not bound wherein the ratio of the antibody to fucose was not bound was changed, that is, anti-ganglioside GM2 human CDR-grafted antibody compositions in which a predetermined amount of the Ms705/GM2 antibody was mixed with the DG44/GM2 antibody in a 0- to 100-fold amount of the Ms705/GM2 antibody, were prepared, and ADCC activity of the antibody compositions was measured.

When the Ms705/GM2 antibody was further added to the Ms705/GM2 antibody, increase of the ADCC activity was observed with increase of the total amount of antibody. On the other hand, when the DG44/GM2 antibody was further added to the Ms705/GM2 antibody, the ADCC activity of the thus prepared antibody composition was reduced on the contrary regardless of the increased total antibody concentration. This result showed that an antibody molecule having sugar chains to which fucose is bound inhibits the activity of an antibody molecule having sugar chains to which fucose is not bound. Also, in the case of antibody compositions in which an antibody molecule having sugar chains to which fucose is bound was mixed with an antibody molecule having sugar chains to which fucose is not bound, an antibody composition in which the ratio of the antibody molecule having sugar chains to which fucose is not bound was more than 20% showed markedly higher ADCC activity than an antibody composition in which said ratio was less than 20%. ADCC activities of a sample of the Ms705/GM2 antibody and an antibody sample prepared by mixing the same amount of the Ms705/GM2 antibody with a 9-fold amount of the DG44/GM2 antibody were measured. The ADCC activity of the Ms705/GM2 antibody was sharply decreased by the addition of DG44/GM2 antibody. Even when antibody concentration of the antibody composition was increased 100-fold or more while keeping the existing ratio of the Ms705/GM2 antibody and the DG44/GM2 antibody at 1/9, the ADCC activity was still fell short of that of the Ms705/GM2 antibody sample having 1/100 antibody concentration. Based on the above, it was found that the antibody composition containing only an anti-ganglioside GM2 human CDR-grafted antibody molecule having sugar chains to which fucose is not bound in the present invention are excellent as a pharmaceutical composition.

Accordingly, patients who have not been able to be treated by the conventional antibody compositions comprising an anti-ganglioside GM2 human CDR-grafted antibody molecule can be treated by the anti-ganglioside GM2 human CDR-grafted antibody comprising sugar chains to which fucose is not bound of the present invention.

Free Text of Sequence Listing

SEQ ID NO:22 - Explanation of artificial sequence: antibody heavy chain region amino acid sequence
SEQ ID NO:23 - Explanation of artificial sequence: antibody heavy chain region amino acid sequence
SEQ ID NO:24 - Explanation of artificial sequence: antibody light chain region amino acid sequence
SEQ ID NO:25 - Explanation of artificial sequence: antibody light chain region amino acid sequence
SEQ ID NO:26 - Explanation of artificial sequence: antibody heavy chain region amino acid sequence
SEQ ID NO:27 - Explanation of artificial sequence: antibody heavy chain region amino acid sequence
SEQ ID NO:28 - Explanation of artificial sequence: antibody heavy chain region amino acid sequence
SEQ ID NO:29 - Explanation of artificial sequence: antibody heavy chain region amino acid sequence
SEQ ID NO:30 - Explanation of artificial sequence: antibody heavy chain region amino acid sequence
SEQ ID NO:31 - Explanation of artificial sequence: antibody light chain region amino acid sequence
SEQ ID NO:32 - Explanation of artificial sequence: antibody light chain region amino acid sequence
SEQ ID NO:33 - Explanation of artificial sequence: antibody light chain region amino acid sequence
SEQ ID NO:34 - Explanation of artificial sequence: antibody light chain region amino acid sequence
SEQ ID NO:35 - Explanation of artificial sequence: antibody light chain region amino acid sequence
SEQ ID NO:36 - Explanation of artificial sequence: synthetic DNA
SEQ ID NO:37 - Explanation of artificial sequence: synthetic DNA
SEQ ID NO:38 - Explanation of artificial sequence: synthetic DNA

SEQ ID NO:39 - Explanation of artificial sequence: synthetic DNA
SEQ ID NO:40 - Explanation of artificial sequence: synthetic DNA
SEQ ID NO:41 - Explanation of artificial sequence: synthetic DNA
SEQ ID NO:42 - Explanation of artificial sequence: synthetic DNA
SEQ ID NO:43 - Explanation of artificial sequence: synthetic DNA

## Claims

1. An antibody composition comprising a recombinant antibody molecule which specifically binds to ganglioside GM2 and has complex type N-glycoside-linked sugar chains in the Fc region, wherein the complex type N-glycoside-linked sugar chains have a structure in which fucose is not bound to N-acetylglucosamine in the reducing end in the sugar chains.

2. The antibody composition according to claim 1, wherein the complex type N-glycoside-linked sugar chains are sugar chains in which 1-position of fucose is not bound to 6-position of N-acetylglucosamine in the reducing end through $\alpha$-bond in the sugar chains.

3. The antibody composition according to claim 1 or 2, which specifically binds to a ganglioside GM2-expressing cell.

4. The antibody composition according to any one of claims 1 to 3, which has cytotoxic activity against a ganglioside GM2-expressing cell.

5. The antibody composition according to any one of claims 1 to 4, which has higher cytotoxic activity against a ganglioside GM2-expressing cell than a monoclonal antibody produced by a non-human animal-derived hybridoma.

6. The antibody composition according to claim 4 or 5, wherein the cytotoxic activity is antibody-dependent cell-mediated cytotoxic (ADCC) activity.

7. The antibody composition according to claim 4 or 5, wherein the cytotoxic activity is complement-dependent cytotoxic (CDC) activity.

8. The antibody composition according to any one of claims 1 to 7, which comprises complementarity determining region (CDR) 1, CDR 2 and CDR 3 of antibody molecule heavy chain (H chain) variable region (V region) consisting of the amino acid sequences represented by SEQ ID NOs:14, 15 and 16, respectively.

9. The antibody composition according to any one of claims 1 to 7, which comprises complementarity determining region (CDR) 1, CDR 2 and CDR 3 of antibody molecule light chain (L chain) variable region (V region) consisting of the amino acid sequences represented by SEQ ID NOs:17, 18 and 19, respectively.

10. The antibody composition according to any one of claims 1 to 9, which comprises complementarity determining region (CDR) 1, CDR 2 and CDR 3 of an antibody molecule heavy chain (H chain) variable region (V region) consisting of the amino acid sequences represented by SEQ ID NOs:14, 15 and 16, respectively; and CDR 1, CDR 2 and CDR 3 of antibody molecule light chain (L chain) V region consisting of the amino acid sequences represented by SEQ ID NOs:17, 18 and 19, respectively.

11. The antibody composition according to any one of claims 1 to 10, wherein the recombinant antibody is a human chimeric antibody or a human CDR-grafted antibody.

12. The antibody composition according to claim 11, wherein the human chimeric antibody comprises complementarity determining regions (CDRs) of heavy chain (H chain) variable region (V region) and light chain (L chain) V region of a monoclonal antibody which specifically binds to ganglioside GM2.

13. The antibody composition according to claim 12, wherein the heavy chain (H chain) variable region (V region) of the antibody molecule comprises the amino acid sequence represented by SEQ ID NO:20.

14. The antibody composition according to claim 12, wherein the light chain (L chain) variable region (V region) of the antibody molecule comprises the amino acid sequence represented by SEQ ID NO:21.

15. The human chimeric antibody composition according to any one of claims 12 to 14, wherein the heavy chain (H chain) variable region (V region) of the antibody molecule comprises the amino acid sequence represented by SEQ ID NO:20; and the light chain (L chain) V region of the antibody molecule comprises the amino acid sequence represented by SEQ ID NO:21.

16. The antibody composition according to claim 11, wherein the human CDR-grafted antibody comprises complementarity determining regions (CDRs) of heavy chain (H chain) variable region (V region) and light chain (L chain) V region of a monoclonal antibody which specifically binds to ganglioside GM2.

**17.** The antibody composition according to claim 16, which comprises complementarity determining regions (CDRs) of heavy chain (H chain) variable region (V region) and light chain (L chain) V region of a monoclonal antibody which specifically binds to ganglioside GM2, and framework regions (FRs) ofH chain V region and L chain V region of a human antibody.

**18.** The antibody composition according to claim 16 or 17, which comprises complementarity determining regions (CDRs) of heavy chain (H chain) variable region (V region) and light chain (L chain) V region of a monoclonal antibody which specifically binds to ganglioside GM2, framework regions (FRs) of H chain V region and L chain V region of a human antibody, and H chain constant region (C region) and L chain C region of a human antibody.

**19.** The antibody composition according to any one of claims 16 to 18, wherein the heavy chain (H chain) variable region (V region) of the antibody molecule comprises the amino acid sequence represented by SEQ ID NO:22 or an amino acid sequence in which at least one amino acid residue selected from the group consisting of Arg at position 38, Ala at position 40, Gln at position 43 and Gly at position 44 is substituted with another amino acid residue in the amino acid sequence represented by SEQ ID NO:22.

**20.** The antibody composition according to any one of claims 16 to 18, wherein the heavy chain (H chain) variable region (V region) of the antibody molecule comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23.

**21.** The antibody composition according to any one of claims 16 to 18, wherein the light chain (L chain) variable region (V region) of the antibody molecule comprises the amino acid sequence represented by SEQ ID NO:24 or an amino acid sequence in which at least one amino acid residue selected from the group consisting of Val at position 15, Tyr at position 35, Leu at position 46, Ser at position 59, Asp at position 69, Phe at position 70, Thr at position 71, Phe at position 72 and Ser at position 76 is substituted with another amino acid residue in the amino acid sequence represented by SEQ ID NO:24.

**22.** The antibody composition according to any one of claims 16 to 18, wherein the light chain (L chain) variable region (V region) of the antibody molecule comprises the amino acid sequence represented by SEQ ID NO:25 or an amino acid sequence in which at least one amino acid residue selected from the group consisting of Met at position 4, Leu at position 11, Val at position 15, Tyr at position 35, Ala at position 42, Leu at position 46, Asp at position 69, Phe at position 70, Thr at position 71, Leu at position 77 and Val at position 103 is substituted with another amino acid residue in the amino acid sequence represented by SEQ ID NO:25.

**23.** The antibody composition according to any one of claims 16 to 19 or 21, wherein the heavy chain (H chain) variable region (V region) of the antibody molecule comprises the amino acid sequence represented by SEQ ID NO:22 or an amino acid sequence in which at least one amino acid residue selected from the group consisting of Arg at position 38, Ala at position 40, Gln at position 43 and Gly at position 44 is substituted with another amino acid residue in the amino acid sequence represented by SEQ ID NO:22; and the light chain (L chain) V region of the antibody molecule comprises the amino acid sequence represented by SEQ ID NO:24 or an amino acid sequence in which at least one amino acid residue selected from the group consisting of Val at position 15, Tyr at position 35, Leu at position 46, Ser at position 59, Asp at position 69, Phe at position 70, Thr at position 71, Phe at position 72 and Ser at position 76 is substituted with another amino acid residue in the amino acid sequence represented by SEQ ID NO:24.

**24.** The antibody composition according to any one of claims 16 to 18, 20 or 21, wherein the heavy chain (H chain) variable region (V region) of the antibody molecule comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23 ; and the light chain (L chain) V region of the antibody molecule comprises the amino acid sequence represented by SEQ ID NO:24 or an amino acid sequence in which at least one amino acid residue selected from the group consisting of Val at position 15, Tyr at position 35, Leu at position 46, Ser at position 59, Asp at position 69, Phe at position 70, Thr at position 71, Phe at position 72

and Ser at position 76 is substituted with another amino acid residue in the amino acid sequence represented by SEQ ID NO:24.

**25.** The antibody composition according to any one of claims 16 to 18, 20 or 22, wherein the heavy chain (H chain) variable region (V region) of the antibody molecule comprises the amino acid sequence represented by SEQ ID NO:23 or an amino acid sequence in which at least one amino acid residue selected from the group consisting of Arg at position 67, Ala at position 72, Ser at position 84 and Arg at position 98 is substituted with another amino acid residue in the amino acid sequence represented by SEQ ID NO:23; and the light chain (L chain) V region of the antibody molecule comprises the amino acid sequence represented by SEQ ID NO:25 or an amino acid sequence in which at least one amino acid residue selected from the group consisting of Met at position 4, Leu at position 11, Val at position 15, Tyr at position 35, Ala at position 42, Leu at position 46, Asp at position 69, Phe at position 70, Thr at position 71, Leu at position 77 and Val at position 103 is substituted with another amino acid residue in the amino acid sequence represented by SEQ ID NO:25.

**26.** The antibody composition according to any one of claims 16 to 20 or 23 to 25, wherein the heavy chain (H chain) variable region (V region) of the antibody molecule comprises an amino acid sequence selected from the group consisting of the amino acid sequences represented by SEQ ID NOs:22, 26, 27, 28, 29 and 30.

**27.** The antibody composition according to any one of claims 16 to 18 or 21 to 25, wherein the light (L chain) variable region (V region) of the antibody molecule comprises an amino acid sequence selected from the group consisting of the amino acid sequences represented by SEQ ID NOs:31, 32, 33, 34 and 35.

**28.** The antibody composition according to any one of claims 16 to 27, wherein the heavy chain (H chain) variable region (V region) of the antibody molecule comprises an amino acid sequence selected from the group consisting of the amino acid sequences represented by SEQ ID NOs:22, 26, 27, 28, 29 and 30; and the light chain (L chain) V region of the antibody molecule comprises an amino acid sequence selected from the group consisting of the amino acid sequences represented by SEQ ID NOs:31, 32, 33, 34 and 35.

**29.** The antibody composition according to any one of claims 16 to 19, 21, 23 or 26 to 28, wherein the heavy chain (H chain) variable region (V region) of the antibody molecule comprises the amino acid sequence represented by SEQ ID NO:26; and the light chain (L chain) V region of the antibody molecule comprises the amino acid sequence represented by SEQ ID NO:31 or 32.

**30.** The antibody composition according to any one of claims 16 to 19, 21 to 23 or 26 to 28, wherein the heavy chain (H chain) variable region (V region) of the antibody molecule comprises the amino acid sequence represented by SEQ ID NO:22; and the light chain (L chain) V region of the antibody molecule comprises the amino acid sequence represented by SEQ ID NO:32 or 35.

**31.** A transformant producing the antibody composition according to any one of claims 1 to 30, which is obtainable by introducing a DNA encoding an antibody molecule which specifically binds to ganglioside GM2 into a host cell.

**32.** The transformant according to claim 31, wherein the host cell is a cell in which genome is modified so as to have deleted activity of an enzyme relating to the synthesis of an intracellular sugar nucleotide, GDP-fucose, or an enzyme relating to the modification of a sugar chain in which 1-position of fucose is bound to 6-position of N-acetylglucosamine in the reducing end through $\alpha$-bond in a complex type N-glycoside-linked sugar chain.

**33.** The transformant according to claim 31, wherein the host cell is a cell in which all of alleles on a genome encoding an enzyme relating to the synthesis of an intracellular sugar nucleotide, GDP-fucose, or an enzyme relating to the modification of a sugar chain in which 1-position of fucose is bound to 6-position of N-acetylglucosamine in the reducing end through $\alpha$-bond in a complex type N-glycoside-linked sugar chain are knocked out.

**34.** The transformant according to claim 32 or 33, wherein the enzyme relating to the synthesis of an intracellular sugar nucleotide, GDP-fucose, is an enzyme selected from GDP-mannose 4,6-dehydratase (GMD) or GDP-4-keto-6-deoxy-D-mannose-3,5-epimerase (Fx).

**35.** The transformant according to claim 34, wherein the GDP-mannose 4,6-dehydratase is a protein encoded by a DNA selected from the group consisting of the following (a) and (b):

(a) a DNA comprising the nucleotide sequence represented by SEQ ID NO:1;
(b) a DNA which hybridizes with the DNA consisting of the nucleotide sequence represented by SEQ ID NO: 1 under stringent conditions and which encodes a protein having GDP-mannose 4,6-dehydratase activity.

**36.** The transformant according to claim 34, wherein the GDP-mannose 4,6-dehydratase is a protein selected from the group consisting of the following (a) to (c):

(a) a protein comprising the amino acid sequence represented by SEQ ID NO:2;
(b) a protein consisting of an amino acid sequence wherein one or more amino acid residue(s) is/are deleted, substituted, inserted and/or added in the amino acid sequence represented by SEQ ID NO:2 and having GDP-mannose 4,6-dehydratase activity;
(c) a protein consisting of an amino acid sequence which has 80% or more homology to the amino acid sequence represented by SEQ ID NO:2 and having GDP-mannose 4,6-dehydratase activity.

**37.** The transformant according to claim 34, wherein the GDP-4-keto-6-deoxy-D-mannose-3,5-epimerase is a protein encoded by a DNA selected from the group consisting of the following (a) and (b):

(a) a DNA comprising the nucleotide sequence represented by SEQ ID NO:3;
(b) a DNA which hybridizes with the DNA consisting of the nucleotide sequence represented by SEQ ID NO:3 under stringent conditions and which encodes a protein having GDP-4-keto-6-deoxy-D-mannose-3,5-epimerase activity.

**38.** The transformant according to claim 34, wherein the GDP-4-keto-6-deoxy-D-mannose-3,5-epimerase is a protein selected from the group consisting of the following (a) to (c):

(a) a protein comprising the amino acid sequence represented by SEQ ID NO:4;
(b) a protein consisting of an amino acid sequence wherein one or more amino acid residue(s) is/are deleted, substituted, inserted and/or added in the amino acid sequence represented by SEQ ID NO:4 and having GDP-4-keto-6-deoxy-D-mannose-3,5-epimerase activity;
(c) a protein consisting of an amino acid sequence which has 80% or more homology to the amino acid sequence represented by SEQ ID NO:4 and having GDP-4-keto-6-deoxy-D-mannose-3,5-epimerase activity.

**39.** The transformant according to claim 32 or 33, wherein the enzyme relating to the modification of a sugar chain in which 1-position of fucose is bound to 6-position of N-acetylglucosamine in the reducing end through α-bond in a complex type N-glycoside-linked sugar chain is α1,6-fucosyltransferase.

**40.** The transformant according to claim 39, wherein the α1,6-fucosyltransferase is a protein encoded by a DNA selected from the group consisting of the following (a) to (d):

(a) a DNA comprising the nucleotide sequence represented by SEQ ID NO:5;
(b) a DNA comprising the nucleotide sequence represented by SEQ ID NO:6;
(c) a DNA which hybridizes with the DNA consisting of the nucleotide sequence represented by SEQ ID NO: under stringent conditions and which encodes a protein having α1,6-fucosyltransferase activity;
(d) a DNA which hybridizes with the DNA consisting of the nucleotide sequence represented by SEQ ID NO:6 under stringent conditions and which encodes a protein having α1,6-fucosyltransferase activity.

**41.** The transformant according to claim 39, wherein the α1,6-fucosyltransferase is a protein selected from the group consisting of the following (a) to (f):

(a) a protein comprising the amino acid sequence represented by SEQ ID NO:7;
(b) a protein comprising the amino acid sequence represented by SEQ ID NO:8;
(c) a protein consisting of an amino acid sequence wherein one or more amino acid residue(s) is/are deleted, substituted, inserted and/or added in the amino acid sequence represented by SEQ ID NO:7 and having α1,6-fucosyltransferase activity;
(d) a protein consisting of an amino acid sequence wherein one or more amino acid residue(s) is/are deleted, substituted, inserted and/or added in the amino acid sequence represented by SEQ ID NO:8 and having α1,6-fucosyltransferase activity;
(e) a protein consisting of an amino acid sequence which has 80% or more homology to the amino acid sequence

represented by SEQ ID NO:7 and having α1,6-fucosyltransferase activity;
(f) a protein consisting of an amino acid sequence which has 80% or more homology to the amino acid sequence represented by SEQ ID NO:8 and having α1,6-fucosyltransferase activity.

**42.** The transformant according to claim 41, wherein the transformant is FERM BP-8470.

**43.** The transformant according to any one of claims 31 to 42, wherein the host cell is a cell selected from the group consisting of the following (a) to (i):

(a) a CHO cell derived from Chinese hamster ovary tissue;
(b) a rat myeloma cell line YB2/3HL.P2.G11.16Ag.20 cell;
(c) a mouse myeloma cell line NSO cell;
(d) a mouse myeloma cell line SP2/0-Ag14 cell;
(e) a BHK cell derived from Syrian hamster kidney tissue;
(f) an antibody-producing hybridoma cell;
(g) a human leukemia cell line Namalwa cell;
(h) an embryonic stem cell;
(i) a fertilized egg cell.

**44.** A process for producing the antibody composition according to any one of claims 1 to 30, which comprises culturing the transformant according to any one of claims 31 to 43 in a medium to form and accumulate the antibody composition in the culture, and recovering and purifying the antibody composition from the culture.

**45.** The antibody composition according to any one of claims 1 to 32, which is obtainable by the process according to claim 44.

**46.** A pharmaceutical composition comprising the antibody composition according to any one of claims 1 to 30 and 45 as an active ingredient.

**47.** A therapeutic agent for diseases relating to a ganglioside GM2, comprising the antibody composition according to any one of claims 1 to 30 and 45 as an active ingredient.

**48.** The therapeutic agent according to claim 47, wherein the diseases relating to a ganglioside GM2 are cancer.

FIG. 1

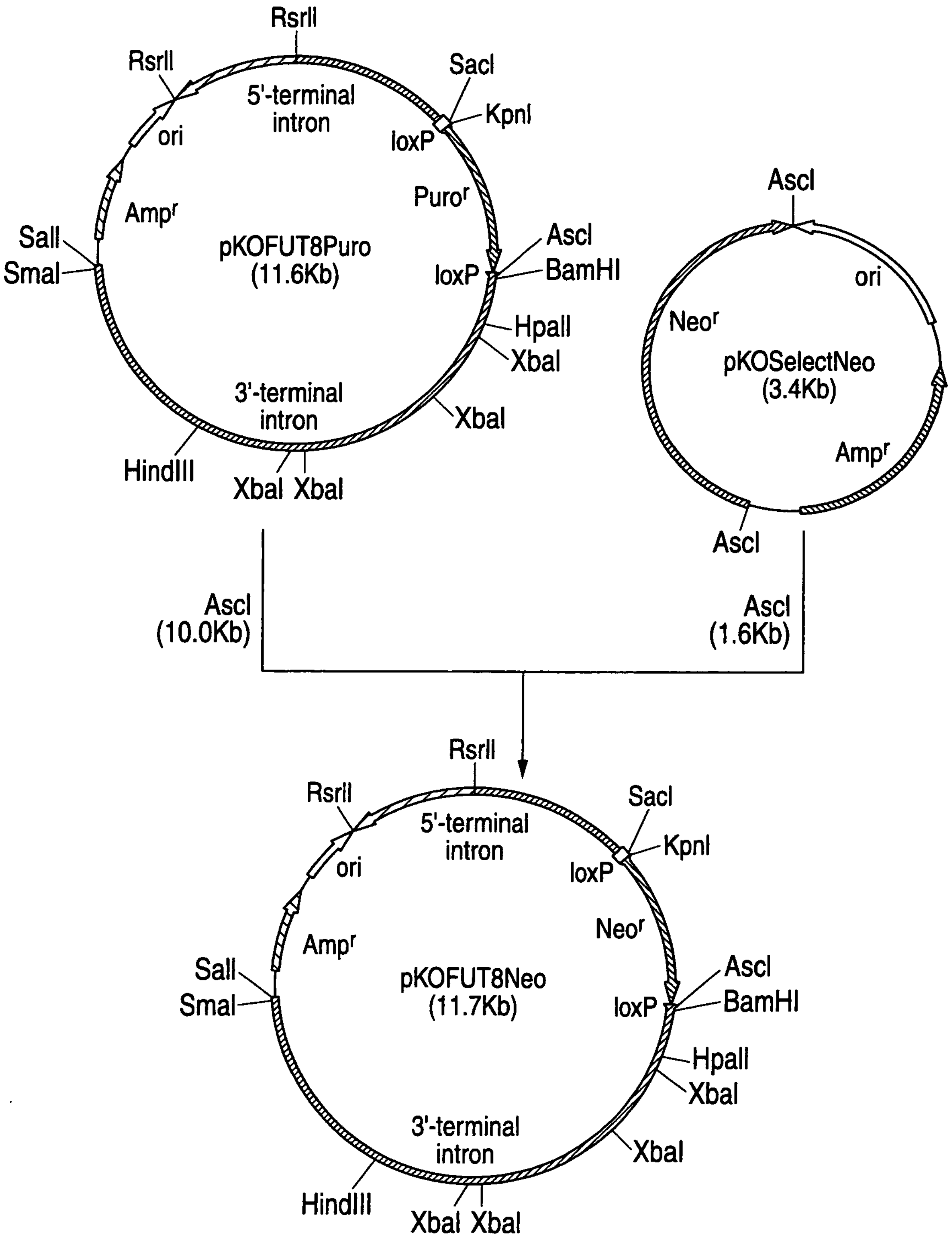
RsrII
RsrII
5'-terminal intron
SacI
KpnI
ori
loxP
Ampr
Puror
SalI
SmaI
pKOFUT8Puro (11.6Kb)
loxP
AscI
BamHI
HpaII
XbaI
3'-terminal intron
XbaI
HindIII
XbaI XbaI
AscI
ori
Neor
pKOSelectNeo (3.4Kb)
Ampr
AscI
AscI (10.0Kb)
AscI (1.6Kb)
RsrII
RsrII
5'-terminal intron
SacI
KpnI
ori
loxP
Ampr
Neor
SalI
SmaI
pKOFUT8Neo (11.7Kb)
loxP
AscI
BamHI
HpaII
XbaI
3'-terminal intron
XbaI
HindIII
XbaI XbaI

FIG. 2

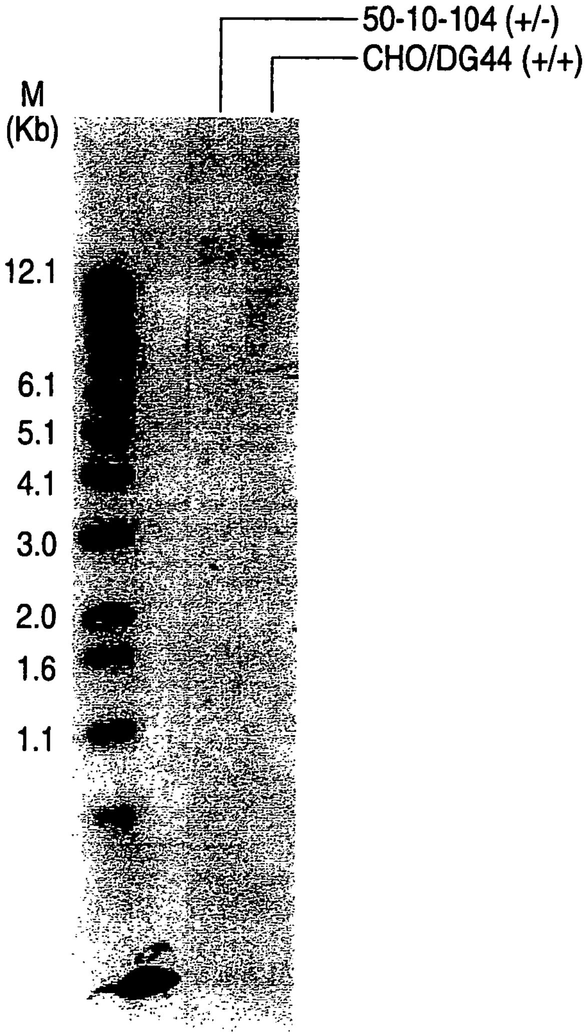

FIG. 3

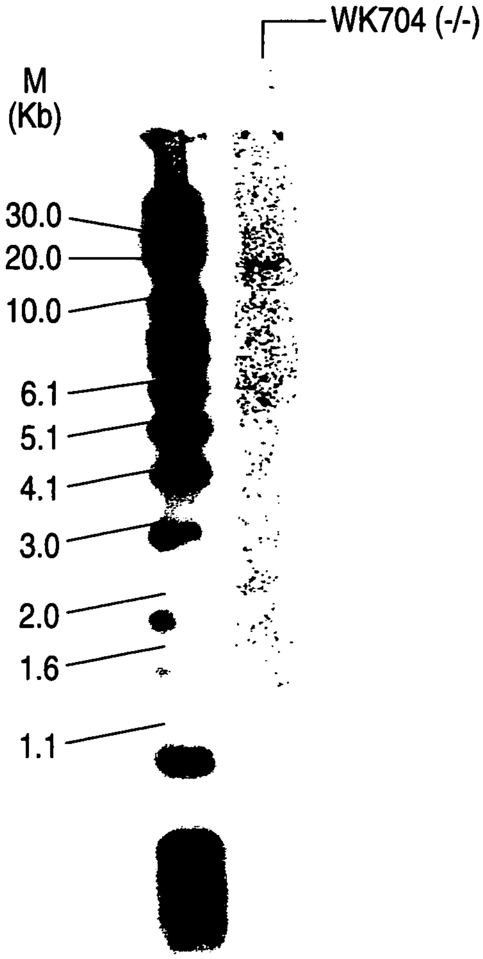

FIG. 4

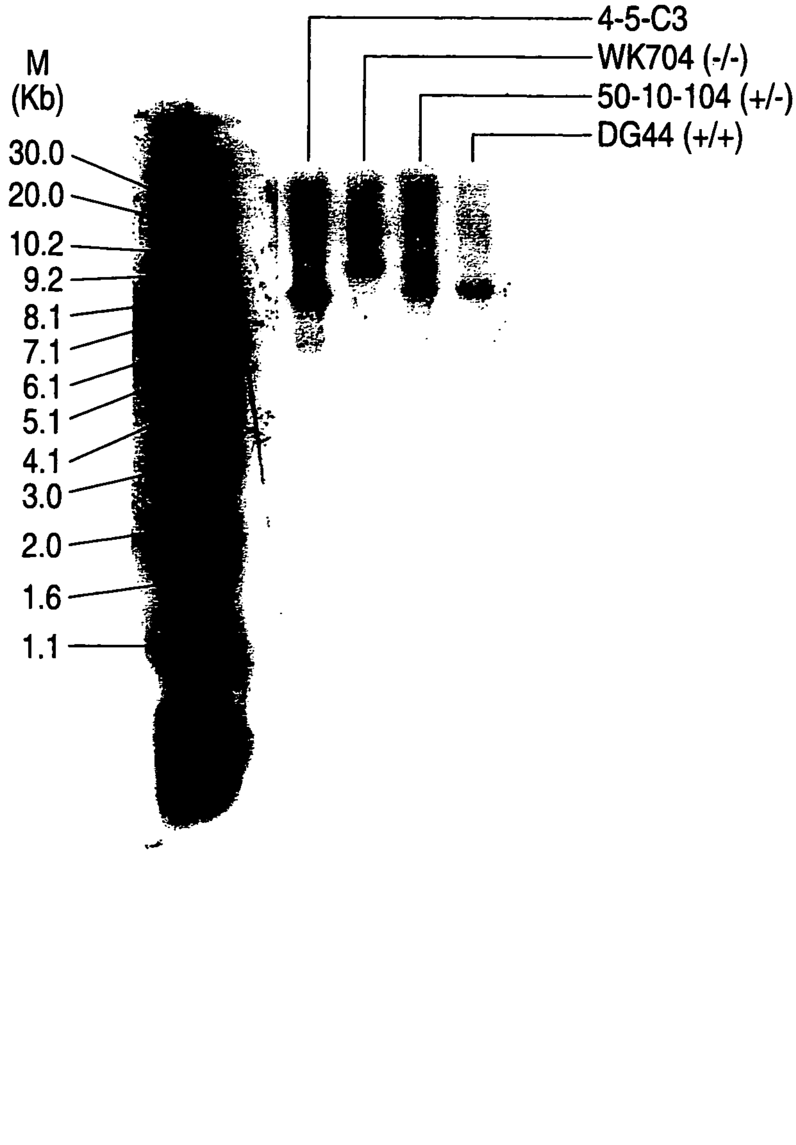
M
(Kb)
30.0
20.0
10.2
9.2
8.1
7.1
6.1
5.1
4.1
3.0
2.0
1.6
1.1
4-5-C3
WK704 (-/-)
50-10-104 (+/-)
DG44 (+/+)

*FIG. 5*

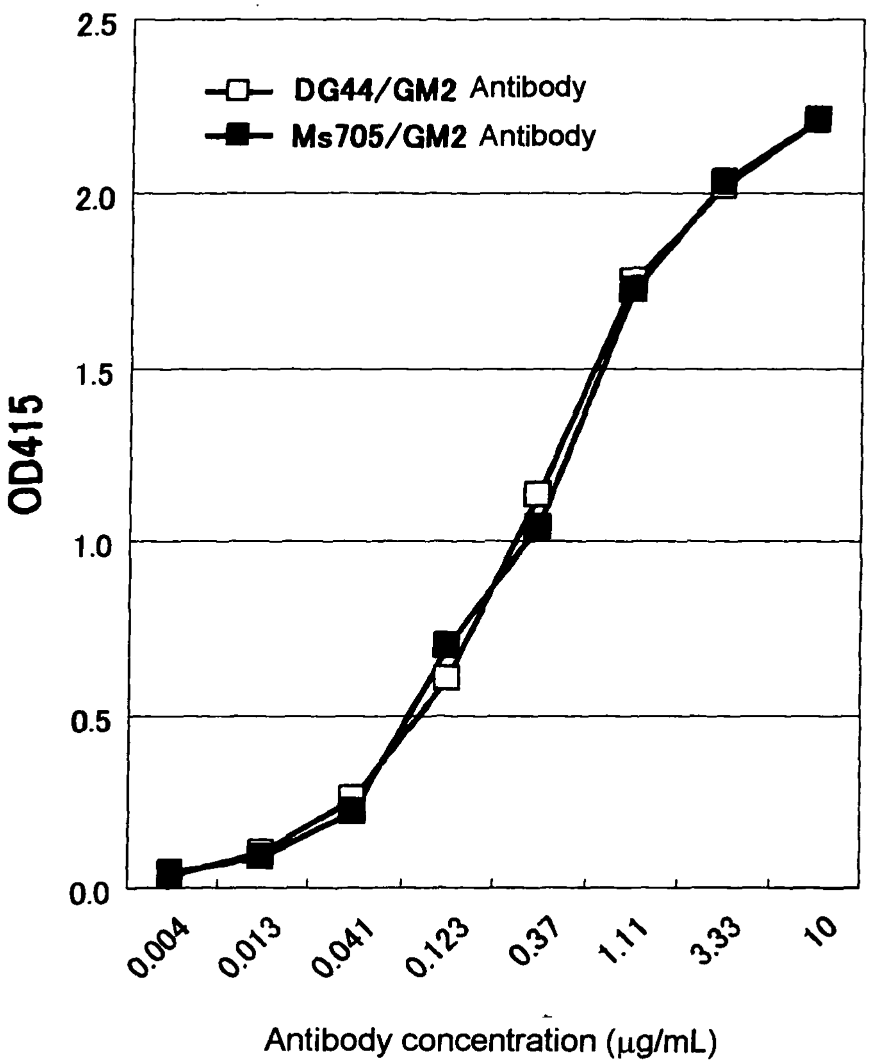
2.5
2.0
1.5
1.0
0.5
0.0
OD415
DG44/GM2 Antibody
Ms705/GM2 Antibody
0.004
0.013
0.041
0.123
0.37
1.11
3.33
10
Antibody concentration (μg/mL)

FIG. 6

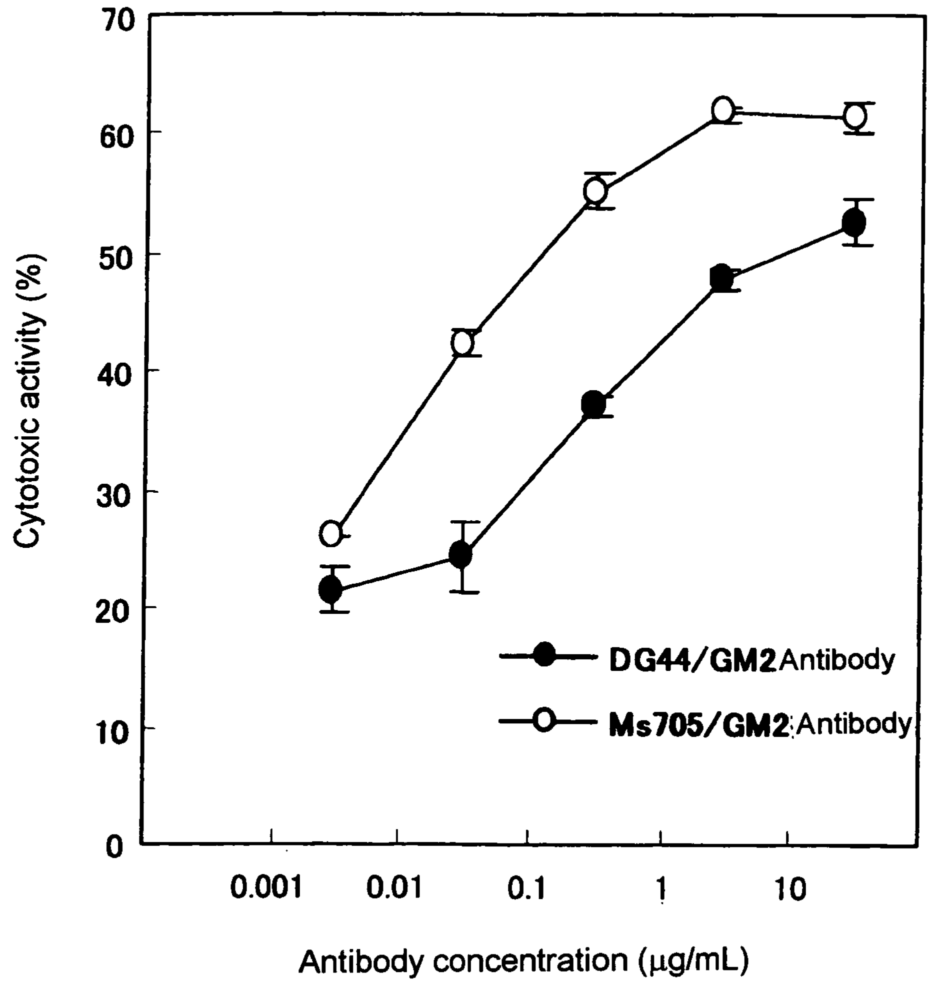
70
60
50
40
30
20
10
0
Cytotoxic activity (%)
0.001
0.01
0.1
1
10
Antibody concentration (μg/mL)
DG44/GM2 Antibody
Ms705/GM2 Antibody

## INTERNATIONAL SEARCH REPORT

International application No.
PCT/JP2004/015317

A. CLASSIFICATION OF SUBJECT MATTER
Int.Cl$^7$ C07K16/18, C12N15/13, C12N5/10, C12P21/08, A61K39/395, A61P35/00

According to International Patent Classification (IPC) or to both national classification and IPC

B. FIELDS SEARCHED

Minimum documentation searched (classification system followed by classification symbols)
Int.Cl$^7$ C07K16/18, C12N15/13, C12N5/10, C12P21/08, A61K39/395, A61P35/00

Documentation searched other than minimum documentation to the extent that such documents are included in the fields searched

Electronic data base consulted during the international search (name of data base and, where practicable, search terms used)
REGISTRY(STN), CA(STN), WPI(DIALOG), BIOSIS(DIALOG), JSTPlus(JOIS), GenBank/EMBL/DDBJ/GeneSeq, SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| Y | WO 02/31140 A1 (Kyowa Hakko Kogyo Co., Ltd.), 18 April, 2002 (18.04.02), & AU 200194198 A & US 2003/0115614 A1 & EP 1331266 A1 & BR 200114475 A & KR 2003081312 A & MX 2003002974 A1 | 1-48 |
| Y | JP 10-257893 A (Kyowa Hakko Kogyo Co., Ltd.), 29 September, 1998 (29.09.98), & AU 9859420 A & EP 882794 A2 & CA 2226400 A & DE 69824234 E & ES 2219802 T3 | 1-48 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 January, 2005 (12.01.05) | 01 February, 2005 (01.02.05) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/015317

C (Continuation). DOCUMENTS CONSIDERED TO BE RELEVANT

| Category* | Citation of document, with indication, where appropriate, of the relevant passages | Relevant to claim No. |
|---|---|---|
| P,X | YAMANE-OHNUKI, N. et al., Establishment of FUT8 knockout Chinese hamster ovary cells: an ideal host cell line for producing completely defucosylated antibodies with enhanced antibody-dependent cellular cytotoxicity. Biotechnol.Bioeng. 05 September, 2004 (05.09.04), Vol.87, No.5, pages 614 to 622 | 1-48 |
| P,X | WO 03/85107 A1 (Kyowa Hakko Kogyo Co., Ltd.), 16 October, 2003 (16.10.03), & AU 2003236022 A1 & US 2004/0110704 A1 | 1-48 |
| P,X | WO 03/85118 A1 (Kyowa Hakko Kogyo Co., Ltd.), 16 October, 2003 (16.10.03), & AU 2003236015 A1 & US 2004/0132140 A1 | 1-48 |

Form PCT/ISA/210 (continuation of second sheet) (January 2004)